# EUROPÄISCHE PATENTANMELDUNG

(11) **EP 3 865 589 A2**
(43) Veröffentlichungstag der Anmeldung: **18.08.2021**
(21) Anmeldenummer: 21157249.0
(22) Anmeldetag: 15.02.2021
(51) Int. Cl.: C12Q 1/6841, B01L 3/00, C12Q 1/689

(54) **VERFAHREN ZUM NACHWEIS VON MIKROORGANISMEN, KORRESPONDIERENDE VERWENDUNG UND PROBENTRÄGER**

(30) Priorität: 14.02.2020 DE 102020103961; 12.02.2021 US 202117175042
(71) Anmelder: Testo bioAnalytics GmbH, 79822 Titisee-Neustadt (DE)
(72) Erfinder: Quehl, Paul, 37085 Göttingen (DE); Dr. Zinaida Vasileuskaya-Schulz, Zinaida, 79111 Freiburg (DE)
(74) Vertreter: Mertzlufft-Paufler, Cornelius

(57) **Zusammenfassung**

Es wird vorgeschlagen, ein Verfahren zum Nachweis von Mikroorganismen, z.B. der Bakterienfamilie der Enterobacteriaceaen, in einer Probe bereitzustellen, mittels Einschleusen identitätsbestimmender Nukleinsäuresonden in die einzelnen Zellkörper, wobei die Nukleinsäuresonden mit den Nukleinsäuren der Mikroorganismen eine Hybridisierung eingehen, und anschließendem optischen Detektieren der in den einzelnen Zellkörpern erzeugten Hybridisierungen, wobei eine Mischung aus wenigstens einer ersten Nukleinsäuresonde und einer zweiten Nukleinsäuresonde verwendet wird, und dass die erste Nukleinsäuresonde und die zweite Nukleinsäuresonde jeweils in einem von zwei miteinander nicht überlappenden Bereichen der Zielnukleinsäure anbinden.

SEQUENCE LISTING

<110> Testo SE & Co. KGaA

<120> Verfahren zum Nachweis von Mikroorganismen, korrespondierende
Verwendung und Probenträger

<130> E 21 0186 C

<160> 1216

<170> BiSSAP 1.3.6

<210> 1
<211> 22
<212> DNA
<213> Artificial Sequence

<220>
<223> Seq. ID NO 1

<400> 1
cctacttctt ttgcaaccca ct                                                22

<210> 2
<211> 34
<212> DNA
<213> Artificial Sequence

<220>
<223> Seq. ID NO 2

<400> 2
cgcggaccta cttcttttgc aacccacttc cgcg                                   34

<210> 3
<211> 34
<212> DNA
<213> Artificial Sequence

<220>
<223> Seq. ID NO 3

<400> 3
ctcgcgccta cttcttttgc aacccactcg cgag                                   34

<210> 4
<211> 34
<212> DNA
<213> Artificial Sequence

<220>
<223> Seq. ID NO 4

<400> 4
ccgaccccta cttcttttgc aacccactgg tcgg                                   34

<210> 5
<211> 34
<212> DNA
<213> Artificial Sequence

<220>
<223> Seq. ID NO 5

<400> 5
cgacgtccta cttcttttgc aacccactac gtcg                                    34

<210> 6
<211> 34
<212> DNA
<213> Artificial Sequence

<220>
<223> Seq. ID NO 6

<400> 6
cccaccccta cttcttttgc aacccactgg tggg                                    34

<210> 7
<211> 34
<212> DNA
<213> Artificial Sequence

<220>
<223> Seq. ID NO 7

<400> 7
cgccgaccta cttcttttgc aacccacttc ggcg                                    34

<210> 8
<211> 34
<212> DNA
<213> Artificial Sequence

<220>
<223> Seq. ID NO 8

<400> 8
ctggcgccta cttcttttgc aacccactcg ccag                                    34

<210> 9
<211> 34
<212> DNA
<213> Artificial Sequence

<220>
<223> Seq. ID NO 9

<400> 9
ctgccgccta cttcttttgc aacccactcg gcag                                    34

<210> 10
<211> 34
<212> DNA
<213> Artificial Sequence

<220>
<223> Seq. ID NO 10

<400> 10
ccgggtccta cttcttttgc aacccactac ccgg                                    34

<210> 11
<211> 34
<212> DNA
<213> Artificial Sequence

<220>
<223> Seq. ID NO 11

<400> 11
gcgcctccta cttcttttgc aacccactag gcgc                                    34

<210> 12
<211> 34
<212> DNA
<213> Artificial Sequence

<220>
<223> Seq. ID NO 12

<400> 12
gagcgcccta cttcttttgc aacccactgc gctc                                    34

<210> 13
<211> 34
<212> DNA
<213> Artificial Sequence

<220>
<223> Seq. IDF NO 13

<400> 13
ggctggccta cttcttttgc aacccactcc agcc                                    34

<210> 14
<211> 34
<212> DNA
<213> Artificial Sequence

<220>

<223> Seq. ID NO 14

<400> 14
gctgcaccta cttcttttgc aacccacttg cagc                                34


<210> 15
<211> 34
<212> DNA
<213> Artificial Sequence


<220>
<223> Seq. ID NO 15

<400> 15
gggtggccta cttcttttgc aacccactcc accc                                34


<210> 16
<211> 34
<212> DNA
<213> Artificial Sequence


<220>
<223> Seq. ID NO 16

<400> 16
gcggctccta cttcttttgc aacccactag ccgc                                34


<210> 17
<211> 34
<212> DNA
<213> Artificial Sequence


<220>
<223> Seq. ID NO 17

<400> 17
gaccgcccta cttcttttgc aacccactgc ggtc                                34


<210> 18
<211> 34
<212> DNA
<213> Artificial Sequence


<220>
<223> Seq. ID NO 18

<400> 18
gacggcccta cttcttttgc aacccactgc cgtc                                34


<210> 19
<211> 34
<212> DNA
<213> Artificial Sequence

<220>
<223> Seq. ID NO 19

<400> 19
ggcccaccta cttcttttgc aacccacttg ggcc                                    34


<210> 20
<211> 27
<212> DNA
<213> Artificial Sequence


<220>
<223> Seq. ID NO 20

<400> 20
cggttaacct tgctacagaa tataagt                                            27


<210> 21
<211> 39
<212> DNA
<213> Artificial Sequence


<220>
<223> Seq. ID NO 21

<400> 21
cgcggacggt taaccttgct acagaatata agttccgcg                               39


<210> 22
<211> 39
<212> DNA
<213> Artificial Sequence


<220>
<223> Seq. ID NO 22

<400> 22
ctcgcgcggt taaccttgct acagaatata agtcgcgag                               39


<210> 23
<211> 39
<212> DNA
<213> Artificial Sequence


<220>
<223> Seq. ID NO 23

<400> 23
ccgacccggt taaccttgct acagaatata agtggtcgg                               39


<210> 24
<211> 39
<212> DNA

<213> Artificial Sequence

<220>
<223> Seq. ID NO 24

<400> 24
cgacgtcggt taaccttgct acagaatata agtacgtcg 39

<210> 25
<211> 39
<212> DNA
<213> Artificial Sequence

<220>
<223> Seq. ID NO 25

<400> 25
cccacccggt taaccttgct acagaatata agtggtggg 39

<210> 26
<211> 39
<212> DNA
<213> Artificial Sequence

<220>
<223> Seq. ID NO 26

<400> 26
cgccgacggt taaccttgct acagaatata agttcggcg 39

<210> 27
<211> 39
<212> DNA
<213> Artificial Sequence

<220>
<223> Seq. ID NO 27

<400> 27
ctggcgcggt taaccttgct acagaatata agtcgccag 39

<210> 28
<211> 39
<212> DNA
<213> Artificial Sequence

<220>
<223> Seq. ID NO 28

<400> 28
ctgccgcggt taaccttgct acagaatata agtcggcag 39

<210> 29

<211> 39
<212> DNA
<213> Artificial Sequence

<220>
<223> Seq. ID NO 29

<400> 29
ccgggtcggt taaccttgct acagaatata agtacccgg 39

<210> 30
<211> 38
<212> DNA
<213> Artificial Sequence

<220>
<223> Seq. ID NO 30

<400> 30
gcgtccggtt aaccttgcta cagaatataa gtggacgc 38

<210> 31
<211> 39
<212> DNA
<213> Artificial Sequence

<220>
<223> Seq. ID NO 31

<400> 31
gcgcctcggt taaccttgct acagaatata agtaggcgc 39

<210> 32
<211> 39
<212> DNA
<213> Artificial Sequence

<220>
<223> Seq. ID NO 32

<400> 32
gagcgccggt taaccttgct acagaatata agtgcgctc 39

<210> 33
<211> 39
<212> DNA
<213> Artificial Sequence

<220>
<223> Seq. ID NO 33

<400> 33
ggctggcggt taaccttgct acagaatata agtccagcc 39

<210> 34
<211> 39
<212> DNA
<213> Artificial Sequence

<220>
<223> Seq. ID NO 34

<400> 34
gctgcacggt taaccttgct acagaatata agttgcagc                              39

<210> 35
<211> 39
<212> DNA
<213> Artificial Sequence

<220>
<223> Seq. ID NO 35

<400> 35
gggtggcggt taaccttgct acagaatata agtccaccc                              39

<210> 36
<211> 39
<212> DNA
<213> Artificial Sequence

<220>
<223> Seq. ID NO 36

<400> 36
gcggctcggt taaccttgct acagaatata agtagccgc                              39

<210> 37
<211> 39
<212> DNA
<213> Artificial Sequence

<220>
<223> Seq. ID NO 37

<400> 37
gaccgccggt taaccttgct acagaatata agtgcggtc                              39

<210> 38
<211> 39
<212> DNA
<213> Artificial Sequence

<220>
<223> Seq. ID NO 38

<400> 38

gacggccggt taaccttgct acagaatata agtgccgtc                                39

<210> 39
<211> 39
<212> DNA
<213> Artificial Sequence

<220>
<223> Seq. ID NO 39

<400> 39
ggcccacggt taaccttgct acagaatata agttgggcc                                39

<210> 40
<211> 38
<212> DNA
<213> Artificial Sequence

<220>
<223> Seq. ID NO 40

<400> 40
cgcagcggtt aaccttgcta cagaatataa gtgctgcg                                 38

<210> 41
<211> 22
<212> DNA
<213> Artificial Sequence

<220>
<223> Seq. ID NO 41

<400> 41
gcttaaaccg ggacaaccgt cg                                                  22

<210> 42
<211> 34
<212> DNA
<213> Artificial Sequence

<220>
<223> Seq. ID NO 42

<400> 42
cgcggagctt aaaccgggac aaccgtcgtc cgcg                                     34

<210> 43
<211> 34
<212> DNA
<213> Artificial Sequence

<220>
<223> Seq. ID NO 43

<400> 43
ctcgcggctt aaaccgggac aaccgtcgcg cgag                                    34

<210> 44
<211> 34
<212> DNA
<213> Artificial Sequence

<220>
<223> Seq. ID NO 44

<400> 44
ccgaccgctt aaaccgggac aaccgtcggg tcgg                                    34

<210> 45
<211> 34
<212> DNA
<213> Artificial Sequence

<220>
<223> Seq. ID NO 45

<400> 45
cgacgtgctt aaaccgggac aaccgtcgac gtcg                                    34

<210> 46
<211> 34
<212> DNA
<213> Artificial Sequence

<220>
<223> Seq. ID NO 46

<400> 46
cccaccgctt aaaccgggac aaccgtcggg tggg                                    34

<210> 47
<211> 34
<212> DNA
<213> Artificial Sequence

<220>
<223> Seq. ID NO 47

<400> 47
cgccgagctt aaaccgggac aaccgtcgtc ggcg                                    34

<210> 48
<211> 34
<212> DNA
<213> Artificial Sequence

<220>
<223> Seq. ID NO 48

<400> 48
ctggcggctt aaaccgggac aaccgtcgcg ccag                                    34


<210> 49
<211> 34
<212> DNA
<213> Artificial Sequence


<220>
<223> Seq. ID NO 49

<400> 49
ctgccggctt aaaccgggac aaccgtcgcg gcag                                    34


<210> 50
<211> 34
<212> DNA
<213> Artificial Sequence


<220>
<223> Seq. ID NO 50

<400> 50
ccgggtgctt aaaccgggac aaccgtcgac ccgg                                    34


<210> 51
<211> 34
<212> DNA
<213> Artificial Sequence


<220>
<223> Seq. ID NO 51

<400> 51
gcgcctgctt aaaccgggac aaccgtcgag gcgc                                    34


<210> 52
<211> 34
<212> DNA
<213> Artificial Sequence


<220>
<223> Seq. ID NO 52

<400> 52
gagcgcgctt aaaccgggac aaccgtcggc gctc                                    34


<210> 53
<211> 34
<212> DNA
<213> Artificial Sequence

<220>
<223> Seq. ID NO 53

<400> 53
ggctgggctt aaaccgggac aaccgtcgcc agcc                                    34


<210> 54
<211> 34
<212> DNA
<213> Artificial Sequence


<220>
<223> Seq. ID NO 54

<400> 54
gctgcagctt aaaccgggac aaccgtcgtg cagc                                    34


<210> 55
<211> 34
<212> DNA
<213> Artificial Sequence


<220>
<223> Seq. ID NO 55

<400> 55
gggtgggctt aaaccgggac aaccgtcgcc accc                                    34


<210> 56
<211> 34
<212> DNA
<213> Artificial Sequence


<220>
<223> Seq. ID NO 56

<400> 56
gcggctgctt aaaccgggac aaccgtcgag ccgc                                    34


<210> 57
<211> 34
<212> DNA
<213> Artificial Sequence


<220>
<223> Seq. ID NO 57

<400> 57
gaccgcgctt aaaccgggac aaccgtcggc ggtc                                    34


<210> 58
<211> 34

<212> DNA
<213> Artificial Sequence

<220>
<223> Seq. ID NO 58

<400> 58
gacggcgctt aaaccgggac aaccgtcggc cgtc 34

<210> 59
<211> 34
<212> DNA
<213> Artificial Sequence

<220>
<223> Seq. ID NO 59

<400> 59
ggcccagctt aaaccgggac aaccgtcgtg ggcc 34

<210> 60
<211> 21
<212> DNA
<213> Artificial Sequence

<220>
<223> Seq. ID NO 60

<400> 60
cgtttccagt agttatcccc c 21

<210> 61
<211> 33
<212> DNA
<213> Artificial Sequence

<220>
<223> Seq. ID NO 61

<400> 61
cgcggacgtt tccagtagtt atccccctcc gcg 33

<210> 62
<211> 33
<212> DNA
<213> Artificial Sequence

<220>
<223> Seq. ID NO 62

<400> 62
ctcgcgcgtt tccagtagtt atccccccgc gag 33

<210> 63
<211> 33
<212> DNA
<213> Artificial Sequence

<220>
<223> Seq. ID NO 63

<400> 63
ccgacccgtt tccagtagtt atcccccggt cgg                                    33

<210> 64
<211> 33
<212> DNA
<213> Artificial Sequence

<220>
<223> Seq. ID NO 64

<400> 64
cgacgtcgtt tccagtagtt atcccccacg tcg                                    33

<210> 65
<211> 33
<212> DNA
<213> Artificial Sequence

<220>
<223> Seq. ID No 65

<400> 65
cccacccgtt tccagtagtt atcccccggt ggg                                    33

<210> 66
<211> 33
<212> DNA
<213> Artificial Sequence

<220>
<223> Seq. ID NO 66

<400> 66
cgccgacgtt tccagtagtt atccccctcg gcg                                    33

<210> 67
<211> 33
<212> DNA
<213> Artificial Sequence

<220>
<223> Seq. ID NO 67

<400> 67
ctggcgcgtt tccagtagtt atccccccgc cag                                    33

<210> 68
<211> 33
<212> DNA
<213> Artificial Sequence

<220>
<223> Seq. ID NO 68

<400> 68
ctgccgcgtt tccagtagtt atccccccgg cag                                    33

<210> 69
<211> 33
<212> DNA
<213> Artificial Sequence

<220>
<223> Seq. ID NO 69

<400> 69
ccgggtcgtt tccagtagtt atcccccacc cgg                                    33

<210> 70
<211> 33
<212> DNA
<213> Artificial Sequence

<220>
<223> Seq. ID NO 70

<400> 70
gcgcctcgtt tccagtagtt atcccccagg cgc                                    33

<210> 71
<211> 33
<212> DNA
<213> Artificial Sequence

<220>
<223> Seq. ID NO 71

<400> 71
gagcgccgtt tccagtagtt atcccccgcg ctc                                    33

<210> 72
<211> 33
<212> DNA
<213> Artificial Sequence

<220>
<223> Seq. ID NO 72

<400> 72
ggctggcgtt tccagtagtt atccccccca gcc                                    33

<210> 73
<211> 33
<212> DNA
<213> Artificial Sequence

<220>
<223> Seq. ID NO 73

<400> 73
gctgcacgtt tccagtagtt atccccctgc agc                                   33

<210> 74
<211> 33
<212> DNA
<213> Artificial Sequence

<220>
<223> Seq. ID NO 74

<400> 74
gggtggcgtt tccagtagtt atccccccca ccc                                    33

<210> 75
<211> 33
<212> DNA
<213> Artificial Sequence

<220>
<223> Seq. ID NO 75

<400> 75
gcggctcgtt tccagtagtt atcccccagc cgc                                   33

<210> 76
<211> 33
<212> DNA
<213> Artificial Sequence

<220>
<223> Seq. ID NO 76

<400> 76
gaccgccgtt tccagtagtt atcccccgcg gtc                                   33

<210> 77
<211> 33
<212> DNA
<213> Artificial Sequence

<220>

<223> Seq. ID NO 77

<400> 77
gacggccgtt tccagtagtt atcccccgcc gtc 33

<210> 78
<211> 33
<212> DNA
<213> Artificial Sequence

<220>
<223> Seq. ID NO 78

<400> 78
ggcccacgtt tccagtagtt atccccctgg gcc 33

<210> 79
<211> 18
<212> DNA
<213> Artificial Sequence

<220>
<223> Seq. ID NO 79

<400> 79
gcgaggtcgc ttctcttt 18

<210> 80
<211> 30
<212> DNA
<213> Artificial Sequence

<220>
<223> Seq. ID NO 80

<400> 80
cgcggagcga ggtcgcttct cttttccgcg 30

<210> 81
<211> 30
<212> DNA
<213> Artificial Sequence

<220>
<223> Seq. ID NO 81

<400> 81
ctcgcggcga ggtcgcttct ctttcgcgag 30

<210> 82
<211> 30
<212> DNA
<213> Artificial Sequence

<220>
<223> SEq. ID NO 82

<400> 82
ccgaccgcga ggtcgcttct ctttggtcgg                                        30


<210> 83
<211> 30
<212> DNA
<213> Artificial Sequence


<220>
<223> Seq. ID NO 83

<400> 83
cgacgtgcga ggtcgcttct ctttacgtcg                                        30


<210> 84
<211> 30
<212> DNA
<213> Artificial Sequence


<220>
<223> Seq. ID NO 84

<400> 84
cccaccgcga ggtcgcttct ctttggtggg                                        30


<210> 85
<211> 30
<212> DNA
<213> Artificial Sequence


<220>
<223> Seq. ID NO 85

<400> 85
cgccgagcga ggtcgcttct cttttcggcg                                        30


<210> 86
<211> 30
<212> DNA
<213> Artificial Sequence


<220>
<223> Seq. ID NO 86

<400> 86
ctggcggcga ggtcgcttct ctttcgccag                                        30


<210> 87
<211> 30
<212> DNA

<213> Artificial Sequence

<220>
<223> Seq. ID NO 87

<400> 87
ctgccggcga ggtcgcttct ctttcggcag                                        30

<210> 88
<211> 30
<212> DNA
<213> Artificial Sequence

<220>
<223> Seq. ID NO 88

<400> 88
ccgggtgcga ggtcgcttct ctttacccgg                                        30

<210> 89
<211> 28
<212> DNA
<213> Artificial Sequence

<220>
<223> Seq. ID NO 89

<400> 89
gttggcgagg tcgcttctct ttgccaac                                          28

<210> 90
<211> 30
<212> DNA
<213> Artificial Sequence

<220>
<223> Seq. ID NO 90

<400> 90
gcgcctgcga ggtcgcttct ctttaggcgc                                        30

<210> 91
<211> 30
<212> DNA
<213> Artificial Sequence

<220>
<223> Seq. ID NO 91

<400> 91
gagcgcgcga ggtcgcttct ctttgcgctc                                        30

<210> 92

<211> 30
<212> DNA
<213> Artificial Sequence

<220>
<223> Seq. ID NO 92

<400> 92
ggctgggcga ggtcgcttct ctttccagcc                                        30

<210> 93
<211> 30
<212> DNA
<213> Artificial Sequence

<220>
<223> Seq. ID NO 93

<400> 93
gctgcagcga ggtcgcttct cttttgcagc                                        30

<210> 94
<211> 30
<212> DNA
<213> Artificial Sequence

<220>
<223> Seq. ID NO 94

<400> 94
gggtgggcga ggtcgcttct ctttccaccc                                        30

<210> 95
<211> 30
<212> DNA
<213> Artificial Sequence

<220>
<223> Seq. ID NO 95

<400> 95
gcggctgcga ggtcgcttct ctttagccgc                                        30

<210> 96
<211> 30
<212> DNA
<213> Artificial Sequence

<220>
<223> Seq. ID NO 96

<400> 96
gaccgcgcga ggtcgcttct ctttgcggtc                                        30

<210> 97
<211> 30
<212> DNA
<213> Artificial Sequence

<220>
<223> Seq. ID NO 97

<400> 97
gacggcgcga ggtcgcttct ctttgccgtc                                        30

<210> 98
<211> 30
<212> DNA
<213> Artificial Sequence

<220>
<223> Seq. ID NO 98

<400> 98
ggcccagcga ggtcgcttct cttttgggcc                                        30

<210> 99
<211> 28
<212> DNA
<213> Artificial Sequence

<220>
<223> Seq. ID NO 99

<400> 99
caacgcgagg tcgcttctct ttgcgttg                                          28

<210> 100
<211> 20
<212> DNA
<213> Artificial Sequence

<220>
<223> Seq. ID NO 100

<400> 100
gttgcagcca gctggtatct                                                   20

<210> 101
<211> 32
<212> DNA
<213> Artificial Sequence

<220>
<223> Seq. ID NO 101

<400> 101

cgcggagttg cagccagctg gtatcttccg cg                                    32

<210> 102
<211> 32
<212> DNA
<213> Artificial Sequence

<220>
<223> Seq. ID NO 102

<400> 102
ctcgcggttg cagccagctg gtatctcgcg ag                                    32

<210> 103
<211> 32
<212> DNA
<213> Artificial Sequence

<220>
<223> Seq. ID NO 103

<400> 103
ccgaccgttg cagccagctg gtatctggtc gg                                    32

<210> 104
<211> 32
<212> DNA
<213> Artificial Sequence

<220>
<223> Seq. ID NO 104

<400> 104
cgacgtgttg cagccagctg gtatctacgt cg                                    32

<210> 105
<211> 32
<212> DNA
<213> Artificial Sequence

<220>
<223> Seq. ID NO 105

<400> 105
cccaccgttg cagccagctg gtatctggtg gg                                    32

<210> 106
<211> 32
<212> DNA
<213> Artificial Sequence

<220>
<223> Seq. ID NO 106

<400> 106
cgccgagttg cagccagctg gtatcttcgg cg                                    32

<210> 107
<211> 32
<212> DNA
<213> Artificial Sequence

<220>
<223> Seq. ID NO 107

<400> 107
ctggcggttg cagccagctg gtatctcgcc ag                                    32

<210> 108
<211> 32
<212> DNA
<213> Artificial Sequence

<220>
<223> Seq. ID NO 108

<400> 108
ctgccggttg cagccagctg gtatctcggc ag                                    32

<210> 109
<211> 32
<212> DNA
<213> Artificial Sequence

<220>
<223> Seq. ID NO 109

<400> 109
ccgggtgttg cagccagctg gtatctaccc gg                                    32

<210> 110
<211> 32
<212> DNA
<213> Artificial Sequence

<220>
<223> Seq. ID NO 110

<400> 110
gcgcctgttg cagccagctg gtatctaggc gc                                    32

<210> 111
<211> 32
<212> DNA
<213> Artificial Sequence

<220>
<223> Seq. ID NO 111

<400> 111
gagcgcgttg cagccagctg gtatctgcgc tc                                    32


<210> 112
<211> 32
<212> DNA
<213> Artificial Sequence


<220>
<223> Seq. ID NO 112

<400> 112
ggctgggttg cagccagctg gtatctccag cc                                    32


<210> 113
<211> 32
<212> DNA
<213> Artificial Sequence


<220>
<223> Seq. ID NO 113

<400> 113
gctgcagttg cagccagctg gtatcttgca gc                                    32


<210> 114
<211> 32
<212> DNA
<213> Artificial Sequence


<220>
<223> Seq. ID NO 114

<400> 114
gggtgggttg cagccagctg gtatctccac cc                                    32


<210> 115
<211> 32
<212> DNA
<213> Artificial Sequence


<220>
<223> Seq. ID NO 115

<400> 115
gcggctgttg cagccagctg gtatctagcc gc                                    32


<210> 116
<211> 32
<212> DNA
<213> Artificial Sequence

<220>
<223> Seq. ID NO 116

<400> 116
gaccgcgttg cagccagctg gtatctgcgg tc                                    32


<210> 117
<211> 32
<212> DNA
<213> Artificial Sequence


<220>
<223> Seq. ID NO 117

<400> 117
gacggcgttg cagccagctg gtatctgccg tc                                    32


<210> 118
<211> 32
<212> DNA
<213> Artificial Sequence


<220>
<223> Seq. ID NO 118

<400> 118
ggcccagttg cagccagctg gtatcttggg cc                                    32


<210> 119
<211> 16
<212> DNA
<213> Artificial Sequence


<220>
<223> Seq. ID NO 119

<400> 119
cctcgccggg gttctt                                                      16


<210> 120
<211> 28
<212> DNA
<213> Artificial Sequence


<220>
<223> Seq. ID NO 120

<400> 120
cgcggacctc gccggggttc tttccgcg                                         28


<210> 121
<211> 28

<212> DNA
<213> Artificial Sequence

<220>
<223> Seq. ID NO 121

<400> 121
ctcgcgcctc gccggggttc ttcgcgag                                          28

<210> 122
<211> 28
<212> DNA
<213> Artificial Sequence

<220>
<223> Seq. ID NO 122

<400> 122
ccgacccctc gccggggttc ttggtcgg                                          28

<210> 123
<211> 28
<212> DNA
<213> Artificial Sequence

<220>
<223> Seq. ID NO 123

<400> 123
cgacgtcctc gccggggttc ttacgtcg                                          28

<210> 124
<211> 28
<212> DNA
<213> Artificial Sequence

<220>
<223> Seq. ID NO 124

<400> 124
cccacccctc gccggggttc ttggtggg                                          28

<210> 125
<211> 28
<212> DNA
<213> Artificial Sequence

<220>
<223> Seq. ID NO 125

<400> 125
cgccgacctc gccggggttc tttcggcg                                          28

<210> 126
<211> 28
<212> DNA
<213> Artificial Sequence

<220>
<223> Seq. ID NO 126

<400> 126
ctggcgcctc gccggggttc ttcgccag                                          28

<210> 127
<211> 28
<212> DNA
<213> Artificial Sequence

<220>
<223> Seq. ID NO 127

<400> 127
ctgccgcctc gccggggttc ttcggcag                                          28

<210> 128
<211> 28
<212> DNA
<213> Artificial Sequence

<220>
<223> Seq. ID NO 128

<400> 128
ccgggtcctc gccggggttc ttacccgg                                          28

<210> 129
<211> 28
<212> DNA
<213> Artificial Sequence

<220>
<223> Seq. ID NO 129

<400> 129
gcgcctcctc gccggggttc ttaggcgc                                          28

<210> 130
<211> 28
<212> DNA
<213> Artificial Sequence

<220>
<223> Seq. ID NO 130

<400> 130
gagcgccctc gccggggttc ttgcgctc                                          28

<210> 131
<211> 28
<212> DNA
<213> Artificial Sequence

<220>
<223> Seq. ID NO 131

<400> 131
ggctggcctc gccggggttc ttccagcc                                          28

<210> 132
<211> 28
<212> DNA
<213> Artificial Sequence

<220>
<223> Seq. ID NO 132

<400> 132
gctgcacctc gccggggttc tttgcagc                                          28

<210> 133
<211> 28
<212> DNA
<213> Artificial Sequence

<220>
<223> Seq. ID NO 133

<400> 133
gggtggcctc gccggggttc ttccaccc                                          28

<210> 134
<211> 28
<212> DNA
<213> Artificial Sequence

<220>
<223> Seq. ID NO 134

<400> 134
gcggctcctc gccggggttc ttagccgc                                          28

<210> 135
<211> 28
<212> DNA
<213> Artificial Sequence

<220>
<223> Seq. ID NO 135

<400> 135
gaccgccctc gccggggttc ttgcggtc                                          28

<210> 136
<211> 28
<212> DNA
<213> Artificial Sequence

<220>
<223> Seq. ID NO 136

<400> 136
gacggccctc gccggggttc ttgccgtc                                          28

<210> 137
<211> 28
<212> DNA
<213> Artificial Sequence

<220>
<223> Seq. ID NO 137

<400> 137
ggcccacctc gccggggttc tttgggcc                                          28

<210> 138
<211> 19
<212> DNA
<213> Artificial Sequence

<220>
<223> Seq. ID NO 138

<400> 138
cgctccccta cccaacaac                                                    19

<210> 139
<211> 31
<212> DNA
<213> Artificial Sequence

<220>
<223> Seq. ID NO 139

<400> 139
cgcggacgct cccctaccca acaactccgc g                                      31

<210> 140
<211> 31
<212> DNA
<213> Artificial Sequence

<220>

<223> Seq. ID NO 140

<400> 140
ctcgcgcgct cccctaccca acaaccgcga g                                    31


<210> 141
<211> 31
<212> DNA
<213> Artificial Sequence


<220>
<223> Seq. ID NO 141

<400> 141
ccgacccgct cccctaccca acaacggtcg g                                    31


<210> 142
<211> 31
<212> DNA
<213> Artificial Sequence


<220>
<223> Seq. ID NO 142

<400> 142
cgacgtcgct cccctaccca acaacacgtc g                                    31


<210> 143
<211> 31
<212> DNA
<213> Artificial Sequence


<220>
<223> Seq. ID NO 143

<400> 143
cccacccgct cccctaccca acaacggtgg g                                    31


<210> 144
<211> 31
<212> DNA
<213> Artificial Sequence


<220>
<223> Seq. ID NO 144

<400> 144
cgccgacgct cccctaccca acaactcggc g                                    31


<210> 145
<211> 31
<212> DNA
<213> Artificial Sequence

<220>
<223> Seq. ID NO 145

<400> 145
ctggcgcgct cccctaccca acaaccgcca g                                    31


<210> 146
<211> 31
<212> DNA
<213> Artificial Sequence


<220>
<223> Seq. ID NO 146

<400> 146
ctgccgcgct cccctaccca acaaccggca g                                    31


<210> 147
<211> 31
<212> DNA
<213> Artificial Sequence


<220>
<223> Seq. ID NO 147

<400> 147
ccgggtcgct cccctaccca acaacacccg g                                    31


<210> 148
<211> 31
<212> DNA
<213> Artificial Sequence


<220>
<223> Seq. ID NO 148

<400> 148
gcgcctcgct cccctaccca acaacaggcg c                                    31


<210> 149
<211> 31
<212> DNA
<213> Artificial Sequence


<220>
<223> Seq. ID NO 149

<400> 149
gagcgccgct cccctaccca acaacgcgct c                                    31


<210> 150
<211> 31
<212> DNA

<213> Artificial Sequence

<220>
<223> Seq. ID NO 150

<400> 150
ggctggcgct cccctaccca acaacccagc c                                    31

<210> 151
<211> 31
<212> DNA
<213> Artificial Sequence

<220>
<223> Seq. ID NO 151

<400> 151
gctgcacgct cccctaccca acaactgcag c                                    31

<210> 152
<211> 31
<212> DNA
<213> Artificial Sequence

<220>
<223> Seq. ID NO 152

<400> 152
gggtggcgct cccctaccca acaacccacc c                                    31

<210> 153
<211> 31
<212> DNA
<213> Artificial Sequence

<220>
<223> Seq. ID NO 153

<400> 153
gcggctcgct cccctaccca acaacagccg c                                    31

<210> 154
<211> 31
<212> DNA
<213> Artificial Sequence

<220>
<223> Seq. ID NO 154

<400> 154
gaccgccgct cccctaccca acaacgcggt c                                    31

<210> 155

<211> 31
<212> DNA
<213> Artificial Sequence

<220>
<223> Seq. ID NO 155

<400> 155
gacggccgct cccctaccca acaacgccgt c                                    31

<210> 156
<211> 31
<212> DNA
<213> Artificial Sequence

<220>
<223> Seq. ID NO 156

<400> 156
ggcccacgct cccctaccca acaactgggc c                                    31

<210> 157
<211> 19
<212> DNA
<213> Artificial Sequence

<220>
<223> Seq. ID NO 157

<400> 157
cagcgtgcct tctcccgaa                                                  19

<210> 158
<211> 31
<212> DNA
<213> Artificial Sequence

<220>
<223> Seq. ID NO 158

<400> 158
cgcggacagc gtgccttctc ccgaatccgc g                                    31

<210> 159
<211> 31
<212> DNA
<213> Artificial Sequence

<220>
<223> Seq. ID NO 159

<400> 159
ctcgcgcagc gtgccttctc ccgaacgcga g                                    31

<210> 160
<211> 31
<212> DNA
<213> Artificial Sequence

<220>
<223> Seq. ID NO 160

<400> 160
ccgacccagc gtgccttctc ccgaaggtcg g                                    31

<210> 161
<211> 31
<212> DNA
<213> Artificial Sequence

<220>
<223> Seq. ID NO 161

<400> 161
cgacgtcagc gtgccttctc ccgaaacgtc g                                    31

<210> 162
<211> 31
<212> DNA
<213> Artificial Sequence

<220>
<223> Seq. ID NO 162

<400> 162
cccacccagc gtgccttctc ccgaaggtgg g                                    31

<210> 163
<211> 31
<212> DNA
<213> Artificial Sequence

<220>
<223> Seq. ID NO 163

<400> 163
cgccgacagc gtgccttctc ccgaatcggc g                                    31

<210> 164
<211> 31
<212> DNA
<213> Artificial Sequence

<220>
<223> Seq. ID NO 164

<400> 164

ctggcgcagc gtgccttctc ccgaacgcca g                                    31

<210> 165
<211> 31
<212> DNA
<213> Artificial Sequence

<220>
<223> Seq. ID NO 165

<400> 165
ctgccgcagc gtgccttctc ccgaacggca g                                    31

<210> 166
<211> 31
<212> DNA
<213> Artificial Sequence

<220>
<223> Seq. ID NO 166

<400> 166
ccgggtcagc gtgccttctc ccgaaacccg g                                    31

<210> 167
<211> 31
<212> DNA
<213> Artificial Sequence

<220>
<223> Seq. ID NO 167

<400> 167
gcgcctcagc gtgccttctc ccgaaaggcg c                                    31

<210> 168
<211> 31
<212> DNA
<213> Artificial Sequence

<220>
<223> Seq. ID NO 168

<400> 168
gagcgccagc gtgccttctc ccgaagcgct c                                    31

<210> 169
<211> 31
<212> DNA
<213> Artificial Sequence

<220>
<223> Seq. ID NO 169

<400> 169
ggctggcagc gtgccttctc ccgaaccagc c                                    31

<210> 170
<211> 31
<212> DNA
<213> Artificial Sequence

<220>
<223> Seq. ID NO 170

<400> 170
gctgcacagc gtgccttctc ccgaatgcag c                                    31

<210> 171
<211> 31
<212> DNA
<213> Artificial Sequence

<220>
<223> Seq. ID NO 171

<400> 171
gggtggcagc gtgccttctc ccgaaccacc c                                    31

<210> 172
<211> 31
<212> DNA
<213> Artificial Sequence

<220>
<223> Seq. ID NO 172

<400> 172
gcggctcagc gtgccttctc ccgaaagccg c                                    31

<210> 173
<211> 31
<212> DNA
<213> Artificial Sequence

<220>
<223> Seq. ID NO 173

<400> 173
gaccgccagc gtgccttctc ccgaagcggt c                                    31

<210> 174
<211> 31
<212> DNA
<213> Artificial Sequence

<220>
<223> Seq. ID NO 174

<400> 174
gacggccagc gtgccttctc ccgaagccgt c                                    31


<210> 175
<211> 31
<212> DNA
<213> Artificial Sequence


<220>
<223> Seq. ID NO 175

<400> 175
ggcccacagc gtgccttctc ccgaatgggc c                                    31


<210> 176
<211> 20
<212> DNA
<213> Artificial Sequence


<220>
<223> Seq. ID NO 176

<400> 176
ctgccgcagc ttcggtgcat                                                 20


<210> 177
<211> 27
<212> DNA
<213> Artificial Sequence


<220>
<223> Seq. ID NO 177

<400> 177
gctgccgcag cttcggtgca tggcagc                                         27


<210> 178
<211> 32
<212> DNA
<213> Artificial Sequence


<220>
<223> Seq. ID NO 178

<400> 178
cgcggactgc cgcagcttcg gtgcattccg cg                                    32


<210> 179
<211> 32
<212> DNA
<213> Artificial Sequence

<220>
<223> Seq. ID NO 179

<400> 179
ctcgcgctgc cgcagcttcg gtgcatcgcg ag                                    32


<210> 180
<211> 32
<212> DNA
<213> Artificial Sequence


<220>
<223> Seq. ID NO 180

<400> 180
ccgaccctgc cgcagcttcg gtgcatggtc gg                                    32


<210> 181
<211> 32
<212> DNA
<213> Artificial Sequence


<220>
<223> Seq. ID NO 181

<400> 181
cgacgtctgc cgcagcttcg gtgcatacgt cg                                    32


<210> 182
<211> 32
<212> DNA
<213> Artificial Sequence


<220>
<223> Seq. ID NO 182

<400> 182
cccaccctgc cgcagcttcg gtgcatggtg gg                                    32


<210> 183
<211> 32
<212> DNA
<213> Artificial Sequence


<220>
<223> Seq. ID NO 183

<400> 183
cgccgactgc cgcagcttcg gtgcattcgg cg                                    32


<210> 184
<211> 32

<212> DNA
<213> Artificial Sequence

<220>
<223> Seq. ID NO 184

<400> 184
ctggcgctgc cgcagcttcg gtgcatcgcc ag                                    32

<210> 185
<211> 32
<212> DNA
<213> Artificial Sequence

<220>
<223> Seq. ID NO 185

<400> 185
ctgccgctgc cgcagcttcg gtgcatcggc ag                                    32

<210> 186
<211> 32
<212> DNA
<213> Artificial Sequence

<220>
<223> Seq. ID NO 186

<400> 186
ccgggtctgc cgcagcttcg gtgcataccc gg                                    32

<210> 187
<211> 27
<212> DNA
<213> Artificial Sequence

<220>
<223> Seq. ID NO 187

<400> 187
cctgccgcag cttcggtgca tggcagg                                          27

<210> 188
<211> 32
<212> DNA
<213> Artificial Sequence

<220>
<223> Seq. ID NO 188

<400> 188
gcgcctctgc cgcagcttcg gtgcataggc gc                                    32

<210> 189
<211> 32
<212> DNA
<213> Artificial Sequence

<220>
<223> Seq. ID NO 189

<400> 189
gagcgcctgc cgcagcttcg gtgcatgcgc tc                                    32

<210> 190
<211> 32
<212> DNA
<213> Artificial Sequence

<220>
<223> Seq. ID NO 190

<400> 190
ggctggctgc cgcagcttcg gtgcatccag cc                                    32

<210> 191
<211> 32
<212> DNA
<213> Artificial Sequence

<220>
<223> Seq. ID NO 191

<400> 191
gctgcactgc cgcagcttcg gtgcattgca gc                                    32

<210> 192
<211> 32
<212> DNA
<213> Artificial Sequence

<220>
<223> Seq. ID NO 192

<400> 192
gggtggctgc cgcagcttcg gtgcatccac cc                                    32

<210> 193
<211> 32
<212> DNA
<213> Artificial Sequence

<220>
<223> Seq. ID NO 193

<400> 193
gcggctctgc cgcagcttcg gtgcatagcc gc                                    32

<210> 194
<211> 32
<212> DNA
<213> Artificial Sequence

<220>
<223> Seq. ID NO 194

<400> 194
gaccgcctgc cgcagcttcg gtgcatgcgg tc                                    32

<210> 195
<211> 32
<212> DNA
<213> Artificial Sequence

<220>
<223> Seq. ID NO 195

<400> 195
gacggcctgc cgcagcttcg gtgcatgccg tc                                    32

<210> 196
<211> 32
<212> DNA
<213> Artificial Sequence

<220>
<223> Seq. ID NO 196

<400> 196
ggcccactgc cgcagcttcg gtgcattggg cc                                    32

<210> 197
<211> 24
<212> DNA
<213> Artificial Sequence

<220>
<223> Seq. ID NO 197

<400> 197
gtgtgtctcc cgtgataaca ttct                                             24

<210> 198
<211> 36
<212> DNA
<213> Artificial Sequence

<220>
<223> Seq. ID NO 198

<400> 198
cgcggagtgt gtctcccgtg ataacattct tccgcg                                36

<210> 199
<211> 36
<212> DNA
<213> Artificial Sequence

<220>
<223> Seq. ID NO 199

<400> 199
ctcgcggtgt gtctcccgtg ataacattct cgcgag                                36

<210> 200
<211> 36
<212> DNA
<213> Artificial Sequence

<220>
<223> Seq. ID NO 200

<400> 200
ccgaccgtgt gtctcccgtg ataacattct ggtcgg                                36

<210> 201
<211> 36
<212> DNA
<213> Artificial Sequence

<220>
<223> Seq. ID NO 201

<400> 201
cgacgtgtgt gtctcccgtg ataacattct acgtcg                                36

<210> 202
<211> 36
<212> DNA
<213> Artificial Sequence

<220>
<223> Seq. ID NO 202

<400> 202
cccaccgtgt gtctcccgtg ataacattct ggtggg                                36

<210> 203
<211> 36
<212> DNA
<213> Artificial Sequence

<220>

<223> Seq. ID NO 203

<400> 203
cgccgagtgt gtctcccgtg ataacattct tcggcg                                36


<210> 204
<211> 36
<212> DNA
<213> Artificial Sequence


<220>
<223> Seq. ID NO 204

<400> 204
ctggcggtgt gtctcccgtg ataacattct cgccag                                36


<210> 205
<211> 36
<212> DNA
<213> Artificial Sequence


<220>
<223> Seq. ID NO 205

<400> 205
ctgccggtgt gtctcccgtg ataacattct cggcag                                36


<210> 206
<211> 36
<212> DNA
<213> Artificial Sequence


<220>
<223> Seq. ID NO 206

<400> 206
ccgggtgtgt gtctcccgtg ataacattct acccgg                                36


<210> 207
<211> 35
<212> DNA
<213> Artificial Sequence


<220>
<223> Seq. ID NO 207

<400> 207
gaccggtgtg tctcccgtga taacattctc cggtc                                 35


<210> 208
<211> 36
<212> DNA
<213> Artificial Sequence

<220>
<223> Seq. ID NO 208

<400> 208
gcgcctgtgt gtctcccgtg ataacattct aggcgc                                36


<210> 209
<211> 36
<212> DNA
<213> Artificial Sequence


<220>
<223> Seq. ID NO 209

<400> 209
gagcgcgtgt gtctcccgtg ataacattct gcgctc                                36


<210> 210
<211> 36
<212> DNA
<213> Artificial Sequence


<220>
<223> Seq. ID NO 210

<400> 210
ggctgggtgt gtctcccgtg ataacattct ccagcc                                36


<210> 211
<211> 36
<212> DNA
<213> Artificial Sequence


<220>
<223> Seq. ID NO 211

<400> 211
gctgcagtgt gtctcccgtg ataacattct tgcagc                                36


<210> 212
<211> 36
<212> DNA
<213> Artificial Sequence


<220>
<223> Seq. ID NO 212

<400> 212
gggtgggtgt gtctcccgtg ataacattct ccaccc                                36


<210> 213
<211> 36
<212> DNA

<213> Artificial Sequence

<220>
<223> Seq. ID NO 213

<400> 213
gcggctgtgt gtctcccgtg ataacattct agccgc                                36

<210> 214
<211> 36
<212> DNA
<213> Artificial Sequence

<220>
<223> Seq. ID NO 214

<400> 214
gaccgcgtgt gtctcccgtg ataacattct gcggtc                                36

<210> 215
<211> 36
<212> DNA
<213> Artificial Sequence

<220>
<223> Seq. ID NO 215

<400> 215
gacggcgtgt gtctcccgtg ataacattct gccgtc                                36

<210> 216
<211> 36
<212> DNA
<213> Artificial Sequence

<220>
<223> Seq. ID NO 216

<400> 216
ggcccagtgt gtctcccgtg ataacattct tgggcc                                36

<210> 217
<211> 35
<212> DNA
<213> Artificial Sequence

<220>
<223> Seq. ID NO 217

<400> 217
ctggcgtgtg tctcccgtga taacattctc gccag                                 35

<210> 218

<211> 21
<212> DNA
<213> Artificial Sequence

<220>
<223> Seq. ID NO 218

<400> 218
ttcgtgtttg cacagtgctg t                                                21

<210> 219
<211> 33
<212> DNA
<213> Artificial Sequence

<220>
<223> Seq. ID NO 219

<400> 219
cgcggattcg tgtttgcaca gtgctgttcc gcg                                   33

<210> 220
<211> 33
<212> DNA
<213> Artificial Sequence

<220>
<223> Seq. ID NO 220

<400> 220
ctcgcgttcg tgtttgcaca gtgctgtcgc gag                                   33

<210> 221
<211> 33
<212> DNA
<213> Artificial Sequence

<220>
<223> Seq. ID NO 221

<400> 221
ccgaccttcg tgtttgcaca gtgctgtggt cgg                                   33

<210> 222
<211> 33
<212> DNA
<213> Artificial Sequence

<220>
<223> Seq. ID NO 222

<400> 222
cgacgtttcg tgtttgcaca gtgctgtacg tcg                                   33

<210> 223
<211> 33
<212> DNA
<213> Artificial Sequence

<220>
<223> Seq. ID NO 223

<400> 223
cccaccttcg tgtttgcaca gtgctgtggt ggg                                    33

<210> 224
<211> 33
<212> DNA
<213> Artificial Sequence

<220>
<223> Seq. ID NO 224

<400> 224
cgccgattcg tgtttgcaca gtgctgttcg gcg                                    33

<210> 225
<211> 33
<212> DNA
<213> Artificial Sequence

<220>
<223> Seq. ID NO 225

<400> 225
ctggcgttcg tgtttgcaca gtgctgtcgc cag                                    33

<210> 226
<211> 33
<212> DNA
<213> Artificial Sequence

<220>
<223> Seq. ID NO 226

<400> 226
ctgccgttcg tgtttgcaca gtgctgtcgg cag                                    33

<210> 227
<211> 33
<212> DNA
<213> Artificial Sequence

<220>
<223> Seq. ID NO 227

<400> 227

ccgggtttcg tgtttgcaca gtgctgtacc cgg 33

<210> 228
<211> 33
<212> DNA
<213> Artificial Sequence

<220>
<223> SEq. ID NO 228

<400> 228
gcgcctttcg tgtttgcaca gtgctgtagg cgc 33

<210> 229
<211> 33
<212> DNA
<213> Artificial Sequence

<220>
<223> Seq. ID NO 229

<400> 229
gagcgcttcg tgtttgcaca gtgctgtgcg ctc 33

<210> 230
<211> 33
<212> DNA
<213> Artificial Sequence

<220>
<223> Seq. ID NO 230

<400> 230
ggctggttcg tgtttgcaca gtgctgtcca gcc 33

<210> 231
<211> 33
<212> DNA
<213> Artificial Sequence

<220>
<223> Seq. ID NO 231

<400> 231
gctgcattcg tgtttgcaca gtgctgttgc agc 33

<210> 232
<211> 33
<212> DNA
<213> Artificial Sequence

<220>
<223> Seq. ID NO 232

<400> 232
gggtggttcg tgtttgcaca gtgctgtcca ccc                                    33

<210> 233
<211> 33
<212> DNA
<213> Artificial Sequence

<220>
<223> Seq. ID NO 233

<400> 233
gcggctttcg tgtttgcaca gtgctgtagc cgc                                    33

<210> 234
<211> 33
<212> DNA
<213> Artificial Sequence

<220>
<223> Seq. ID NO 234

<400> 234
gaccgcttcg tgtttgcaca gtgctgtgcg gtc                                    33

<210> 235
<211> 33
<212> DNA
<213> Artificial Sequence

<220>
<223> Seq. ID NO 235

<400> 235
gacggcttcg tgtttgcaca gtgctgtgcc gtc                                    33

<210> 236
<211> 33
<212> DNA
<213> Artificial Sequence

<220>
<223> Seq. ID NO 236

<400> 236
ggcccattcg tgtttgcaca gtgctgttgg gcc                                    33

<210> 237
<211> 19
<212> DNA
<213> Artificial Sequence

<220>
<223> Seq. ID NO 237

<400> 237
gctcttcctc cgttcgttc                                                    19


<210> 238
<211> 31
<212> DNA
<213> Artificial Sequence


<220>
<223> Seq. ID NO_238

<400> 238
cgcggagctc ttcctccgtt cgttctccgc g                                      31


<210> 239
<211> 31
<212> DNA
<213> Artificial Sequence


<220>
<223> Seq. ID NO_239

<400> 239
ctcgcggctc ttcctccgtt cgttccgcga g                                      31


<210> 240
<211> 31
<212> DNA
<213> Artificial Sequence


<220>
<223> Seq. ID NO_240

<400> 240
ccgaccgctc ttcctccgtt cgttcggtcg g                                      31


<210> 241
<211> 31
<212> DNA
<213> Artificial Sequence


<220>
<223> Seq. ID NO_241

<400> 241
cgacgtgctc ttcctccgtt cgttcacgtc g                                      31


<210> 242
<211> 31
<212> DNA
<213> Artificial Sequence

<220>
<223> Seq. ID NO_242

<400> 242
cccaccgctc ttcctccgtt cgttcggtgg g                                    31


<210> 243
<211> 31
<212> DNA
<213> Artificial Sequence


<220>
<223> Seq. ID NO_243

<400> 243
cgccgagctc ttcctccgtt cgttctcggc g                                    31


<210> 244
<211> 31
<212> DNA
<213> Artificial Sequence


<220>
<223> Seq. ID NO_244

<400> 244
ctggcggctc ttcctccgtt cgttccgcca g                                    31


<210> 245
<211> 31
<212> DNA
<213> Artificial Sequence


<220>
<223> Seq. ID NO_245

<400> 245
ccgcgcgctc ttcctccgtt cgttcgcgcg g                                    31


<210> 246
<211> 31
<212> DNA
<213> Artificial Sequence


<220>
<223> Seq. ID NO_246

<400> 246
ctgccggctc ttcctccgtt cgttccggca g                                    31


<210> 247
<211> 31

<212> DNA
<213> Artificial Sequence

<220>
<223> Seq. ID NO_247

<400> 247
ccgggtgctc ttcctccgtt cgttcacccg g                                    31

<210> 248
<211> 31
<212> DNA
<213> Artificial Sequence

<220>
<223> Seq. ID NO_248

<400> 248
gcgcctgctc ttcctccgtt cgttcaggcg c                                    31

<210> 249
<211> 31
<212> DNA
<213> Artificial Sequence

<220>
<223> Seq. ID NO_249

<400> 249
gagcgcgctc ttcctccgtt cgttcgcgct c                                    31

<210> 250
<211> 31
<212> DNA
<213> Artificial Sequence

<220>
<223> Seq. ID NO_250

<400> 250
ggctgggctc ttcctccgtt cgttcccagc c                                    31

<210> 251
<211> 31
<212> DNA
<213> Artificial Sequence

<220>
<223> Seq. ID NO_251

<400> 251
gctgcagctc ttcctccgtt cgttctgcag c                                    31

<210> 252
<211> 31
<212> DNA
<213> Artificial Sequence

<220>
<223> Seq. ID NO_252

<400> 252
gggtgggctc ttcctccgtt cgttcccacc c                                    31

<210> 253
<211> 31
<212> DNA
<213> Artificial Sequence

<220>
<223> Seq. ID NO_253

<400> 253
gcggctgctc ttcctccgtt cgttcagccg c                                    31

<210> 254
<211> 31
<212> DNA
<213> Artificial Sequence

<220>
<223> Seq. ID NO_254

<400> 254
gaccgcgctc ttcctccgtt cgttcgcggt c                                    31

<210> 255
<211> 31
<212> DNA
<213> Artificial Sequence

<220>
<223> Seq. ID NO_255

<400> 255
gacggcgctc ttcctccgtt cgttcgccgt c                                    31

<210> 256
<211> 31
<212> DNA
<213> Artificial Sequence

<220>
<223> Seq. ID NO_256

<400> 256
ggcccagctc ttcctccgtt cgttctgggc c                                    31

<210> 257
<211> 18
<212> DNA
<213> Artificial Sequence

<220>
<223> Seq. ID NO_257

<400> 257
agcaagctct tcctccgt                                                    18

<210> 258
<211> 30
<212> DNA
<213> Artificial Sequence

<220>
<223> Seq. ID NO_258

<400> 258
cgcggaagca agctcttcct ccgttccgcg                                       30

<210> 259
<211> 30
<212> DNA
<213> Artificial Sequence

<220>
<223> Seq. ID NO_259

<400> 259
ctcgcgagca agctcttcct ccgtcgcgag                                       30

<210> 260
<211> 30
<212> DNA
<213> Artificial Sequence

<220>
<223> Seq. ID NO_260

<400> 260
ccgaccagca agctcttcct ccgtggtcgg                                       30

<210> 261
<211> 30
<212> DNA
<213> Artificial Sequence

<220>
<223> Seq. ID NO_261

<400> 261
cgacgtagca agctcttcct ccgtacgtcg                                        30

<210> 262
<211> 30
<212> DNA
<213> Artificial Sequence

<220>
<223> Seq. ID NO_262

<400> 262
cccaccagca agctcttcct ccgtggtggg                                        30

<210> 263
<211> 30
<212> DNA
<213> Artificial Sequence

<220>
<223> Seq. ID NO_263

<400> 263
cgccgaagca agctcttcct ccgttcggcg                                        30

<210> 264
<211> 30
<212> DNA
<213> Artificial Sequence

<220>
<223> Seq. ID NO_264

<400> 264
ctggcgagca agctcttcct ccgtcgccag                                        30

<210> 265
<211> 30
<212> DNA
<213> Artificial Sequence

<220>
<223> Seq. ID NO_265

<400> 265
ctgccgagca agctcttcct ccgtcggcag                                        30

<210> 266
<211> 30
<212> DNA
<213> Artificial Sequence

<220>

<223> Seq. ID NO_266

<400> 266
ccgggtagca agctcttcct ccgtacccgg                                        30


<210> 267
<211> 30
<212> DNA
<213> Artificial Sequence


<220>
<223> Seq. ID NO_267

<400> 267
gcgcctagca agctcttcct ccgtaggcgc                                        30


<210> 268
<211> 30
<212> DNA
<213> Artificial Sequence


<220>
<223> Seq. ID NO_268

<400> 268
gagcgcagca agctcttcct ccgtgcgctc                                        30


<210> 269
<211> 30
<212> DNA
<213> Artificial Sequence


<220>
<223> Seq. ID NO_269

<400> 269
ggctggagca agctcttcct ccgtccagcc                                        30


<210> 270
<211> 30
<212> DNA
<213> Artificial Sequence


<220>
<223> Seq. ID NO_270

<400> 270
gctgcaagca agctcttcct ccgttgcagc                                        30


<210> 271
<211> 30
<212> DNA
<213> Artificial Sequence

<220>
<223> Seq. ID NO_271

<400> 271
gggtggagca agctcttcct ccgtccaccc                                        30


<210> 272
<211> 30
<212> DNA
<213> Artificial Sequence


<220>
<223> Seq. ID NO_272

<400> 272
gcggctagca agctcttcct ccgtagccgc                                        30


<210> 273
<211> 30
<212> DNA
<213> Artificial Sequence


<220>
<223> Seq. ID NO_273

<400> 273
gaccgcagca agctcttcct ccgtgcggtc                                        30


<210> 274
<211> 30
<212> DNA
<213> Artificial Sequence


<220>
<223> Seq. ID NO_274

<400> 274
gacggcagca agctcttcct ccgtgccgtc                                        30


<210> 275
<211> 30
<212> DNA
<213> Artificial Sequence


<220>
<223> Seq. ID NO_275

<400> 275
ggcccaagca agctcttcct ccgttgggcc                                        30


<210> 276
<211> 20
<212> DNA

<213> Artificial Sequence

<220>
<223> Seq. ID NO_276

<400> 276
gccactaact ttggaagagc                                                    20

<210> 277
<211> 32
<212> DNA
<213> Artificial Sequence

<220>
<223> Seq. ID NO_277

<400> 277
cgcggagcca ctaactttgg aagagctccg cg                                      32

<210> 278
<211> 32
<212> DNA
<213> Artificial Sequence

<220>
<223> Seq. ID NO_278

<400> 278
ctcgcggcca ctaactttgg aagagccgcg ag                                      32

<210> 279
<211> 32
<212> DNA
<213> Artificial Sequence

<220>
<223> Seq. ID NO_279

<400> 279
ccgaccgcca ctaactttgg aagagcggtc gg                                      32

<210> 280
<211> 32
<212> DNA
<213> Artificial Sequence

<220>
<223> Seq. ID NO_280

<400> 280
cgacgtgcca ctaactttgg aagagcacgt cg                                      32

<210> 281

<211> 32
<212> DNA
<213> Artificial Sequence

<220>
<223> Seq. ID NO_281

<400> 281
cccaccgcca ctaactttgg aagagcggtg gg                                    32

<210> 282
<211> 32
<212> DNA
<213> Artificial Sequence

<220>
<223> Seq. ID NO_282

<400> 282
cgccgagcca ctaactttgg aagagctcgg cg                                    32

<210> 283
<211> 32
<212> DNA
<213> Artificial Sequence

<220>
<223> Seq. ID NO_283

<400> 283
ctggcggcca ctaactttgg aagagccgcc ag                                    32

<210> 284
<211> 32
<212> DNA
<213> Artificial Sequence

<220>
<223> Seq. ID NO_284

<400> 284
ctgccggcca ctaactttgg aagagccggc ag                                    32

<210> 285
<211> 32
<212> DNA
<213> Artificial Sequence

<220>
<223> Seq. ID NO_285

<400> 285
ccgggtgcca ctaactttgg aagagcaccc gg                                    32

<210> 286
<211> 32
<212> DNA
<213> Artificial Sequence

<220>
<223> Seq. ID NO_286

<400> 286
gcgcctgcca ctaactttgg aagagcaggc gc                                    32

<210> 287
<211> 32
<212> DNA
<213> Artificial Sequence

<220>
<223> Seq. ID NO_287

<400> 287
gagcgcgcca ctaactttgg aagagcgcgc tc                                    32

<210> 288
<211> 32
<212> DNA
<213> Artificial Sequence

<220>
<223> Seq. ID NO_288

<400> 288
ggctgggcca ctaactttgg aagagcccag cc                                    32

<210> 289
<211> 32
<212> DNA
<213> Artificial Sequence

<220>
<223> Seq. ID NO_289

<400> 289
gctgcagcca ctaactttgg aagagctgca gc                                    32

<210> 290
<211> 32
<212> DNA
<213> Artificial Sequence

<220>
<223> Seq. ID NO_290

<400> 290

gggtgggcca ctaactttgg aagagcccac cc 32

<210> 291
<211> 32
<212> DNA
<213> Artificial Sequence

<220>
<223> Seq. ID NO_291

<400> 291
gcggctgcca ctaactttgg aagagcagcc gc 32

<210> 292
<211> 32
<212> DNA
<213> Artificial Sequence

<220>
<223> Seq. ID NO_292

<400> 292
gaccgcgcca ctaactttgg aagagcgcgg tc 32

<210> 293
<211> 32
<212> DNA
<213> Artificial Sequence

<220>
<223> Seq. ID NO_293

<400> 293
gacggcgcca ctaactttgg aagagcgccg tc 32

<210> 294
<211> 32
<212> DNA
<213> Artificial Sequence

<220>
<223> Seq. ID NO_294

<400> 294
ggcccagcca ctaactttgg aagagctggg cc 32

<210> 295
<211> 19
<212> DNA
<213> Artificial Sequence

<220>
<223> Seq. ID NO_295

<400> 295
atccccaact tacaggcag                                                    19

<210> 296
<211> 31
<212> DNA
<213> Artificial Sequence

<220>
<223> Seq. ID NO_296

<400> 296
cgcggaatcc ccaacttaca ggcagtccgc g                                      31

<210> 297
<211> 31
<212> DNA
<213> Artificial Sequence

<220>
<223> Seq. ID NO_297

<400> 297
ctcgcgatcc ccaacttaca ggcagcgcga g                                      31

<210> 298
<211> 31
<212> DNA
<213> Artificial Sequence

<220>
<223> Seq. ID NO_298

<400> 298
ccgcgcatcc ccaacttaca ggcaggcgcg g                                      31

<210> 299
<211> 31
<212> DNA
<213> Artificial Sequence

<220>
<223> Seq. ID NO_299

<400> 299
ccgaccatcc ccaacttaca ggcagggtcg g                                      31

<210> 300
<211> 31
<212> DNA
<213> Artificial Sequence

<220>
<223> Seq. ID NO_300

<400> 300
cgacgtatcc ccaacttaca ggcagacgtc g                                    31


<210> 301
<211> 31
<212> DNA
<213> Artificial Sequence


<220>
<223> Seq. ID NO_301

<400> 301
cccaccatcc ccaacttaca ggcagggtgg g                                    31


<210> 302
<211> 31
<212> DNA
<213> Artificial Sequence


<220>
<223> Seq. ID NO_302

<400> 302
cgccgaatcc ccaacttaca ggcagtcggc g                                    31


<210> 303
<211> 31
<212> DNA
<213> Artificial Sequence


<220>
<223> Seq. ID NO_303

<400> 303
ctggcgatcc ccaacttaca ggcagcgcca g                                    31


<210> 304
<211> 31
<212> DNA
<213> Artificial Sequence


<220>
<223> Seq. ID NO_304

<400> 304
ctgccgatcc ccaacttaca ggcagcggca g                                    31


<210> 305
<211> 31
<212> DNA
<213> Artificial Sequence

<220>
<223> Seq. ID NO_305

<400> 305
ccgggtatcc ccaacttaca ggcagacccg g                                    31


<210> 306
<211> 31
<212> DNA
<213> Artificial Sequence


<220>
<223> Seq. ID NO_306

<400> 306
gcgcctatcc ccaacttaca ggcagaggcg c                                    31


<210> 307
<211> 31
<212> DNA
<213> Artificial Sequence


<220>
<223> Seq. ID NO_307

<400> 307
gagcgcatcc ccaacttaca ggcaggcgct c                                    31


<210> 308
<211> 31
<212> DNA
<213> Artificial Sequence


<220>
<223> Seq. ID NO_308

<400> 308
ggctggatcc ccaacttaca ggcagccagc c                                    31


<210> 309
<211> 31
<212> DNA
<213> Artificial Sequence


<220>
<223> Seq. ID NO_309

<400> 309
gctgcaatcc ccaacttaca ggcagtgcag c                                    31


<210> 310
<211> 31

<212> DNA
<213> Artificial Sequence

<220>
<223> Seq. ID NO_310

<400> 310
gggtggatcc ccaacttaca ggcagccacc c 31

<210> 311
<211> 31
<212> DNA
<213> Artificial Sequence

<220>
<223> Seq. ID NO_311

<400> 311
gcggctatcc ccaacttaca ggcagagccg c 31

<210> 312
<211> 31
<212> DNA
<213> Artificial Sequence

<220>
<223> Seq. ID NO_312

<400> 312
gaccgcatcc ccaacttaca ggcaggcggt c 31

<210> 313
<211> 31
<212> DNA
<213> Artificial Sequence

<220>
<223> Seq. ID NO_313

<400> 313
gacggcatcc ccaacttaca ggcaggccgt c 31

<210> 314
<211> 31
<212> DNA
<213> Artificial Sequence

<220>
<223> Seq. ID NO_314

<400> 314
ggcccaatcc ccaacttaca ggcagtgggc c 31

<210> 315
<211> 21
<212> DNA
<213> Artificial Sequence

<220>
<223> Seq. ID NO_315

<400> 315
ctctatcatt cggtattagc c                                                21

<210> 316
<211> 33
<212> DNA
<213> Artificial Sequence

<220>
<223> Seq. ID NO_316

<400> 316
cgcggactct atcattcggt attagcctcc gcg                                   33

<210> 317
<211> 33
<212> DNA
<213> Artificial Sequence

<220>
<223> Seq. ID NO_317

<400> 317
ctcgcgctct atcattcggt attagcccgc gag                                   33

<210> 318
<211> 33
<212> DNA
<213> Artificial Sequence

<220>
<223> Seq. ID NO_318

<400> 318
ccgaccctct atcattcggt attagccggt cgg                                   33

<210> 319
<211> 33
<212> DNA
<213> Artificial Sequence

<220>
<223> Seq. ID NO_319

<400> 319
cgacgtctct atcattcggt attagccacg tcg                                   33

<210> 320
<211> 33
<212> DNA
<213> Artificial Sequence

<220>
<223> Seq. ID NO_320

<400> 320
cccaccctct atcattcggt attagccggt ggg                                    33

<210> 321
<211> 33
<212> DNA
<213> Artificial Sequence

<220>
<223> Seq. ID NO_321

<400> 321
cgccgactct atcattcggt attagcctcg gcg                                    33

<210> 322
<211> 33
<212> DNA
<213> Artificial Sequence

<220>
<223> Seq. ID NO_322

<400> 322
ctggcgctct atcattcggt attagcccgc cag                                    33

<210> 323
<211> 33
<212> DNA
<213> Artificial Sequence

<220>
<223> Seq. ID NO_323

<400> 323
ctgccgctct atcattcggt attagcccgg cag                                    33

<210> 324
<211> 33
<212> DNA
<213> Artificial Sequence

<220>
<223> Seq. ID NO_324

<400> 324
ccgggtctct atcattcggt attagccacc cgg                                    33

<210> 325
<211> 33
<212> DNA
<213> Artificial Sequence

<220>
<223> Seq. ID NO_325

<400> 325
gcgcctctct atcattcggt attagccagg cgc                                    33

<210> 326
<211> 33
<212> DNA
<213> Artificial Sequence

<220>
<223> Seq. ID NO_326

<400> 326
gagcgcctct atcattcggt attagccgcg ctc                                    33

<210> 327
<211> 33
<212> DNA
<213> Artificial Sequence

<220>
<223> Seq. ID NO_327

<400> 327
ggctggctct atcattcggt attagcccca gcc                                    33

<210> 328
<211> 33
<212> DNA
<213> Artificial Sequence

<220>
<223> Seq. ID NO_328

<400> 328
gctgcactct atcattcggt attagcctgc agc                                    33

<210> 329
<211> 33
<212> DNA
<213> Artificial Sequence

<220>

<223> Seq. ID NO_329

<400> 329
gggtggctct atcattcggt attagcccca ccc                                    33


<210> 330
<211> 33
<212> DNA
<213> Artificial Sequence


<220>
<223> Seq. ID NO_330

<400> 330
gcggctctct atcattcggt attagccagc cgc                                    33


<210> 331
<211> 33
<212> DNA
<213> Artificial Sequence


<220>
<223> Seq. ID NO_331

<400> 331
gaccgcctct atcattcggt attagccgcg gtc                                    33


<210> 332
<211> 33
<212> DNA
<213> Artificial Sequence


<220>
<223> Seq. ID NO_332

<400> 332
gacggcctct atcattcggt attagccgcc gtc                                    33


<210> 333
<211> 33
<212> DNA
<213> Artificial Sequence


<220>
<223> Seq. ID NO_333

<400> 333
ggcccactct atcattcggt attagcctgg gcc                                    33


<210> 334
<211> 19
<212> DNA
<213> Artificial Sequence

<220>
<223> Seq. ID NO_334

<400> 334
gcatgcgcca cactttatg                                                    19


<210> 335
<211> 31
<212> DNA
<213> Artificial Sequence


<220>
<223> Seq. ID NO_335

<400> 335
cgcggagcat gcgccacact ttatgtccgc g                                      31


<210> 336
<211> 31
<212> DNA
<213> Artificial Sequence


<220>
<223> Seq. ID NO_336

<400> 336
ctcgcggcat gcgccacact ttatgcgcga g                                      31


<210> 337
<211> 31
<212> DNA
<213> Artificial Sequence


<220>
<223> Seq. ID NO_337

<400> 337
ccgaccgcat gcgccacact ttatgggtcg g                                      31


<210> 338
<211> 31
<212> DNA
<213> Artificial Sequence


<220>
<223> Seq. ID NO_338

<400> 338
cgacgtgcat gcgccacact ttatgacgtc g                                      31


<210> 339
<211> 31
<212> DNA

<213> Artificial Sequence

<220>
<223> Seq. ID NO_339

<400> 339
cccaccgcat gcgccacact ttatgggtgg g                                    31

<210> 340
<211> 31
<212> DNA
<213> Artificial Sequence

<220>
<223> Seq. ID NO_340

<400> 340
cgccgagcat gcgccacact ttatgtcggc g                                    31

<210> 341
<211> 31
<212> DNA
<213> Artificial Sequence

<220>
<223> Seq. ID NO_341

<400> 341
ctggcggcat gcgccacact ttatgcgcca g                                    31

<210> 342
<211> 31
<212> DNA
<213> Artificial Sequence

<220>
<223> Seq. ID NO_342

<400> 342
ctgccggcat gcgccacact ttatgcggca g                                    31

<210> 343
<211> 31
<212> DNA
<213> Artificial Sequence

<220>
<223> Seq. ID NO_343

<400> 343
ccgggtgcat gcgccacact ttatgacccg g                                    31

<210> 344

<211> 31
<212> DNA
<213> Artificial Sequence

<220>
<223> Seq. ID NO_344

<400> 344
gcgcctgcat gcgccacact ttatgaggcg c                                    31

<210> 345
<211> 31
<212> DNA
<213> Artificial Sequence

<220>
<223> Seq. ID NO_345

<400> 345
gagcgcgcat gcgccacact ttatggcgct c                                    31

<210> 346
<211> 31
<212> DNA
<213> Artificial Sequence

<220>
<223> Seq. ID NO_346

<400> 346
ggctgggcat gcgccacact ttatgccagc c                                    31

<210> 347
<211> 31
<212> DNA
<213> Artificial Sequence

<220>
<223> Seq. ID NO_347

<400> 347
gctgcagcat gcgccacact ttatgtgcag c                                    31

<210> 348
<211> 31
<212> DNA
<213> Artificial Sequence

<220>
<223> Seq. ID NO_348

<400> 348
gggtgggcat gcgccacact ttatgccacc c                                    31

<210> 349
<211> 31
<212> DNA
<213> Artificial Sequence

<220>
<223> Seq. ID NO_349

<400> 349
gcggctgcat gcgccacact ttatgagccg c                                    31

<210> 350
<211> 31
<212> DNA
<213> Artificial Sequence

<220>
<223> Seq. ID NO_350

<400> 350
gaccgcgcat gcgccacact ttatggcggt c                                    31

<210> 351
<211> 31
<212> DNA
<213> Artificial Sequence

<220>
<223> Seq. ID NO_351

<400> 351
gacggcgcat gcgccacact ttatggccgt c                                    31

<210> 352
<211> 31
<212> DNA
<213> Artificial Sequence

<220>
<223> Seq. ID NO_352

<400> 352
ggcccagcat gcgccacact ttatgtgggc c                                    31

<210> 353
<211> 31
<212> DNA
<213> Artificial Sequence

<220>
<223> Seq. ID NO_353

<400> 353

ccgggcgcat gcgccacact ttatcgcccg g                                    31

<210> 354
<211> 17
<212> DNA
<213> Artificial Sequence

<220>
<223> Seq. ID NO_354

<400> 354
aagcgtggca tgcgcca                                                    17

<210> 355
<211> 29
<212> DNA
<213> Artificial Sequence

<220>
<223> Seq. ID NO_355

<400> 355
cgcggaaagc gtggcatgcg ccatccgcg                                       29

<210> 356
<211> 29
<212> DNA
<213> Artificial Sequence

<220>
<223> Seq. ID NO_356

<400> 356
ctcgcgaagc gtggcatgcg ccacgcgag                                       29

<210> 357
<211> 29
<212> DNA
<213> Artificial Sequence

<220>
<223> Seq. ID NO_357

<400> 357
ccgaccaagc gtggcatgcg ccaggtcgg                                       29

<210> 358
<211> 29
<212> DNA
<213> Artificial Sequence

<220>
<223> Seq. ID NO_358

<400> 358
cgacgtaagc gtggcatgcg ccaacgtcg                                          29


<210> 359
<211> 29
<212> DNA
<213> Artificial Sequence


<220>
<223> Seq. ID NO_359

<400> 359
cccaccaagc gtggcatgcg ccaggtggg                                          29


<210> 360
<211> 29
<212> DNA
<213> Artificial Sequence


<220>
<223> Seq. ID NO_360

<400> 360
cgccgaaagc gtggcatgcg ccatcggcg                                          29


<210> 361
<211> 29
<212> DNA
<213> Artificial Sequence


<220>
<223> Seq. ID NO_361

<400> 361
ctggcgaagc gtggcatgcg ccacgccag                                          29


<210> 362
<211> 29
<212> DNA
<213> Artificial Sequence


<220>
<223> Seq. ID NO_362

<400> 362
ctgccgaagc gtggcatgcg ccacggcag                                          29


<210> 363
<211> 29
<212> DNA
<213> Artificial Sequence

<220>
<223> Seq. ID NO_363

<400> 363
ccgggtaagc gtggcatgcg ccaacccgg                                          29


<210> 364
<211> 29
<212> DNA
<213> Artificial Sequence


<220>
<223> Seq. ID NO_364

<400> 364
gcgcctaagc gtggcatgcg ccaaggcgc                                          29


<210> 365
<211> 29
<212> DNA
<213> Artificial Sequence


<220>
<223> Seq. ID NO_365

<400> 365
gagcgcaagc gtggcatgcg ccagcgctc                                          29


<210> 366
<211> 29
<212> DNA
<213> Artificial Sequence


<220>
<223> Seq. ID NO_366

<400> 366
ggctggaagc gtggcatgcg ccaccagcc                                          29


<210> 367
<211> 29
<212> DNA
<213> Artificial Sequence


<220>
<223> Seq. ID NO_367

<400> 367
gctgcaaagc gtggcatgcg ccatgcagc                                          29


<210> 368
<211> 29
<212> DNA
<213> Artificial Sequence

<220>
<223> Seq. ID NO_368

<400> 368
gggtggaagc gtggcatgcg ccaccaccc                                          29


<210> 369
<211> 29
<212> DNA
<213> Artificial Sequence


<220>
<223> Seq. ID NO_369

<400> 369
gcggctaagc gtggcatgcg ccaagccgc                                          29


<210> 370
<211> 29
<212> DNA
<213> Artificial Sequence


<220>
<223> Seq. ID NO_370

<400> 370
gaccgcaagc gtggcatgcg ccagcggtc                                          29


<210> 371
<211> 29
<212> DNA
<213> Artificial Sequence


<220>
<223> Seq. ID NO_371

<400> 371
gacggcaagc gtggcatgcg ccagccgtc                                          29


<210> 372
<211> 29
<212> DNA
<213> Artificial Sequence


<220>
<223> Seq. ID NO_372

<400> 372
ggcccaaagc gtggcatgcg ccatgggcc                                          29


<210> 373
<211> 20

<212> DNA
<213> Artificial Sequence

<220>
<223> Seq. ID NO_373

<400> 373
acaagcagtt actcttatcc                                                    20

<210> 374
<211> 32
<212> DNA
<213> Artificial Sequence

<220>
<223> Seq. ID NO_374

<400> 374
cgcggaacaa gcagttactc ttatcctccg cg                                      32

<210> 375
<211> 32
<212> DNA
<213> Artificial Sequence

<220>
<223> Seq. ID NO_375

<400> 375
ctcgcgacaa gcagttactc ttatcccgcg ag                                      32

<210> 376
<211> 32
<212> DNA
<213> Artificial Sequence

<220>
<223> Seq. ID NO_376

<400> 376
ccgaccacaa gcagttactc ttatccggtc gg                                      32

<210> 377
<211> 32
<212> DNA
<213> Artificial Sequence

<220>
<223> Seq. ID NO_377

<400> 377
cgacgtacaa gcagttactc ttatccacgt cg                                      32

<210> 378
<211> 32
<212> DNA
<213> Artificial Sequence

<220>
<223> Seq. ID NO_378

<400> 378
cccaccacaa gcagttactc ttatccggtg gg                                    32

<210> 379
<211> 32
<212> DNA
<213> Artificial Sequence

<220>
<223> Seq. ID NO_379

<400> 379
cgccgaacaa gcagttactc ttatcctcgg cg                                    32

<210> 380
<211> 43
<212> DNA
<213> Artificial Sequence

<220>
<223> Seq. ID NO_380

<400> 380
agacttcggc tgtgattaca agcagttact cttatccccg aag                        43

<210> 381
<211> 32
<212> DNA
<213> Artificial Sequence

<220>
<223> Seq. ID NO_381

<400> 381
ctggcgacaa gcagttactc ttatcccgcc ag                                    32

<210> 382
<211> 32
<212> DNA
<213> Artificial Sequence

<220>
<223> Seq. ID NO_382

<400> 382
ctgccgacaa gcagttactc ttatcccggc ag                                    32

<210> 383
<211> 32
<212> DNA
<213> Artificial Sequence

<220>
<223> Seq. ID NO_383

<400> 383
ccgggtacaa gcagttactc ttatccaccc gg                                    32

<210> 384
<211> 32
<212> DNA
<213> Artificial Sequence

<220>
<223> Seq. ID NO_384

<400> 384
gcgcctacaa gcagttactc ttatccaggc gc                                    32

<210> 385
<211> 32
<212> DNA
<213> Artificial Sequence

<220>
<223> Seq. ID NO_385

<400> 385
gagcgcacaa gcagttactc ttatccgcgc tc                                    32

<210> 386
<211> 32
<212> DNA
<213> Artificial Sequence

<220>
<223> Seq. ID NO_386

<400> 386
ggctggacaa gcagttactc ttatccccag cc                                    32

<210> 387
<211> 32
<212> DNA
<213> Artificial Sequence

<220>
<223> Seq. ID NO_387

<400> 387
gctgcaacaa gcagttactc ttatcctgca gc                                    32


<210> 388
<211> 32
<212> DNA
<213> Artificial Sequence


<220>
<223> Seq. ID NO_388

<400> 388
gggtggacaa gcagttactc ttatccccac cc                                    32


<210> 389
<211> 32
<212> DNA
<213> Artificial Sequence


<220>
<223> Seq. ID NO_389

<400> 389
gcggctacaa gcagttactc ttatccagcc gc                                    32


<210> 390
<211> 32
<212> DNA
<213> Artificial Sequence


<220>
<223> Seq. ID NO_390

<400> 390
gaccgcacaa gcagttactc ttatccgcgg tc                                    32


<210> 391
<211> 32
<212> DNA
<213> Artificial Sequence


<220>
<223> Seq. ID NO_391

<400> 391
gacggcacaa gcagttactc ttatccgccg tc                                    32


<210> 392
<211> 32
<212> DNA
<213> Artificial Sequence


<220>

<223> Seq. ID NO_392

<400> 392
ggcccaacaa gcagttactc ttatcctggg cc                                    32


<210> 393
<211> 21
<212> DNA
<213> Artificial Sequence


<220>
<223> Seq. ID NO_393

<400> 393
ggacaagcag ttactcttat c                                                21


<210> 394
<211> 33
<212> DNA
<213> Artificial Sequence


<220>
<223> Seq. ID NO_394

<400> 394
cgcggaggac aagcagttac tcttatctcc gcg                                   33


<210> 395
<211> 33
<212> DNA
<213> Artificial Sequence


<220>
<223> Seq. ID NO_395

<400> 395
ctcgcgggac aagcagttac tcttatccgc gag                                   33


<210> 396
<211> 33
<212> DNA
<213> Artificial Sequence


<220>
<223> Seq. ID NO_396

<400> 396
ccgaccggac aagcagttac tcttatcggt cgg                                   33


<210> 397
<211> 33
<212> DNA
<213> Artificial Sequence

<220>
<223> Seq. ID NO_397

<400> 397
cgacgtggac aagcagttac tcttatcacg tcg                                    33


<210> 398
<211> 33
<212> DNA
<213> Artificial Sequence


<220>
<223> Seq. ID NO_398

<400> 398
cccaccggac aagcagttac tcttatcggt ggg                                    33


<210> 399
<211> 33
<212> DNA
<213> Artificial Sequence


<220>
<223> Seq. ID NO_399

<400> 399
cgccgaggac aagcagttac tcttatctcg gcg                                    33


<210> 400
<211> 33
<212> DNA
<213> Artificial Sequence


<220>
<223> Seq. ID NO_400

<400> 400
ctggcgggac aagcagttac tcttatccgc cag                                    33


<210> 401
<211> 33
<212> DNA
<213> Artificial Sequence


<220>
<223> Seq. ID NO_401

<400> 401
ctgccgggac aagcagttac tcttatccgg cag                                    33


<210> 402
<211> 33
<212> DNA

<213> Artificial Sequence

<220>
<223> Seq. ID NO_402

<400> 402
ccgggtggac aagcagttac tcttatcacc cgg                                    33

<210> 403
<211> 33
<212> DNA
<213> Artificial Sequence

<220>
<223> Seq. ID NO_403

<400> 403
gcgcctggac aagcagttac tcttatcagg cgc                                    33

<210> 404
<211> 33
<212> DNA
<213> Artificial Sequence

<220>
<223> Seq. ID NO_404

<400> 404
gagcgcggac aagcagttac tcttatcgcg ctc                                    33

<210> 405
<211> 33
<212> DNA
<213> Artificial Sequence

<220>
<223> Seq. ID NO_405

<400> 405
ggctggggac aagcagttac tcttatccca gcc                                    33

<210> 406
<211> 33
<212> DNA
<213> Artificial Sequence

<220>
<223> Seq. ID NO_406

<400> 406
gctgcaggac aagcagttac tcttatctgc agc                                    33

<210> 407

<211> 33
<212> DNA
<213> Artificial Sequence

<220>
<223> Seq. ID NO_407

<400> 407
gggtggggac aagcagttac tcttatccca ccc                                    33

<210> 408
<211> 33
<212> DNA
<213> Artificial Sequence

<220>
<223> Seq. ID NO_408

<400> 408
gcggctggac aagcagttac tcttatcagc cgc                                    33

<210> 409
<211> 33
<212> DNA
<213> Artificial Sequence

<220>
<223> Seq. ID NO_409

<400> 409
gaccgcggac aagcagttac tcttatcgcg gtc                                    33

<210> 410
<211> 33
<212> DNA
<213> Artificial Sequence

<220>
<223> Seq. ID NO_410

<400> 410
gacggcggac aagcagttac tcttatcgcc gtc                                    33

<210> 411
<211> 33
<212> DNA
<213> Artificial Sequence

<220>
<223> Seq. ID NO_411

<400> 411
ggcccaggac aagcagttac tcttatctgg gcc                                    33

<210> 412
<211> 21
<212> DNA
<213> Artificial Sequence

<220>
<223> Seq. ID NO_412

<400> 412
ctatccgact aaagatagtg g                                                21

<210> 413
<211> 33
<212> DNA
<213> Artificial Sequence

<220>
<223> Seq. ID NO_413

<400> 413
cgcggactat ccgactaaag atagtggtcc gcg                                   33

<210> 414
<211> 30
<212> DNA
<213> Artificial Sequence

<220>
<223> Seq. ID NO_414

<400> 414
cgcgcctatc cgactaaaga tagtggcgcg                                       30

<210> 415
<211> 33
<212> DNA
<213> Artificial Sequence

<220>
<223> Seq. ID NO_415

<400> 415
ctcgcgctat ccgactaaag atagtggcgc gag                                   33

<210> 416
<211> 33
<212> DNA
<213> Artificial Sequence

<220>
<223> Seq. ID NO_416

<400> 416

ccgaccctat ccgactaaag atagtggggt cgg 33

<210> 417
<211> 33
<212> DNA
<213> Artificial Sequence

<220>
<223> Seq. ID NO_417

<400> 417
cgacgtctat ccgactaaag atagtggacg tcg 33

<210> 418
<211> 33
<212> DNA
<213> Artificial Sequence

<220>
<223> Seq. ID NO_418

<400> 418
cccaccctat ccgactaaag atagtggggt ggg 33

<210> 419
<211> 33
<212> DNA
<213> Artificial Sequence

<220>
<223> Seq. ID NO_419

<400> 419
cgccgactat ccgactaaag atagtggtcg gcg 33

<210> 420
<211> 33
<212> DNA
<213> Artificial Sequence

<220>
<223> Seq. ID NO_420

<400> 420
ctggcgctat ccgactaaag atagtggcgc cag 33

<210> 421
<211> 33
<212> DNA
<213> Artificial Sequence

<220>
<223> Seq. ID NO_421

<400> 421
ctgccgctat ccgactaaag atagtggcgg cag                                    33

<210> 422
<211> 33
<212> DNA
<213> Artificial Sequence

<220>
<223> Seq. ID NO_422

<400> 422
ccgggtctat ccgactaaag atagtggacc cgg                                    33

<210> 423
<211> 33
<212> DNA
<213> Artificial Sequence

<220>
<223> Seq. ID NO_423

<400> 423
gcgcctctat ccgactaaag atagtggagg cgc                                    33

<210> 424
<211> 33
<212> DNA
<213> Artificial Sequence

<220>
<223> Seq. ID NO_424

<400> 424
gagcgcctat ccgactaaag atagtgggcg ctc                                    33

<210> 425
<211> 33
<212> DNA
<213> Artificial Sequence

<220>
<223> Seq. ID NO_425

<400> 425
ggctggctat ccgactaaag atagtggcca gcc                                    33

<210> 426
<211> 33
<212> DNA
<213> Artificial Sequence

<220>
<223> Seq. ID NO_426

<400> 426
gctgcactat ccgactaaag atagtggtgc agc                                    33


<210> 427
<211> 33
<212> DNA
<213> Artificial Sequence


<220>
<223> Seq. ID NO_427

<400> 427
gggtggctat ccgactaaag atagtggcca ccc                                    33


<210> 428
<211> 33
<212> DNA
<213> Artificial Sequence


<220>
<223> Seq. ID NO_428

<400> 428
gcggctctat ccgactaaag atagtggagc cgc                                    33


<210> 429
<211> 33
<212> DNA
<213> Artificial Sequence


<220>
<223> Seq. ID NO_429

<400> 429
gaccgcctat ccgactaaag atagtgggcg gtc                                    33


<210> 430
<211> 33
<212> DNA
<213> Artificial Sequence


<220>
<223> Seq. ID NO_430

<400> 430
gacggcctat ccgactaaag atagtgggcc gtc                                    33


<210> 431
<211> 33
<212> DNA
<213> Artificial Sequence

<220>
<223> Seq. ID NO_431

<400> 431
ggcccactat ccgactaaag atagtggtgg gcc                                    33


<210> 432
<211> 18
<212> DNA
<213> Artificial Sequence


<220>
<223> Seq. ID NO_432

<400> 432
tccgactaaa gatagtgg                                                     18


<210> 433
<211> 30
<212> DNA
<213> Artificial Sequence


<220>
<223> Seq. ID NO_433

<400> 433
cgcggatccg actaaagata gtggtccgcg                                        30


<210> 434
<211> 30
<212> DNA
<213> Artificial Sequence


<220>
<223> Seq. ID NO_434

<400> 434
ctcgcgtccg actaaagata gtggcgcgag                                        30


<210> 435
<211> 30
<212> DNA
<213> Artificial Sequence


<220>
<223> Seq. ID NO_435

<400> 435
ccgacctccg actaaagata gtggggtcgg                                        30


<210> 436
<211> 30

<212> DNA
<213> Artificial Sequence

<220>
<223> Seq. ID NO_436

<400> 436
cgacgttccg actaaagata gtggacgtcg                                        30

<210> 437
<211> 30
<212> DNA
<213> Artificial Sequence

<220>
<223> Seq. ID NO_437

<400> 437
cccacctccg actaaagata gtgggtggg                                         30

<210> 438
<211> 30
<212> DNA
<213> Artificial Sequence

<220>
<223> Seq. ID NO_438

<400> 438
cgccgatccg actaaagata gtggtcggcg                                        30

<210> 439
<211> 30
<212> DNA
<213> Artificial Sequence

<220>
<223> Seq. ID NO_439

<400> 439
ctggcgtccg actaaagata gtggcgccag                                        30

<210> 440
<211> 30
<212> DNA
<213> Artificial Sequence

<220>
<223> Seq. ID NO_440

<400> 440
ctgccgtccg actaaagata gtggcggcag                                        30

<210> 441
<211> 30
<212> DNA
<213> Artificial Sequence

<220>
<223> Seq. ID NO_441

<400> 441
ccgggttccg actaaagata gtggacccgg                                        30

<210> 442
<211> 30
<212> DNA
<213> Artificial Sequence

<220>
<223> Seq. ID NO_442

<400> 442
gcgccttccg actaaagata gtggaggcgc                                        30

<210> 443
<211> 30
<212> DNA
<213> Artificial Sequence

<220>
<223> Seq. ID NO_443

<400> 443
gagcgctccg actaaagata gtgggcgctc                                        30

<210> 444
<211> 30
<212> DNA
<213> Artificial Sequence

<220>
<223> Seq. ID NO_444

<400> 444
ggctggtccg actaaagata gtggccagcc                                        30

<210> 445
<211> 30
<212> DNA
<213> Artificial Sequence

<220>
<223> Seq. ID NO_445

<400> 445
gctgcatccg actaaagata gtggtgcagc                                        30

<210> 446
<211> 30
<212> DNA
<213> Artificial Sequence

<220>
<223> Seq. ID NO_446

<400> 446
gggtggtccg actaaagata gtggccaccc                                        30

<210> 447
<211> 30
<212> DNA
<213> Artificial Sequence

<220>
<223> Seq. ID NO_447

<400> 447
gcggcttccg actaaagata gtggagccgc                                        30

<210> 448
<211> 30
<212> DNA
<213> Artificial Sequence

<220>
<223> Seq. ID NO_448

<400> 448
gaccgctccg actaaagata gtgggcggtc                                        30

<210> 449
<211> 30
<212> DNA
<213> Artificial Sequence

<220>
<223> Seq. ID NO_449

<400> 449
gacggctccg actaaagata gtgggccgtc                                        30

<210> 450
<211> 30
<212> DNA
<213> Artificial Sequence

<220>
<223> Seq. ID NO_450

<400> 450
ggcccatccg actaaagata gtggtgggcc                                        30


<210> 451
<211> 21
<212> DNA
<213> Artificial Sequence


<220>
<223> Seq. ID NO_451

<400> 451
ctggagggaa agccatttca a                                                 21


<210> 452
<211> 33
<212> DNA
<213> Artificial Sequence


<220>
<223> Seq. ID NO_452

<400> 452
cgcggactgg agggaaagcc atttcaatcc gcg                                    33


<210> 453
<211> 33
<212> DNA
<213> Artificial Sequence


<220>
<223> Seq. ID NO_453

<400> 453
ctcgcgctgg agggaaagcc atttcaacgc gag                                    33


<210> 454
<211> 30
<212> DNA
<213> Artificial Sequence


<220>
<223> Seq. ID NO_454

<400> 454
cgcgctggag ggaaagccat ttcaagcgcg                                        30


<210> 455
<211> 33
<212> DNA
<213> Artificial Sequence


<220>

<223> Seq. ID NO_455

<400> 455
ccgaccctgg agggaaagcc atttcaaggt cgg                                    33


<210> 456
<211> 33
<212> DNA
<213> Artificial Sequence


<220>
<223> Seq. ID NO_456

<400> 456
cgacgtctgg agggaaagcc atttcaaacg tcg                                    33


<210> 457
<211> 33
<212> DNA
<213> Artificial Sequence


<220>
<223> Seq. ID NO_457

<400> 457
cccaccctgg agggaaagcc atttcaaggt ggg                                    33


<210> 458
<211> 33
<212> DNA
<213> Artificial Sequence


<220>
<223> Seq. ID NO_458

<400> 458
cgccgactgg agggaaagcc atttcaatcg gcg                                    33


<210> 459
<211> 33
<212> DNA
<213> Artificial Sequence


<220>
<223> Seq. ID NO_459

<400> 459
ctggcgctgg agggaaagcc atttcaacgc cag                                    33


<210> 460
<211> 33
<212> DNA
<213> Artificial Sequence

<220>
<223> Seq. ID NO_460

<400> 460
ctgccgctgg agggaaagcc atttcaacgg cag                                    33


<210> 461
<211> 33
<212> DNA
<213> Artificial Sequence


<220>
<223> Seq. ID NO_461

<400> 461
ccgggtctgg agggaaagcc atttcaaacc cgg                                    33


<210> 462
<211> 33
<212> DNA
<213> Artificial Sequence


<220>
<223> Seq. ID NO_462

<400> 462
gcgcctctgg agggaaagcc atttcaaagg cgc                                    33


<210> 463
<211> 33
<212> DNA
<213> Artificial Sequence


<220>
<223> Seq. ID NO_463

<400> 463
gagcgcctgg agggaaagcc atttcaagcg ctc                                    33


<210> 464
<211> 33
<212> DNA
<213> Artificial Sequence


<220>
<223> Seq. ID NO_464

<400> 464
ggctggctgg agggaaagcc atttcaacca gcc                                    33


<210> 465
<211> 33
<212> DNA

<213> Artificial Sequence

<220>
<223> Seq. ID NO_465

<400> 465
gctgcactgg agggaaagcc atttcaatgc agc                                    33

<210> 466
<211> 33
<212> DNA
<213> Artificial Sequence

<220>
<223> Seq. ID NO_466

<400> 466
gggtggctgg agggaaagcc atttcaacca ccc                                    33

<210> 467
<211> 33
<212> DNA
<213> Artificial Sequence

<220>
<223> Seq. ID NO_467

<400> 467
gcggctctgg agggaaagcc atttcaaagc cgc                                    33

<210> 468
<211> 33
<212> DNA
<213> Artificial Sequence

<220>
<223> Seq. ID NO_468

<400> 468
gaccgcctgg agggaaagcc atttcaagcg gtc                                    33

<210> 469
<211> 33
<212> DNA
<213> Artificial Sequence

<220>
<223> Seq. ID NO_469

<400> 469
gacggcctgg agggaaagcc atttcaagcc gtc                                    33

<210> 470

<211> 33
<212> DNA
<213> Artificial Sequence

<220>
<223> Seq. ID NO_470

<400> 470
ggcccactgg agggaaagcc atttcaatgg gcc                                    33

<210> 471
<211> 24
<212> DNA
<213> Artificial Sequence

<220>
<223> Seq. ID NO_471

<400> 471
atttcaacta ccgggctgtt accg                                              24

<210> 472
<211> 36
<212> DNA
<213> Artificial Sequence

<220>
<223> Seq. ID NO_472

<400> 472
cgcggaattt caactaccgg gctgttaccg tccgcg                                 36

<210> 473
<211> 36
<212> DNA
<213> Artificial Sequence

<220>
<223> Seq. ID NO_473

<400> 473
ctcgcgattt caactaccgg gctgttaccg cgcgag                                 36

<210> 474
<211> 36
<212> DNA
<213> Artificial Sequence

<220>
<223> Seq. ID NO_474

<400> 474
ccgaccattt caactaccgg gctgttaccg ggtcgg                                 36

<210> 475
<211> 36
<212> DNA
<213> Artificial Sequence

<220>
<223> Seq. ID NO_475

<400> 475
cgacgtattt caactaccgg gctgttaccg acgtcg                                36

<210> 476
<211> 36
<212> DNA
<213> Artificial Sequence

<220>
<223> Seq. ID NO_476

<400> 476
cccaccattt caactaccgg gctgttaccg ggtggg                                36

<210> 477
<211> 36
<212> DNA
<213> Artificial Sequence

<220>
<223> Seq. ID NO_477

<400> 477
cgccgaattt caactaccgg gctgttaccg tcggcg                                36

<210> 478
<211> 36
<212> DNA
<213> Artificial Sequence

<220>
<223> Seq. ID NO_478

<400> 478
ctggcgattt caactaccgg gctgttaccg cgccag                                36

<210> 479
<211> 36
<212> DNA
<213> Artificial Sequence

<220>
<223> Seq. ID NO_479

<400> 479

ctgccgattt caactaccgg gctgttaccg cggcag                                36

<210> 480
<211> 36
<212> DNA
<213> Artificial Sequence

<220>
<223> Seq. ID NO_480

<400> 480
ccgggtattt caactaccgg gctgttaccg acccgg                                36

<210> 481
<211> 36
<212> DNA
<213> Artificial Sequence

<220>
<223> Seq. ID NO_481

<400> 481
gcgcctattt caactaccgg gctgttaccg aggcgc                                36

<210> 482
<211> 36
<212> DNA
<213> Artificial Sequence

<220>
<223> Seq. ID NO_482

<400> 482
gagcgcattt caactaccgg gctgttaccg gcgctc                                36

<210> 483
<211> 36
<212> DNA
<213> Artificial Sequence

<220>
<223> Seq. ID NO_483

<400> 483
ggctggattt caactaccgg gctgttaccg ccagcc                                36

<210> 484
<211> 36
<212> DNA
<213> Artificial Sequence

<220>
<223> Seq. ID NO_484

<400> 484
gctgcaattt caactaccgg gctgttaccg tgcagc                                36

<210> 485
<211> 36
<212> DNA
<213> Artificial Sequence

<220>
<223> Seq. ID NO_485

<400> 485
gggtggattt caactaccgg gctgttaccg ccaccc                                36

<210> 486
<211> 36
<212> DNA
<213> Artificial Sequence

<220>
<223> Seq. ID NO_486

<400> 486
gcggctattt caactaccgg gctgttaccg agccgc                                36

<210> 487
<211> 36
<212> DNA
<213> Artificial Sequence

<220>
<223> Seq. ID NO_487

<400> 487
gaccgcattt caactaccgg gctgttaccg gcggtc                                36

<210> 488
<211> 36
<212> DNA
<213> Artificial Sequence

<220>
<223> Seq. ID NO_488

<400> 488
gacggcattt caactaccgg gctgttaccg gccgtc                                36

<210> 489
<211> 36
<212> DNA
<213> Artificial Sequence

<220>
<223> Seq. ID NO_489

<400> 489
ggcccaattt caactaccgg gctgttaccg tgggcc                                36

<210> 490
<211> 19
<212> DNA
<213> Artificial Sequence

<220>
<223> Seq. ID NO_490

<400> 490
gatccactct ggagggaaa                                                   19

<210> 491
<211> 31
<212> DNA
<213> Artificial Sequence

<220>
<223> Seq. ID NO_491

<400> 491
cgcggagatc cactctggag ggaaatccgc g                                     31

<210> 492
<211> 31
<212> DNA
<213> Artificial Sequence

<220>
<223> Seq. ID NO_492

<400> 492
ctcgcggatc cactctggag ggaaacgcga g                                     31

<210> 493
<211> 31
<212> DNA
<213> Artificial Sequence

<220>
<223> Seq. ID NO_493

<400> 493
ccgaccgatc cactctggag ggaaaggtcg g                                     31

<210> 494
<211> 31
<212> DNA
<213> Artificial Sequence

<220>
<223> Seq. ID NO_494

<400> 494
cgacgtgatc cactctggag ggaaaacgtc g                                    31


<210> 495
<211> 31
<212> DNA
<213> Artificial Sequence


<220>
<223> Seq. ID NO_495

<400> 495
cccaccgatc cactctggag ggaaaggtgg g                                    31


<210> 496
<211> 31
<212> DNA
<213> Artificial Sequence


<220>
<223> Seq. ID NO_496

<400> 496
cgccgagatc cactctggag ggaaatcggc g                                    31


<210> 497
<211> 31
<212> DNA
<213> Artificial Sequence


<220>
<223> Seq. ID NO_497

<400> 497
ctggcggatc cactctggag ggaaacgcca g                                    31


<210> 498
<211> 31
<212> DNA
<213> Artificial Sequence


<220>
<223> Seq. ID NO_498

<400> 498
ctgccggatc cactctggag ggaaacggca g                                    31


<210> 499
<211> 31

<212> DNA
<213> Artificial Sequence

<220>
<223> Seq. ID NO_499

<400> 499
ccgggtgatc cactctggag ggaaaacccg g                                    31

<210> 500
<211> 31
<212> DNA
<213> Artificial Sequence

<220>
<223> Seq. ID NO_500

<400> 500
gcgcctgatc cactctggag ggaaaaggcg c                                    31

<210> 501
<211> 31
<212> DNA
<213> Artificial Sequence

<220>
<223> Seq. ID NO_501

<400> 501
gagcgcgatc cactctggag ggaaagcgct c                                    31

<210> 502
<211> 31
<212> DNA
<213> Artificial Sequence

<220>
<223> Seq. ID NO_502

<400> 502
ggctgggatc cactctggag ggaaaccagc c                                    31

<210> 503
<211> 31
<212> DNA
<213> Artificial Sequence

<220>
<223> Seq. ID NO_503

<400> 503
gctgcagatc cactctggag ggaaatgcag c                                    31

<210> 504
<211> 31
<212> DNA
<213> Artificial Sequence

<220>
<223> Seq. ID NO_504

<400> 504
gggtgggatc cactctggag ggaaaccacc c                                    31

<210> 505
<211> 31
<212> DNA
<213> Artificial Sequence

<220>
<223> Seq. ID NO_505

<400> 505
gcggctgatc cactctggag ggaaaagccg c                                    31

<210> 506
<211> 31
<212> DNA
<213> Artificial Sequence

<220>
<223> Seq. ID NO_506

<400> 506
gaccgcgatc cactctggag ggaaagcggt c                                    31

<210> 507
<211> 31
<212> DNA
<213> Artificial Sequence

<220>
<223> Seq. ID NO_507

<400> 507
gacggcgatc cactctggag ggaaagccgt c                                    31

<210> 508
<211> 31
<212> DNA
<213> Artificial Sequence

<220>
<223> Seq. ID NO_508

<400> 508
ggcccagatc cactctggag ggaaatgggc c                                    31

<210> 509
<211> 23
<212> DNA
<213> Artificial Sequence

<220>
<223> Seq. ID NO_509

<400> 509
tgactcccga gtataagtac atg                                               23

<210> 510
<211> 35
<212> DNA
<213> Artificial Sequence

<220>
<223> Seq. ID NO_510

<400> 510
cgcggatgac tcccgagtat aagtacatgt ccgcg                                  35

<210> 511
<211> 35
<212> DNA
<213> Artificial Sequence

<220>
<223> Seq. ID NO_511

<400> 511
ctcgcgtgac tcccgagtat aagtacatgc gcgag                                  35

<210> 512
<211> 35
<212> DNA
<213> Artificial Sequence

<220>
<223> Seq. ID NO_512

<400> 512
ccgacctgac tcccgagtat aagtacatgg gtcgg                                  35

<210> 513
<211> 35
<212> DNA
<213> Artificial Sequence

<220>
<223> Seq. ID NO_513

<400> 513
cgacgttgac tcccgagtat aagtacatga cgtcg                                35

<210> 514
<211> 35
<212> DNA
<213> Artificial Sequence

<220>
<223> Seq. ID NO_514

<400> 514
cccacctgac tcccgagtat aagtacatgg gtggg                                35

<210> 515
<211> 35
<212> DNA
<213> Artificial Sequence

<220>
<223> Seq. ID NO_515

<400> 515
cgccgatgac tcccgagtat aagtacatgt cggcg                                35

<210> 516
<211> 35
<212> DNA
<213> Artificial Sequence

<220>
<223> Seq. ID NO_516

<400> 516
ctggcgtgac tcccgagtat aagtacatgc gccag                                35

<210> 517
<211> 35
<212> DNA
<213> Artificial Sequence

<220>
<223> Seq. ID NO_517

<400> 517
ctgccgtgac tcccgagtat aagtacatgc ggcag                                35

<210> 518
<211> 35
<212> DNA
<213> Artificial Sequence

<220>

<223> Seq. ID NO_518

<400> 518
ccgggttgac tcccgagtat aagtacatga cccgg                                35


<210> 519
<211> 35
<212> DNA
<213> Artificial Sequence


<220>
<223> Seq. ID NO_519

<400> 519
gcgccttgac tcccgagtat aagtacatga ggcgc                                35


<210> 520
<211> 35
<212> DNA
<213> Artificial Sequence


<220>
<223> Seq. ID NO_520

<400> 520
gagcgctgac tcccgagtat aagtacatgg cgctc                                35


<210> 521
<211> 35
<212> DNA
<213> Artificial Sequence


<220>
<223> Seq. ID NO_521

<400> 521
ggctggtgac tcccgagtat aagtacatgc cagcc                                35


<210> 522
<211> 35
<212> DNA
<213> Artificial Sequence


<220>
<223> Seq. ID NO_522

<400> 522
gctgcatgac tcccgagtat aagtacatgt gcagc                                35


<210> 523
<211> 35
<212> DNA
<213> Artificial Sequence

<220>
<223> Seq. ID NO_523

<400> 523
gggtggtgac tcccgagtat aagtacatgc caccc                                35


<210> 524
<211> 34
<212> DNA
<213> Artificial Sequence


<220>
<223> Seq. ID NO_524

<400> 524
ccggtctgac tcccgagtat aagtacatga ccgg                                 34


<210> 525
<211> 35
<212> DNA
<213> Artificial Sequence


<220>
<223> Seq. ID NO_525

<400> 525
gcggcttgac tcccgagtat aagtacatga gccgc                                35


<210> 526
<211> 35
<212> DNA
<213> Artificial Sequence


<220>
<223> Seq. ID NO_526

<400> 526
gaccgctgac tcccgagtat aagtacatgg cggtc                                35


<210> 527
<211> 35
<212> DNA
<213> Artificial Sequence


<220>
<223> Seq. ID NO_527

<400> 527
gacggctgac tcccgagtat aagtacatgg ccgtc                                35


<210> 528
<211> 35
<212> DNA

<213> Artificial Sequence

<220>
<223> Seq. ID NO_528

<400> 528
ggcccatgac tcccgagtat aagtacatgt gggcc 35

<210> 529
<211> 20
<212> DNA
<213> Artificial Sequence

<220>
<223> Seq. ID NO_529

<400> 529
cgaacctcta aagaggttca 20

<210> 530
<211> 32
<212> DNA
<213> Artificial Sequence

<220>
<223> Seq. ID NO_530

<400> 530
cgcggacgaa cctctaaaga ggttcatccg cg 32

<210> 531
<211> 32
<212> DNA
<213> Artificial Sequence

<220>
<223> Seq. ID NO_531

<400> 531
ctcgcgcgaa cctctaaaga ggttcacgcg ag 32

<210> 532
<211> 32
<212> DNA
<213> Artificial Sequence

<220>
<223> Seq. ID NO_532

<400> 532
ccgacccgaa cctctaaaga ggttcaggtc gg 32

<210> 533

<211> 32
<212> DNA
<213> Artificial Sequence

<220>
<223> Seq. ID NO_533

<400> 533
cgacgtcgaa cctctaaaga ggttcaacgt cg                                    32

<210> 534
<211> 32
<212> DNA
<213> Artificial Sequence

<220>
<223> Seq. ID NO_534

<400> 534
cccacccgaa cctctaaaga ggttcaggtg gg                                    32

<210> 535
<211> 32
<212> DNA
<213> Artificial Sequence

<220>
<223> Seq. ID NO_535

<400> 535
cgccgacgaa cctctaaaga ggttcatcgg cg                                    32

<210> 536
<211> 32
<212> DNA
<213> Artificial Sequence

<220>
<223> Seq. ID NO_536

<400> 536
ctggcgcgaa cctctaaaga ggttcacgcc ag                                    32

<210> 537
<211> 32
<212> DNA
<213> Artificial Sequence

<220>
<223> Seq. ID NO_537

<400> 537
ctgccgcgaa cctctaaaga ggttcacggc ag                                    32

<210> 538
<211> 32
<212> DNA
<213> Artificial Sequence

<220>
<223> Seq. ID NO_538

<400> 538
ccgggtcgaa cctctaaaga ggttcaaccc gg                                    32

<210> 539
<211> 32
<212> DNA
<213> Artificial Sequence

<220>
<223> Seq. ID NO_539

<400> 539
gcgcctcgaa cctctaaaga ggttcaaggc gc                                    32

<210> 540
<211> 32
<212> DNA
<213> Artificial Sequence

<220>
<223> Seq. ID NO_540

<400> 540
gagcgccgaa cctctaaaga ggttcagcgc tc                                    32

<210> 541
<211> 32
<212> DNA
<213> Artificial Sequence

<220>
<223> Seq. ID NO_541

<400> 541
ggctggcgaa cctctaaaga ggttcaccag cc                                    32

<210> 542
<211> 32
<212> DNA
<213> Artificial Sequence

<220>
<223> Seq. ID NO_542

<400> 542

gctgcacgaa cctctaaaga ggttcatgca gc                                    32

<210> 543
<211> 32
<212> DNA
<213> Artificial Sequence

<220>
<223> Seq. ID NO_543

<400> 543
gggtggcgaa cctctaaaga ggttcaccac cc                                    32

<210> 544
<211> 32
<212> DNA
<213> Artificial Sequence

<220>
<223> Seq. ID NO_544

<400> 544
gcggctcgaa cctctaaaga ggttcaagcc gc                                    32

<210> 545
<211> 32
<212> DNA
<213> Artificial Sequence

<220>
<223> Seq. ID NO_545

<400> 545
gaccgccgaa cctctaaaga ggttcagcgg tc                                    32

<210> 546
<211> 32
<212> DNA
<213> Artificial Sequence

<220>
<223> Seq. ID NO_546

<400> 546
gacggccgaa cctctaaaga ggttcagccg tc                                    32

<210> 547
<211> 32
<212> DNA
<213> Artificial Sequence

<220>
<223> Seq. ID NO_547

<400> 547
ggcccacgaa cctctaaaga ggttcatggg cc                                    32

<210> 548
<211> 23
<212> DNA
<213> Artificial Sequence

<220>
<223> Seq. ID NO_548

<400> 548
catcggttaa acaattagca ctg                                              23

<210> 549
<211> 35
<212> DNA
<213> Artificial Sequence

<220>
<223> Seq. ID NO_549

<400> 549
cgcggacatc ggttaaacaa ttagcactgt ccgcg                                 35

<210> 550
<211> 35
<212> DNA
<213> Artificial Sequence

<220>
<223> Seq. ID NO_550

<400> 550
ctcgcgcatc ggttaaacaa ttagcactgc gcgag                                 35

<210> 551
<211> 35
<212> DNA
<213> Artificial Sequence

<220>
<223> Seq. ID NO_551

<400> 551
ccgacccatc ggttaaacaa ttagcactgg gtcgg                                 35

<210> 552
<211> 35
<212> DNA
<213> Artificial Sequence

<220>
<223> Seq. ID NO_552

<400> 552
cgacgtcatc ggttaaacaa ttagcactga cgtcg                                35


<210> 553
<211> 35
<212> DNA
<213> Artificial Sequence


<220>
<223> Seq. ID NO_553

<400> 553
cccacccatc ggttaaacaa ttagcactgg gtggg                                35


<210> 554
<211> 35
<212> DNA
<213> Artificial Sequence


<220>
<223> Seq. ID NO_554

<400> 554
cgccgacatc ggttaaacaa ttagcactgt cggcg                                35


<210> 555
<211> 35
<212> DNA
<213> Artificial Sequence


<220>
<223> Seq. ID NO_555

<400> 555
ctggcgcatc ggttaaacaa ttagcactgc gccag                                35


<210> 556
<211> 33
<212> DNA
<213> Artificial Sequence


<220>
<223> Seq. ID NO_556

<400> 556
cgcgccatcg gttaaacaat tagcactggc gcg                                  33


<210> 557
<211> 35
<212> DNA
<213> Artificial Sequence

<220>
<223> Seq. ID NO_557

<400> 557
ctgccgcatc ggttaaacaa ttagcactgc ggcag                                35


<210> 558
<211> 35
<212> DNA
<213> Artificial Sequence


<220>
<223> Seq. ID NO_558

<400> 558
ccgggtcatc ggttaaacaa ttagcactga cccgg                                35


<210> 559
<211> 35
<212> DNA
<213> Artificial Sequence


<220>
<223> Seq. ID NO_559

<400> 559
gcgcctcatc ggttaaacaa ttagcactga ggcgc                                35


<210> 560
<211> 35
<212> DNA
<213> Artificial Sequence


<220>
<223> Seq. ID NO_560

<400> 560
gagcgccatc ggttaaacaa ttagcactgg cgctc                                35


<210> 561
<211> 35
<212> DNA
<213> Artificial Sequence


<220>
<223> Seq. ID NO_561

<400> 561
ggctggcatc ggttaaacaa ttagcactgc cagcc                                35


<210> 562
<211> 35

<212> DNA
<213> Artificial Sequence

<220>
<223> Seq. ID NO_562

<400> 562
gctgcacatc ggttaaacaa ttagcactgt gcagc 35

<210> 563
<211> 35
<212> DNA
<213> Artificial Sequence

<220>
<223> Seq. ID NO_563

<400> 563
gggtggcatc ggttaaacaa ttagcactgc caccc 35

<210> 564
<211> 35
<212> DNA
<213> Artificial Sequence

<220>
<223> Seq. ID NO_564

<400> 564
gcggctcatc ggttaaacaa ttagcactga gccgc 35

<210> 565
<211> 35
<212> DNA
<213> Artificial Sequence

<220>
<223> Seq. ID NO_565

<400> 565
gaccgccatc ggttaaacaa ttagcactgg cggtc 35

<210> 566
<211> 35
<212> DNA
<213> Artificial Sequence

<220>
<223> Seq. ID NO_566

<400> 566
gacggccatc ggttaaacaa ttagcactgg ccgtc 35

<210> 567
<211> 35
<212> DNA
<213> Artificial Sequence

<220>
<223> Seq. ID NO_567

<400> 567
ggcccacatc ggttaaacaa ttagcactgt gggcc                                35

<210> 568
<211> 23
<212> DNA
<213> Artificial Sequence

<220>
<223> Seq. ID NO_568

<400> 568
tgcgattccc tatccttctg tgt                                             23

<210> 569
<211> 35
<212> DNA
<213> Artificial Sequence

<220>
<223> Seq. ID NO_569

<400> 569
cgcggatgcg attccctatc cttctgtgtt ccgcg                                35

<210> 570
<211> 35
<212> DNA
<213> Artificial Sequence

<220>
<223> Seq. ID NO_570

<400> 570
ctcgcgtgcg attccctatc cttctgtgtc gcgag                                35

<210> 571
<211> 35
<212> DNA
<213> Artificial Sequence

<220>
<223> Seq. ID NO_571

<400> 571
ccgacctgcg attccctatc cttctgtgtg gtcgg                                35

<210> 572
<211> 35
<212> DNA
<213> Artificial Sequence

<220>
<223> Seq. ID NO_572

<400> 572
cgacgttgcg attccctatc cttctgtgta cgtcg                                35

<210> 573
<211> 35
<212> DNA
<213> Artificial Sequence

<220>
<223> Seq. ID NO_573

<400> 573
cccacctgcg attccctatc cttctgtgtg gtggg                                35

<210> 574
<211> 35
<212> DNA
<213> Artificial Sequence

<220>
<223> Seq. ID NO_574

<400> 574
cgccgatgcg attccctatc cttctgtgtt cggcg                                35

<210> 575
<211> 35
<212> DNA
<213> Artificial Sequence

<220>
<223> Seq. ID NO_575

<400> 575
ctggcgtgcg attccctatc cttctgtgtc gccag                                35

<210> 576
<211> 35
<212> DNA
<213> Artificial Sequence

<220>
<223> Seq. ID NO_576

<400> 576
ctgccgtgcg attccctatc cttctgtgtc ggcag                                35

<210> 577
<211> 35
<212> DNA
<213> Artificial Sequence

<220>
<223> Seq. ID NO_577

<400> 577
ccgggttgcg attccctatc cttctgtgta cccgg                                35

<210> 578
<211> 35
<212> DNA
<213> Artificial Sequence

<220>
<223> Seq. ID NO_578

<400> 578
gcgccttgcg attccctatc cttctgtgta ggcgc                                35

<210> 579
<211> 35
<212> DNA
<213> Artificial Sequence

<220>
<223> Seq. ID NO_579

<400> 579
gagcgctgcg attccctatc cttctgtgtg cgctc                                35

<210> 580
<211> 35
<212> DNA
<213> Artificial Sequence

<220>
<223> Seq. ID NO_580

<400> 580
ggctggtgcg attccctatc cttctgtgtc cagcc                                35

<210> 581
<211> 35
<212> DNA
<213> Artificial Sequence

<220>

<223> Seq. ID NO_581

<400> 581
gctgcatgcg attccctatc cttctgtgtt gcagc                                35

<210> 582
<211> 35
<212> DNA
<213> Artificial Sequence

<220>
<223> Seq. ID NO_582

<400> 582
gggtggtgcg attccctatc cttctgtgtc caccc                                35

<210> 583
<211> 35
<212> DNA
<213> Artificial Sequence

<220>
<223> Seq. ID NO_583

<400> 583
gcggcttgcg attccctatc cttctgtgta gccgc                                35

<210> 584
<211> 35
<212> DNA
<213> Artificial Sequence

<220>
<223> Seq. ID NO_584

<400> 584
gaccgctgcg attccctatc cttctgtgtg cggtc                                35

<210> 585
<211> 35
<212> DNA
<213> Artificial Sequence

<220>
<223> Seq. ID NO_585

<400> 585
ccagcctgcg attccctatc cttctgtgtg gctgg                                35

<210> 586
<211> 35
<212> DNA
<213> Artificial Sequence

<220>
<223> Seq. ID NO_586

<400> 586
gacggctgcg attccctatc cttctgtgtg ccgtc                                35


<210> 587
<211> 35
<212> DNA
<213> Artificial Sequence


<220>
<223> Seq. ID NO_587

<400> 587
ggcccatgcg attccctatc cttctgtgtt gggcc                                35


<210> 588
<211> 24
<212> DNA
<213> Artificial Sequence


<220>
<223> Seq. ID NO_588

<400> 588
ggatttgcct acttctcaca ctca                                            24


<210> 589
<211> 36
<212> DNA
<213> Artificial Sequence


<220>
<223> Seq. ID NO_589

<400> 589
cgcggaggat ttgcctactt ctcacactca tccgcg                               36


<210> 590
<211> 36
<212> DNA
<213> Artificial Sequence


<220>
<223> Seq. ID NO_590

<400> 590
ctcgcgggat ttgcctactt ctcacactca cgcgag                               36


<210> 591
<211> 36
<212> DNA

<213> Artificial Sequence

<220>
<223> Seq. ID NO_591

<400> 591
ccgaccggat ttgcctactt ctcacactca ggtcgg 36

<210> 592
<211> 36
<212> DNA
<213> Artificial Sequence

<220>
<223> Seq. ID NO_592

<400> 592
cgacgtggat ttgcctactt ctcacactca acgtcg 36

<210> 593
<211> 36
<212> DNA
<213> Artificial Sequence

<220>
<223> Seq. ID NO_593

<400> 593
cccaccggat ttgcctactt ctcacactca ggtggg 36

<210> 594
<211> 36
<212> DNA
<213> Artificial Sequence

<220>
<223> Seq. ID NO_594

<400> 594
cgccgaggat ttgcctactt ctcacactca tcggcg 36

<210> 595
<211> 36
<212> DNA
<213> Artificial Sequence

<220>
<223> Seq. ID NO_595

<400> 595
ctggcgggat ttgcctactt ctcacactca cgccag 36

<210> 596

<211> 36
<212> DNA
<213> Artificial Sequence

<220>
<223> Seq. ID NO_596

<400> 596
ctgccgggat ttgcctactt ctcacactca cggcag                                36

<210> 597
<211> 36
<212> DNA
<213> Artificial Sequence

<220>
<223> Seq. ID NO_597

<400> 597
ccgggtggat ttgcctactt ctcacactca acccgg                                36

<210> 598
<211> 35
<212> DNA
<213> Artificial Sequence

<220>
<223> Seq. ID NO_598

<400> 598
cccctggatt tgcctacttc tcacactcac agggg                                 35

<210> 599
<211> 36
<212> DNA
<213> Artificial Sequence

<220>
<223> Seq. ID NO_599

<400> 599
gcgcctggat ttgcctactt ctcacactca aggcgc                                36

<210> 600
<211> 36
<212> DNA
<213> Artificial Sequence

<220>
<223> Seq. ID NO_600

<400> 600
gagcgcggat ttgcctactt ctcacactca gcgctc                                36

<210> 601
<211> 36
<212> DNA
<213> Artificial Sequence

<220>
<223> Seq. ID NO_601

<400> 601
ggctggggat ttgcctactt ctcacactca ccagcc                                36

<210> 602
<211> 36
<212> DNA
<213> Artificial Sequence

<220>
<223> Seq. ID NO_602

<400> 602
gctgcaggat ttgcctactt ctcacactca tgcagc                                36

<210> 603
<211> 36
<212> DNA
<213> Artificial Sequence

<220>
<223> Seq. ID NO_603

<400> 603
gggtggggat ttgcctactt ctcacactca ccaccc                                36

<210> 604
<211> 36
<212> DNA
<213> Artificial Sequence

<220>
<223> Seq. ID NO_604

<400> 604
gcggctggat ttgcctactt ctcacactca agccgc                                36

<210> 605
<211> 36
<212> DNA
<213> Artificial Sequence

<220>
<223> Seq. ID NO_605

<400> 605

gaccgcggat ttgcctactt ctcacactca gcggtc 36

<210> 606
<211> 36
<212> DNA
<213> Artificial Sequence

<220>
<223> Seq. ID NO_606

<400> 606
gacggcggat ttgcctactt ctcacactca gccgtc 36

<210> 607
<211> 36
<212> DNA
<213> Artificial Sequence

<220>
<223> Seq. ID NO_607

<400> 607
ggcccaggat ttgcctactt ctcacactca tgggcc 36

<210> 608
<211> 18
<212> DNA
<213> Artificial Sequence

<220>
<223> Seq. ID NO_608

<400> 608
gcacacgtct cttcggct 18

<210> 609
<211> 30
<212> DNA
<213> Artificial Sequence

<220>
<223> Seq. ID NO_609

<400> 609
cgcggagcac acgtctcttc ggcttccgcg 30

<210> 610
<211> 30
<212> DNA
<213> Artificial Sequence

<220>
<223> Seq. ID NO_610

<400> 610
ctcgcggcac acgtctcttc ggctcgcgag                                        30

<210> 611
<211> 30
<212> DNA
<213> Artificial Sequence

<220>
<223> Seq. ID NO_611

<400> 611
ccgaccgcac acgtctcttc ggctggtcgg                                        30

<210> 612
<211> 30
<212> DNA
<213> Artificial Sequence

<220>
<223> Seq. ID NO_612

<400> 612
cgacgtgcac acgtctcttc ggctacgtcg                                        30

<210> 613
<211> 30
<212> DNA
<213> Artificial Sequence

<220>
<223> Seq. ID NO_613

<400> 613
cccaccgcac acgtctcttc ggctggtggg                                        30

<210> 614
<211> 30
<212> DNA
<213> Artificial Sequence

<220>
<223> Seq. ID NO_614

<400> 614
cgccgagcac acgtctcttc ggcttcggcg                                        30

<210> 615
<211> 30
<212> DNA
<213> Artificial Sequence

<220>
<223> Seq. ID NO_615

<400> 615
ctggcggcac acgtctcttc ggctcgccag                                        30


<210> 616
<211> 30
<212> DNA
<213> Artificial Sequence


<220>
<223> Seq. ID NO_616

<400> 616
ctgccggcac acgtctcttc ggctcggcag                                        30


<210> 617
<211> 30
<212> DNA
<213> Artificial Sequence


<220>
<223> Seq. ID NO_617

<400> 617
ccgggtgcac acgtctcttc ggctacccgg                                        30


<210> 618
<211> 30
<212> DNA
<213> Artificial Sequence


<220>
<223> Seq. ID NO_618

<400> 618
gcgcctgcac acgtctcttc ggctaggcgc                                        30


<210> 619
<211> 30
<212> DNA
<213> Artificial Sequence


<220>
<223> Seq. ID NO_619

<400> 619
gagcgcgcac acgtctcttc ggctgcgctc                                        30


<210> 620
<211> 30
<212> DNA
<213> Artificial Sequence

<220>
<223> Seq. ID NO_620

<400> 620
ggctgggcac acgtctcttc ggctccagcc                                        30


<210> 621
<211> 30
<212> DNA
<213> Artificial Sequence


<220>
<223> Seq. ID NO_621

<400> 621
gctgcagcac acgtctcttc ggcttgcagc                                        30


<210> 622
<211> 30
<212> DNA
<213> Artificial Sequence


<220>
<223> Seq. ID NO_622

<400> 622
gggtgggcac acgtctcttc ggctccaccc                                        30


<210> 623
<211> 30
<212> DNA
<213> Artificial Sequence


<220>
<223> Seq. ID NO_623

<400> 623
gcggctgcac acgtctcttc ggctagccgc                                        30


<210> 624
<211> 30
<212> DNA
<213> Artificial Sequence


<220>
<223> Seq. ID NO_624

<400> 624
gaccgcgcac acgtctcttc ggctgcggtc                                        30


<210> 625
<211> 30

<212> DNA
<213> Artificial Sequence

<220>
<223> Seq. ID NO_625

<400> 625
gacggcgcac acgtctcttc ggctgccgtc                                        30

<210> 626
<211> 30
<212> DNA
<213> Artificial Sequence

<220>
<223> Seq. ID NO_626

<400> 626
ggcccagcac acgtctcttc ggcttgggcc                                        30

<210> 627
<211> 19
<212> DNA
<213> Artificial Sequence

<220>
<223> Seq. ID NO_627

<400> 627
accattgcct cctcgtatg                                                    19

<210> 628
<211> 31
<212> DNA
<213> Artificial Sequence

<220>
<223> Seq. ID NO_628

<400> 628
cgcggaacca ttgcctcctc gtatgtccgc g                                      31

<210> 629
<211> 31
<212> DNA
<213> Artificial Sequence

<220>
<223> Seq. ID NO_629

<400> 629
ctcgcgacca ttgcctcctc gtatgcgcga g                                      31

<210> 630
<211> 31
<212> DNA
<213> Artificial Sequence

<220>
<223> Seq. ID NO_630

<400> 630
ccgaccacca ttgcctcctc gtatgggtcg g                                    31

<210> 631
<211> 31
<212> DNA
<213> Artificial Sequence

<220>
<223> Seq. ID NO_631

<400> 631
cgacgtacca ttgcctcctc gtatgacgtc g                                    31

<210> 632
<211> 31
<212> DNA
<213> Artificial Sequence

<220>
<223> Seq. ID NO_632

<400> 632
cccaccacca ttgcctcctc gtatgggtgg g                                    31

<210> 633
<211> 31
<212> DNA
<213> Artificial Sequence

<220>
<223> Seq. ID NO_633

<400> 633
cgccgaacca ttgcctcctc gtatgtcggc g                                    31

<210> 634
<211> 31
<212> DNA
<213> Artificial Sequence

<220>
<223> Seq. ID NO_634

<400> 634
ctggcgacca ttgcctcctc gtatgcgcca g                                    31

<210> 635
<211> 31
<212> DNA
<213> Artificial Sequence

<220>
<223> Seq. ID NO_635

<400> 635
ctgccgacca ttgcctcctc gtatgcggca g                                    31

<210> 636
<211> 31
<212> DNA
<213> Artificial Sequence

<220>
<223> Seq. ID NO_636

<400> 636
ccgggtacca ttgcctcctc gtatgacccg g                                    31

<210> 637
<211> 31
<212> DNA
<213> Artificial Sequence

<220>
<223> Seq. ID NO_637

<400> 637
gcgcctacca ttgcctcctc gtatgaggcg c                                    31

<210> 638
<211> 31
<212> DNA
<213> Artificial Sequence

<220>
<223> Seq. ID NO_638

<400> 638
gagcgcacca ttgcctcctc gtatggcgct c                                    31

<210> 639
<211> 31
<212> DNA
<213> Artificial Sequence

<220>
<223> Seq. ID NO_639

<400> 639
ggctggacca ttgcctcctc gtatgccagc c                                    31

<210> 640
<211> 31
<212> DNA
<213> Artificial Sequence

<220>
<223> Seq. ID NO_640

<400> 640
gctgcaacca ttgcctcctc gtatgtgcag c                                    31

<210> 641
<211> 31
<212> DNA
<213> Artificial Sequence

<220>
<223> Seq. ID NO_641

<400> 641
gggtggacca ttgcctcctc gtatgccacc c                                    31

<210> 642
<211> 31
<212> DNA
<213> Artificial Sequence

<220>
<223> Seq. ID NO_642

<400> 642
gcggctacca ttgcctcctc gtatgagccg c                                    31

<210> 643
<211> 31
<212> DNA
<213> Artificial Sequence

<220>
<223> Seq. ID NO_643

<400> 643
gaccgcacca ttgcctcctc gtatggcggt c                                    31

<210> 644
<211> 31
<212> DNA
<213> Artificial Sequence

<220>

<223> Seq. ID NO_644

<400> 644
gacggcacca ttgcctcctc gtatggccgt c                                    31


<210> 645
<211> 31
<212> DNA
<213> Artificial Sequence


<220>
<223> Seq. ID NO_645

<400> 645
ggcccaacca ttgcctcctc gtatgtgggc c                                    31


<210> 646
<211> 16
<212> DNA
<213> Artificial Sequence


<220>
<223> Seq. ID NO_646

<400> 646
cacgcgctaa gcgtgg                                                     16


<210> 647
<211> 28
<212> DNA
<213> Artificial Sequence


<220>
<223> Seq. ID NO_647

<400> 647
cgcggacacg cgctaagcgt ggtccgcg                                        28


<210> 648
<211> 28
<212> DNA
<213> Artificial Sequence


<220>
<223> Seq. ID NO_648

<400> 648
ctcgcgcacg cgctaagcgt ggcgcgag                                        28


<210> 649
<211> 28
<212> DNA
<213> Artificial Sequence

<220>
<223> Seq. ID NO_649

<400> 649
ccgacccacg cgctaagcgt ggggtcgg                                          28


<210> 650
<211> 28
<212> DNA
<213> Artificial Sequence


<220>
<223> Seq. ID NO_650

<400> 650
cgacgtcacg cgctaagcgt ggacgtcg                                          28


<210> 651
<211> 28
<212> DNA
<213> Artificial Sequence


<220>
<223> Seq. ID NO_651

<400> 651
cccacccacg cgctaagcgt ggggtggg                                          28


<210> 652
<211> 28
<212> DNA
<213> Artificial Sequence


<220>
<223> Seq. ID NO_652

<400> 652
cgccgacacg cgctaagcgt ggtcggcg                                          28


<210> 653
<211> 28
<212> DNA
<213> Artificial Sequence


<220>
<223> Seq. ID NO_653

<400> 653
ctggcgcacg cgctaagcgt ggcgccag                                          28


<210> 654
<211> 28
<212> DNA

<213> Artificial Sequence

<220>
<223> Seq. ID NO_654

<400> 654
ctgccgcacg cgctaagcgt ggcggcag                                          28

<210> 655
<211> 28
<212> DNA
<213> Artificial Sequence

<220>
<223> Seq. ID NO_655

<400> 655
ccgggtcacg cgctaagcgt ggacccgg                                          28

<210> 656
<211> 28
<212> DNA
<213> Artificial Sequence

<220>
<223> Seq. ID NO_656

<400> 656
gcgcctcacg cgctaagcgt ggaggcgc                                          28

<210> 657
<211> 28
<212> DNA
<213> Artificial Sequence

<220>
<223> Seq. ID NO_657

<400> 657
gagcgccacg cgctaagcgt gggcgctc                                          28

<210> 658
<211> 28
<212> DNA
<213> Artificial Sequence

<220>
<223> Seq. ID NO_658

<400> 658
ggctggcacg cgctaagcgt ggccagcc                                          28

<210> 659

<211> 28
<212> DNA
<213> Artificial Sequence

<220>
<223> Seq. ID NO_659

<400> 659
gctgcacacg cgctaagcgt ggtgcagc                                          28

<210> 660
<211> 28
<212> DNA
<213> Artificial Sequence

<220>
<223> Seq. ID NO_660

<400> 660
gggtggcacg cgctaagcgt ggccaccc                                          28

<210> 661
<211> 28
<212> DNA
<213> Artificial Sequence

<220>
<223> Seq. ID NO_661

<400> 661
gcggctcacg cgctaagcgt ggagccgc                                          28

<210> 662
<211> 28
<212> DNA
<213> Artificial Sequence

<220>
<223> Seq. ID NO_662

<400> 662
gaccgccacg cgctaagcgt gggcggtc                                          28

<210> 663
<211> 28
<212> DNA
<213> Artificial Sequence

<220>
<223> Seq. ID NO_663

<400> 663
gacggccacg cgctaagcgt gggccgtc                                          28

<210> 664
<211> 28
<212> DNA
<213> Artificial Sequence

<220>
<223> Seq. ID NO_664

<400> 664
ggcccacacg cgctaagcgt ggtgggcc                                          28

<210> 665
<211> 22
<212> DNA
<213> Artificial Sequence

<220>
<223> Seq. ID NO_665

<400> 665
atgtcgccat acttccactc ca                                                22

<210> 666
<211> 34
<212> DNA
<213> Artificial Sequence

<220>
<223> Seq. ID NO_666

<400> 666
cgcggaatgt cgccatactt ccactccatc cgcg                                   34

<210> 667
<211> 34
<212> DNA
<213> Artificial Sequence

<220>
<223> Seq. ID NO_667

<400> 667
ctcgcgatgt cgccatactt ccactccacg cgag                                   34

<210> 668
<211> 34
<212> DNA
<213> Artificial Sequence

<220>
<223> Seq. ID NO_668

<400> 668

ccgaccatgt cgccatactt ccactccagg tcgg 34

<210> 669
<211> 34
<212> DNA
<213> Artificial Sequence

<220>
<223> Seq. ID NO_669

<400> 669
cgacgtatgt cgccatactt ccactccaac gtcg 34

<210> 670
<211> 34
<212> DNA
<213> Artificial Sequence

<220>
<223> Seq. ID NO_670

<400> 670
cccaccatgt cgccatactt ccactccagg tggg 34

<210> 671
<211> 34
<212> DNA
<213> Artificial Sequence

<220>
<223> Seq. ID NO_671

<400> 671
cgccgaatgt cgccatactt ccactccatc ggcg 34

<210> 672
<211> 34
<212> DNA
<213> Artificial Sequence

<220>
<223> Seq. ID NO_672

<400> 672
ctggcgatgt cgccatactt ccactccacg ccag 34

<210> 673
<211> 34
<212> DNA
<213> Artificial Sequence

<220>
<223> Seq. ID NO_673

<400> 673
ctgccgatgt cgccatactt ccactccacg gcag                                    34

<210> 674
<211> 34
<212> DNA
<213> Artificial Sequence

<220>
<223> Seq. ID NO_674

<400> 674
ccgggtatgt cgccatactt ccactccaac ccgg                                    34

<210> 675
<211> 34
<212> DNA
<213> Artificial Sequence

<220>
<223> Seq. ID NO_675

<400> 675
cgcgctatgt cgccatactt ccactccaag cgcg                                    34

<210> 676
<211> 34
<212> DNA
<213> Artificial Sequence

<220>
<223> Seq. ID NO_676

<400> 676
gcgcctatgt cgccatactt ccactccaag gcgc                                    34

<210> 677
<211> 34
<212> DNA
<213> Artificial Sequence

<220>
<223> Seq. ID NO_677

<400> 677
gagcgcatgt cgccatactt ccactccagc gctc                                    34

<210> 678
<211> 34
<212> DNA
<213> Artificial Sequence

<220>
<223> Seq. ID NO_678

<400> 678
ggctggatgt cgccatactt ccactccacc agcc                                    34


<210> 679
<211> 34
<212> DNA
<213> Artificial Sequence


<220>
<223> Seq. ID NO_679

<400> 679
gctgcaatgt cgccatactt ccactccatg cagc                                    34


<210> 680
<211> 34
<212> DNA
<213> Artificial Sequence


<220>
<223> Seq. ID NO_680

<400> 680
gggtggatgt cgccatactt ccactccacc accc                                    34


<210> 681
<211> 34
<212> DNA
<213> Artificial Sequence


<220>
<223> Seq. ID NO_681

<400> 681
gcggctatgt cgccatactt ccactccaag ccgc                                    34


<210> 682
<211> 34
<212> DNA
<213> Artificial Sequence


<220>
<223> Seq. ID NO_682

<400> 682
gaccgcatgt cgccatactt ccactccagc ggtc                                    34


<210> 683
<211> 34
<212> DNA
<213> Artificial Sequence

<220>
<223> Seq. ID NO_683

<400> 683
gacggcatgt cgccatactt ccactccagc cgtc                                    34


<210> 684
<211> 34
<212> DNA
<213> Artificial Sequence


<220>
<223> Seq. ID NO_684

<400> 684
ggcccaatgt cgccatactt ccactccatg ggcc                                    34


<210> 685
<211> 21
<212> DNA
<213> Artificial Sequence


<220>
<223> Seq. ID NO_685

<400> 685
gcctttccag accgcttcat t                                                  21


<210> 686
<211> 33
<212> DNA
<213> Artificial Sequence


<220>
<223> Seq. ID NO_686

<400> 686
cgcggagcct ttccagaccg cttcatttcc gcg                                     33


<210> 687
<211> 33
<212> DNA
<213> Artificial Sequence


<220>
<223> Seq. ID NO_687

<400> 687
ctcgcggcct ttccagaccg cttcattcgc gag                                     33


<210> 688
<211> 33

<212> DNA
<213> Artificial Sequence

<220>
<223> Seq. ID NO_688

<400> 688
ccgaccgcct ttccagaccg cttcattggt cgg 33

<210> 689
<211> 33
<212> DNA
<213> Artificial Sequence

<220>
<223> Seq. ID NO_689

<400> 689
cgacgtgcct ttccagaccg cttcattacg tcg 33

<210> 690
<211> 32
<212> DNA
<213> Artificial Sequence

<220>
<223> Seq. ID NO_690

<400> 690
ctcccgcctt tccagaccgc ttcattcggg ag 32

<210> 691
<211> 33
<212> DNA
<213> Artificial Sequence

<220>
<223> Seq. ID NO_691

<400> 691
cccaccgcct ttccagaccg cttcattggt ggg 33

<210> 692
<211> 33
<212> DNA
<213> Artificial Sequence

<220>
<223> Seq. ID NO_692

<400> 692
cgccgagcct ttccagaccg cttcatttcg gcg 33

<210> 693
<211> 33
<212> DNA
<213> Artificial Sequence

<220>
<223> Seq. ID NO_693

<400> 693
ctggcggcct ttccagaccg cttcattcgc cag                                33

<210> 694
<211> 33
<212> DNA
<213> Artificial Sequence

<220>
<223> Seq. ID NO_694

<400> 694
ctgccggcct ttccagaccg cttcattcgg cag                                33

<210> 695
<211> 33
<212> DNA
<213> Artificial Sequence

<220>
<223> Seq. ID NO_695

<400> 695
ccgggtgcct ttccagaccg cttcattacc cgg                                33

<210> 696
<211> 33
<212> DNA
<213> Artificial Sequence

<220>
<223> Seq. ID NO_696

<400> 696
gcgcctgcct ttccagaccg cttcattagg cgc                                33

<210> 697
<211> 33
<212> DNA
<213> Artificial Sequence

<220>
<223> Seq. ID NO_697

<400> 697
gagcgcgcct ttccagaccg cttcattgcg ctc                                33

<210> 698
<211> 33
<212> DNA
<213> Artificial Sequence

<220>
<223> Seq. ID NO_698

<400> 698
ggctgggcct ttccagaccg cttcattcca gcc                                    33

<210> 699
<211> 33
<212> DNA
<213> Artificial Sequence

<220>
<223> Seq. ID NO_699

<400> 699
gctgcagcct ttccagaccg cttcatttgc agc                                    33

<210> 700
<211> 33
<212> DNA
<213> Artificial Sequence

<220>
<223> Seq. ID NO_700

<400> 700
gggtgggcct ttccagaccg cttcattcca ccc                                    33

<210> 701
<211> 33
<212> DNA
<213> Artificial Sequence

<220>
<223> Seq. ID NO_701

<400> 701
gcggctgcct ttccagaccg cttcattagc cgc                                    33

<210> 702
<211> 33
<212> DNA
<213> Artificial Sequence

<220>
<223> Seq. ID NO_702

<400> 702
gaccgcgcct ttccagaccg cttcattgcg gtc                                    33


<210> 703
<211> 33
<212> DNA
<213> Artificial Sequence


<220>
<223> Seq. ID NO_703

<400> 703
gacggcgcct ttccagaccg cttcattgcc gtc                                    33


<210> 704
<211> 33
<212> DNA
<213> Artificial Sequence


<220>
<223> Seq. ID NO_704

<400> 704
ggcccagcct ttccagaccg cttcatttgg gcc                                    33


<210> 705
<211> 22
<212> DNA
<213> Artificial Sequence


<220>
<223> Seq. ID NO_705

<400> 705
tcgggtctac gacctgttac tt                                                22


<210> 706
<211> 34
<212> DNA
<213> Artificial Sequence


<220>
<223> Seq. ID NO_706

<400> 706
cgcggatcgg gtctacgacc tgttactttc cgcg                                   34


<210> 707
<211> 34
<212> DNA
<213> Artificial Sequence


<220>

<223> Seq. ID NO_707

<400> 707
ctcgcgtcgg gtctacgacc tgttacttcg cgag                                    34


<210> 708
<211> 34
<212> DNA
<213> Artificial Sequence


<220>
<223> Seq. ID NO_708

<400> 708
ccgacctcgg gtctacgacc tgttacttgg tcgg                                    34


<210> 709
<211> 34
<212> DNA
<213> Artificial Sequence


<220>
<223> Seq. ID NO_709

<400> 709
cgacgttcgg gtctacgacc tgttacttac gtcg                                    34


<210> 710
<211> 34
<212> DNA
<213> Artificial Sequence


<220>
<223> Seq. ID NO_710

<400> 710
cccacctcgg gtctacgacc tgttacttgg tggg                                    34


<210> 711
<211> 34
<212> DNA
<213> Artificial Sequence


<220>
<223> Seq. ID NO_711

<400> 711
cgccgatcgg gtctacgacc tgttactttc ggcg                                    34


<210> 712
<211> 34
<212> DNA
<213> Artificial Sequence

<220>
<223> Seq. ID NO_712

<400> 712
ctggcgtcgg gtctacgacc tgttacttcg ccag                                    34


<210> 713
<211> 34
<212> DNA
<213> Artificial Sequence


<220>
<223> Seq. ID NO_713

<400> 713
ctgccgtcgg gtctacgacc tgttacttcg gcag                                    34


<210> 714
<211> 34
<212> DNA
<213> Artificial Sequence


<220>
<223> Seq. ID NO_714

<400> 714
ccgggttcgg gtctacgacc tgttacttac ccgg                                    34


<210> 715
<211> 34
<212> DNA
<213> Artificial Sequence


<220>
<223> Seq. ID NO_715

<400> 715
gcgccttcgg gtctacgacc tgttacttag gcgc                                    34


<210> 716
<211> 34
<212> DNA
<213> Artificial Sequence


<220>
<223> Seq. ID NO_716

<400> 716
gagcgctcgg gtctacgacc tgttacttgc gctc                                    34


<210> 717
<211> 34
<212> DNA

<213> Artificial Sequence

<220>
<223> Seq. ID NO_717

<400> 717
ggctggtcgg gtctacgacc tgttacttcc agcc 34

<210> 718
<211> 34
<212> DNA
<213> Artificial Sequence

<220>
<223> Seq. ID NO_718

<400> 718
gctgcatcgg gtctacgacc tgttactttg cagc 34

<210> 719
<211> 34
<212> DNA
<213> Artificial Sequence

<220>
<223> Seq. ID NO_719

<400> 719
gggtggtcgg gtctacgacc tgttacttcc accc 34

<210> 720
<211> 34
<212> DNA
<213> Artificial Sequence

<220>
<223> Seq. ID NO_720

<400> 720
gcggcttcgg gtctacgacc tgttacttag ccgc 34

<210> 721
<211> 34
<212> DNA
<213> Artificial Sequence

<220>
<223> Seq. ID NO_721

<400> 721
gaccgctcgg gtctacgacc tgttacttgc ggtc 34

<210> 722

<211> 32
<212> DNA
<213> Artificial Sequence

<220>
<223> Seq. ID NO_722

<400> 722
cccctcgggt ctacgacctg ttacttgagg gg                                    32

<210> 723
<211> 34
<212> DNA
<213> Artificial Sequence

<220>
<223> Seq. ID NO_723

<400> 723
gacggctcgg gtctacgacc tgttacttgc cgtc                                  34

<210> 724
<211> 34
<212> DNA
<213> Artificial Sequence

<220>
<223> Seq. ID NO_724

<400> 724
ggcccatcgg gtctacgacc tgttactttg ggcc                                  34

<210> 725
<211> 24
<212> DNA
<213> Artificial Sequence

<220>
<223> Seq. ID NO_725

<400> 725
accttagata ttcggtggaa ggga                                             24

<210> 726
<211> 36
<212> DNA
<213> Artificial Sequence

<220>
<223> Seq. ID NO_726

<400> 726
cgcggaacct tagatattcg gtggaaggga tccgcg                                36

<210> 727
<211> 36
<212> DNA
<213> Artificial Sequence

<220>
<223> Seq. ID NO_727

<400> 727
ctcgcgacct tagatattcg gtggaaggga cgcgag 36

<210> 728
<211> 36
<212> DNA
<213> Artificial Sequence

<220>
<223> Seq. ID NO_728

<400> 728
ccgaccacct tagatattcg gtggaaggga ggtcgg 36

<210> 729
<211> 36
<212> DNA
<213> Artificial Sequence

<220>
<223> Seq. ID NO_729

<400> 729
cgacgtacct tagatattcg gtggaaggga acgtcg 36

<210> 730
<211> 36
<212> DNA
<213> Artificial Sequence

<220>
<223> Seq. ID NO_730

<400> 730
cccaccacct tagatattcg gtggaaggga ggtggg 36

<210> 731
<211> 36
<212> DNA
<213> Artificial Sequence

<220>
<223> Seq. ID NO_731

<400> 731

cgccgaacct tagatattcg gtggaaggga tcggcg                                36

<210> 732
<211> 36
<212> DNA
<213> Artificial Sequence

<220>
<223> Seq. ID NO_732

<400> 732
ctggcgacct tagatattcg gtggaaggga cgccag                                36

<210> 733
<211> 36
<212> DNA
<213> Artificial Sequence

<220>
<223> Seq. ID NO_733

<400> 733
ctgccgacct tagatattcg gtggaaggga cggcag                                36

<210> 734
<211> 36
<212> DNA
<213> Artificial Sequence

<220>
<223> Seq. ID NO_734

<400> 734
ccgggtacct tagatattcg gtggaaggga acccgg                                36

<210> 735
<211> 36
<212> DNA
<213> Artificial Sequence

<220>
<223> Seq. ID NO_735

<400> 735
gcgcctacct tagatattcg gtggaaggga aggcgc                                36

<210> 736
<211> 36
<212> DNA
<213> Artificial Sequence

<220>
<223> Seq. ID NO_736

<400> 736
gagcgcacct tagatattcg gtggaaggga gcgctc                                36

<210> 737
<211> 36
<212> DNA
<213> Artificial Sequence

<220>
<223> Seq. ID NO_737

<400> 737
ggctggacct tagatattcg gtggaaggga ccagcc                                36

<210> 738
<211> 36
<212> DNA
<213> Artificial Sequence

<220>
<223> Seq. ID NO_738

<400> 738
gctgcaacct tagatattcg gtggaaggga tgcagc                                36

<210> 739
<211> 36
<212> DNA
<213> Artificial Sequence

<220>
<223> Seq. ID NO_739

<400> 739
gggtggacct tagatattcg gtggaaggga ccaccc                                36

<210> 740
<211> 36
<212> DNA
<213> Artificial Sequence

<220>
<223> Seq. ID NO_740

<400> 740
gcggctacct tagatattcg gtggaaggga agccgc                                36

<210> 741
<211> 36
<212> DNA
<213> Artificial Sequence

<220>
<223> Seq. ID NO_741

<400> 741
gaccgcacct tagatattcg gtggaaggga gcggtc                                36


<210> 742
<211> 31
<212> DNA
<213> Artificial Sequence


<220>
<223> Seq. ID NO_742

<400> 742
ccaccttaga tattcggtgg aagggaggtg g                                     31


<210> 743
<211> 36
<212> DNA
<213> Artificial Sequence


<220>
<223> Seq. ID NO_743

<400> 743
gacggcacct tagatattcg gtggaaggga gccgtc                                36


<210> 744
<211> 36
<212> DNA
<213> Artificial Sequence


<220>
<223> Seq. ID NO_744

<400> 744
ggcccaacct tagatattcg gtggaaggga tgggcc                                36


<210> 745
<211> 24
<212> DNA
<213> Artificial Sequence


<220>
<223> Seq. ID NO_745

<400> 745
gaacttccgt acttaatttc cttc                                             24


<210> 746
<211> 36
<212> DNA
<213> Artificial Sequence

<220>
<223> Seq. ID NO_746

<400> 746
cgcggagaac ttccgtactt aatttccttc tccgcg                                36


<210> 747
<211> 36
<212> DNA
<213> Artificial Sequence


<220>
<223> Seq. ID NO_747

<400> 747
ctcgcggaac ttccgtactt aatttccttc cgcgag                                36


<210> 748
<211> 36
<212> DNA
<213> Artificial Sequence


<220>
<223> Seq. ID NO_748

<400> 748
ccgaccgaac ttccgtactt aatttccttc ggtcgg                                36


<210> 749
<211> 36
<212> DNA
<213> Artificial Sequence


<220>
<223> Seq. ID NO_749

<400> 749
cgacgtgaac ttccgtactt aatttccttc acgtcg                                36


<210> 750
<211> 36
<212> DNA
<213> Artificial Sequence


<220>
<223> Seq. ID NO_750

<400> 750
cccaccgaac ttccgtactt aatttccttc ggtggg                                36


<210> 751
<211> 36

<212> DNA
<213> Artificial Sequence

<220>
<223> Seq. ID NO_751

<400> 751
cgccgagaac ttccgtactt aatttccttc tcggcg 36

<210> 752
<211> 36
<212> DNA
<213> Artificial Sequence

<220>
<223> Seq. ID NO_752

<400> 752
ctggcggaac ttccgtactt aatttccttc cgccag 36

<210> 753
<211> 36
<212> DNA
<213> Artificial Sequence

<220>
<223> Seq. ID NO_753

<400> 753
ctgccggaac ttccgtactt aatttccttc cggcag 36

<210> 754
<211> 36
<212> DNA
<213> Artificial Sequence

<220>
<223> Seq. ID NO_754

<400> 754
ccgggtgaac ttccgtactt aatttccttc acccgg 36

<210> 755
<211> 36
<212> DNA
<213> Artificial Sequence

<220>
<223> Seq. ID NO_755

<400> 755
gcgcctgaac ttccgtactt aatttccttc aggcgc 36

<210> 756
<211> 36
<212> DNA
<213> Artificial Sequence

<220>
<223> Seq. ID NO_756

<400> 756
gagcgcgaac ttccgtactt aatttccttc gcgctc                                36

<210> 757
<211> 36
<212> DNA
<213> Artificial Sequence

<220>
<223> Seq. ID NO_757

<400> 757
ggctgggaac ttccgtactt aatttccttc ccagcc                                36

<210> 758
<211> 36
<212> DNA
<213> Artificial Sequence

<220>
<223> Seq. ID NO_758

<400> 758
gctgcagaac ttccgtactt aatttccttc tgcagc                                36

<210> 759
<211> 36
<212> DNA
<213> Artificial Sequence

<220>
<223> Seq. ID NO_759

<400> 759
gggtgggaac ttccgtactt aatttccttc ccaccc                                36

<210> 760
<211> 36
<212> DNA
<213> Artificial Sequence

<220>
<223> Seq. ID NO_760

<400> 760
gcggctgaac ttccgtactt aatttccttc agccgc                                36

<210> 761
<211> 36
<212> DNA
<213> Artificial Sequence

<220>
<223> Seq. ID NO_761

<400> 761
gaccgcgaac ttccgtactt aatttccttc gcggtc                                36

<210> 762
<211> 36
<212> DNA
<213> Artificial Sequence

<220>
<223> Seq. ID NO_762

<400> 762
gacggcgaac ttccgtactt aatttccttc gccgtc                                36

<210> 763
<211> 36
<212> DNA
<213> Artificial Sequence

<220>
<223> Seq. ID NO_763

<400> 763
ggcccagaac ttccgtactt aatttccttc tgggcc                                36

<210> 764
<211> 34
<212> DNA
<213> Artificial Sequence

<220>
<223> Seq. ID NO_764

<400> 764
ccttcgaact tccgtactta atttccttcg aagg                                  34

<210> 765
<211> 20
<212> DNA
<213> Artificial Sequence

<220>
<223> Seq. ID NO_765

<400> 765
tttgcggtac gggcagtact                                                    20

<210> 766
<211> 32
<212> DNA
<213> Artificial Sequence

<220>
<223> Seq. ID NO_766

<400> 766
cgcggatttg cggtacgggc agtacttccg cg                                      32

<210> 767
<211> 32
<212> DNA
<213> Artificial Sequence

<220>
<223> Seq. ID NO_767

<400> 767
ctcgcgtttg cggtacgggc agtactcgcg ag                                      32

<210> 768
<211> 32
<212> DNA
<213> Artificial Sequence

<220>
<223> Seq. ID NO_768

<400> 768
ccgacctttg cggtacgggc agtactggtc gg                                      32

<210> 769
<211> 32
<212> DNA
<213> Artificial Sequence

<220>
<223> Seq. ID NO_769

<400> 769
cgacgttttg cggtacgggc agtactacgt cg                                      32

<210> 770
<211> 32
<212> DNA
<213> Artificial Sequence

<220>

<223> Seq. ID NO_770

<400> 770
cccacctttg cggtacgggc agtactggtg gg                                    32


<210> 771
<211> 32
<212> DNA
<213> Artificial Sequence


<220>
<223> Seq. ID NO_771

<400> 771
cgccgatttg cggtacgggc agtacttcgg cg                                    32


<210> 772
<211> 32
<212> DNA
<213> Artificial Sequence


<220>
<223> Seq. ID NO_772

<400> 772
ctggcgtttg cggtacgggc agtactcgcc ag                                    32


<210> 773
<211> 32
<212> DNA
<213> Artificial Sequence


<220>
<223> Seq. ID NO_773

<400> 773
ctgccgtttg cggtacgggc agtactcggc ag                                    32


<210> 774
<211> 32
<212> DNA
<213> Artificial Sequence


<220>
<223> Seq. ID NO_774

<400> 774
ccgggttttg cggtacgggc agtactaccc gg                                    32


<210> 775
<211> 32
<212> DNA
<213> Artificial Sequence

<220>
<223> Seq. ID NO_775

<400> 775
gcgccttttg cggtacgggc agtactaggc gc                                    32


<210> 776
<211> 25
<212> DNA
<213> Artificial Sequence


<220>
<223> Seq. ID NO_776

<400> 776
ccagtagcgg tacgggcagt actgg                                           25


<210> 777
<211> 32
<212> DNA
<213> Artificial Sequence


<220>
<223> Seq. ID NO_777

<400> 777
gagcgctttg cggtacgggc agtactgcgc tc                                   32


<210> 778
<211> 32
<212> DNA
<213> Artificial Sequence


<220>
<223> Seq. ID NO_778

<400> 778
ggctggtttg cggtacgggc agtactccag cc                                   32


<210> 779
<211> 32
<212> DNA
<213> Artificial Sequence


<220>
<223> Seq. ID NO_779

<400> 779
gctgcatttg cggtacgggc agtacttgca gc                                   32


<210> 780
<211> 32
<212> DNA

<213> Artificial Sequence

<220>
<223> Seq. ID NO_780

<400> 780
gggtggtttg cggtacgggc agtactccac cc                                    32

<210> 781
<211> 32
<212> DNA
<213> Artificial Sequence

<220>
<223> Seq. ID NO_781

<400> 781
gcggcttttg cggtacgggc agtactagcc gc                                    32

<210> 782
<211> 32
<212> DNA
<213> Artificial Sequence

<220>
<223> Seq. ID NO_782

<400> 782
gaccgctttg cggtacgggc agtactgcgg tc                                    32

<210> 783
<211> 32
<212> DNA
<213> Artificial Sequence

<220>
<223> Seq. ID NO_783

<400> 783
gacggctttg cggtacgggc agtactgccg tc                                    32

<210> 784
<211> 32
<212> DNA
<213> Artificial Sequence

<220>
<223> Seq. ID NO_784

<400> 784
ggcccatttg cggtacgggc agtacttggg cc                                    32

<210> 785

<211> 20
<212> DNA
<213> Artificial Sequence

<220>
<223> Seq. ID NO_785

<400> 785
ggcagtacta ctcttcctac                                                   20

<210> 786
<211> 32
<212> DNA
<213> Artificial Sequence

<220>
<223> Seq. ID NO_786

<400> 786
cgcggaggca gtactactct tcctactccg cg                                     32

<210> 787
<211> 32
<212> DNA
<213> Artificial Sequence

<220>
<223> Seq. ID NO_787

<400> 787
ctcgcgggca gtactactct tcctaccgcg ag                                     32

<210> 788
<211> 32
<212> DNA
<213> Artificial Sequence

<220>
<223> Seq. ID NO_788

<400> 788
ccgaccggca gtactactct tcctacggtc gg                                     32

<210> 789
<211> 32
<212> DNA
<213> Artificial Sequence

<220>
<223> Seq. ID NO_789

<400> 789
cgacgtggca gtactactct tcctacacgt cg                                     32

<210> 790
<211> 32
<212> DNA
<213> Artificial Sequence

<220>
<223> Seq. ID NO_790

<400> 790
cccaccggca gtactactct tcctacggtg gg                                    32

<210> 791
<211> 32
<212> DNA
<213> Artificial Sequence

<220>
<223> Seq. ID NO_791

<400> 791
cgccgaggca gtactactct tcctactcgg cg                                    32

<210> 792
<211> 32
<212> DNA
<213> Artificial Sequence

<220>
<223> Seq. ID NO_792

<400> 792
ctggcgggca gtactactct tcctaccgcc ag                                    32

<210> 793
<211> 32
<212> DNA
<213> Artificial Sequence

<220>
<223> Seq. ID NO_793

<400> 793
ctgccgggca gtactactct tcctaccggc ag                                    32

<210> 794
<211> 32
<212> DNA
<213> Artificial Sequence

<220>
<223> Seq. ID NO_794

<400> 794

ccgggtggca gtactactct tcctacaccc gg                                    32

<210> 795
<211> 32
<212> DNA
<213> Artificial Sequence

<220>
<223> Seq. ID NO_795

<400> 795
gcgcctggca gtactactct tcctacaggc gc                                    32

<210> 796
<211> 32
<212> DNA
<213> Artificial Sequence

<220>
<223> Seq. ID NO_796

<400> 796
gagcgcggca gtactactct tcctacgcgc tc                                    32

<210> 797
<211> 32
<212> DNA
<213> Artificial Sequence

<220>
<223> Seq. ID NO_797

<400> 797
ggctggggca gtactactct tcctacccag cc                                    32

<210> 798
<211> 32
<212> DNA
<213> Artificial Sequence

<220>
<223> Seq. ID NO_798

<400> 798
gctgcaggca gtactactct tcctactgca gc                                    32

<210> 799
<211> 32
<212> DNA
<213> Artificial Sequence

<220>
<223> Seq. ID NO_799

<400> 799
gggtggggca gtactactct tcctacccac cc                                    32

<210> 800
<211> 30
<212> DNA
<213> Artificial Sequence

<220>
<223> Seq. ID NO_800

<400> 800
cccctggcag tactactctt cctacagggg                                       30

<210> 801
<211> 32
<212> DNA
<213> Artificial Sequence

<220>
<223> Seq. ID NO_801

<400> 801
gcggctggca gtactactct tcctacagcc gc                                    32

<210> 802
<211> 32
<212> DNA
<213> Artificial Sequence

<220>
<223> Seq. ID NO_802

<400> 802
gaccgcggca gtactactct tcctacgcgg tc                                    32

<210> 803
<211> 32
<212> DNA
<213> Artificial Sequence

<220>
<223> Seq. ID NO_803

<400> 803
gacggcggca gtactactct tcctacgccg tc                                    32

<210> 804
<211> 32
<212> DNA
<213> Artificial Sequence

<220>
<223> Seq. ID NO_804

<400> 804
ggcccaggca gtactactct tcctactggg cc                                    32


<210> 805
<211> 25
<212> DNA
<213> Artificial Sequence


<220>
<223> Seq. ID NO_805

<400> 805
ggtacaaaca ttccatatca ggttg                                            25


<210> 806
<211> 37
<212> DNA
<213> Artificial Sequence


<220>
<223> Seq. ID NO_806

<400> 806
cgcggaggta caaacattcc atatcaggtt gtccgcg                               37


<210> 807
<211> 37
<212> DNA
<213> Artificial Sequence


<220>
<223> Seq. ID NO_807

<400> 807
ctcgcgggta caaacattcc atatcaggtt gcgcgag                               37


<210> 808
<211> 37
<212> DNA
<213> Artificial Sequence


<220>
<223> Seq. ID NO_808

<400> 808
ccgaccggta caaacattcc atatcaggtt gggtcgg                               37


<210> 809
<211> 37
<212> DNA
<213> Artificial Sequence

<220>
<223> Seq. ID NO_809

<400> 809
cgacgtggta caaacattcc atatcaggtt gacgtcg                                37


<210> 810
<211> 37
<212> DNA
<213> Artificial Sequence


<220>
<223> Seq. ID NO_810

<400> 810
cccaccggta caaacattcc atatcaggtt gggtggg                                37


<210> 811
<211> 37
<212> DNA
<213> Artificial Sequence


<220>
<223> Seq. ID NO_811

<400> 811
cgccgaggta caaacattcc atatcaggtt gtcggcg                                37


<210> 812
<211> 37
<212> DNA
<213> Artificial Sequence


<220>
<223> Seq. ID NO_812

<400> 812
ctggcgggta caaacattcc atatcaggtt gcgccag                                37


<210> 813
<211> 37
<212> DNA
<213> Artificial Sequence


<220>
<223> Seq. ID NO_813

<400> 813
ctgccgggta caaacattcc atatcaggtt gcggcag                                37


<210> 814
<211> 37

<212> DNA
<213> Artificial Sequence

<220>
<223> Seq. ID NO_814

<400> 814
ccgggtggta caaacattcc atatcaggtt gacccgg                                37

<210> 815
<211> 37
<212> DNA
<213> Artificial Sequence

<220>
<223> Seq. ID NO_815

<400> 815
gcgcctggta caaacattcc atatcaggtt gaggcgc                                37

<210> 816
<211> 37
<212> DNA
<213> Artificial Sequence

<220>
<223> Seq. ID NO_816

<400> 816
gagcgcggta caaacattcc atatcaggtt ggcgctc                                37

<210> 817
<211> 37
<212> DNA
<213> Artificial Sequence

<220>
<223> Seq. ID NO_817

<400> 817
ggctggggta caaacattcc atatcaggtt gccagcc                                37

<210> 818
<211> 37
<212> DNA
<213> Artificial Sequence

<220>
<223> Seq. ID NO_818

<400> 818
gctgcaggta caaacattcc atatcaggtt gtgcagc                                37

<210> 819
<211> 37
<212> DNA
<213> Artificial Sequence

<220>
<223> Seq. ID NO_819

<400> 819
gggtggggta caaacattcc atatcaggtt gccaccc                                37

<210> 820
<211> 37
<212> DNA
<213> Artificial Sequence

<220>
<223> Seq. ID NO_820

<400> 820
gcggctggta caaacattcc atatcaggtt gagccgc                                37

<210> 821
<211> 37
<212> DNA
<213> Artificial Sequence

<220>
<223> Seq. ID NO_821

<400> 821
gaccgcggta caaacattcc atatcaggtt ggcggtc                                37

<210> 822
<211> 35
<212> DNA
<213> Artificial Sequence

<220>
<223> Seq. ID NO_822

<400> 822
cccccaggta caaacattcc atatcaggtt ggggg                                  35

<210> 823
<211> 37
<212> DNA
<213> Artificial Sequence

<220>
<223> Seq. ID NO_823

<400> 823
gacggcggta caaacattcc atatcaggtt ggccgtc                                37

<210> 824
<211> 37
<212> DNA
<213> Artificial Sequence

<220>
<223> Seq. ID NO_824

<400> 824
ggcccaggta caaacattcc atatcaggtt gtgggcc 37

<210> 825
<211> 19
<212> DNA
<213> Artificial Sequence

<220>
<223> Seq. ID NO_825

<400> 825
cgcgaccctt tgtactatc 19

<210> 826
<211> 31
<212> DNA
<213> Artificial Sequence

<220>
<223> Seq. ID NO_826

<400> 826
cgcggacgcg accctttgta ctatctccgc g 31

<210> 827
<211> 31
<212> DNA
<213> Artificial Sequence

<220>
<223> Seq. ID NO_827

<400> 827
ctcgcgcgcg accctttgta ctatccgcga g 31

<210> 828
<211> 31
<212> DNA
<213> Artificial Sequence

<220>
<223> Seq. ID NO_828

<400> 828
ccgacccgcg accctttgta ctatcggtcg g                                    31

<210> 829
<211> 31
<212> DNA
<213> Artificial Sequence

<220>
<223> Seq. ID NO_829

<400> 829
cgacgtcgcg accctttgta ctatcacgtc g                                    31

<210> 830
<211> 31
<212> DNA
<213> Artificial Sequence

<220>
<223> Seq. ID NO_830

<400> 830
cccacccgcg accctttgta ctatcggtgg g                                    31

<210> 831
<211> 31
<212> DNA
<213> Artificial Sequence

<220>
<223> Seq. ID NO_831

<400> 831
cgccgacgcg accctttgta ctatctcggc g                                    31

<210> 832
<211> 31
<212> DNA
<213> Artificial Sequence

<220>
<223> Seq. ID NO_832

<400> 832
ctggcgcgcg accctttgta ctatccgcca g                                    31

<210> 833
<211> 31
<212> DNA
<213> Artificial Sequence

<220>

<223> Seq. ID NO_833

<400> 833
ctgccgcgcg accctttgta ctatccggca g                                    31


<210> 834
<211> 31
<212> DNA
<213> Artificial Sequence


<220>
<223> Seq. ID NO_834

<400> 834
ccgggtcgcg accctttgta ctatcacccg g                                    31


<210> 835
<211> 31
<212> DNA
<213> Artificial Sequence


<220>
<223> Seq. ID NO_835

<400> 835
cccccccgcg accctttgta ctatcggggg g                                    31


<210> 836
<211> 31
<212> DNA
<213> Artificial Sequence


<220>
<223> Seq. ID NO_836

<400> 836
gcgcctcgcg accctttgta ctatcaggcg c                                    31


<210> 837
<211> 31
<212> DNA
<213> Artificial Sequence


<220>
<223> Seq. ID NO_837

<400> 837
gagcgccgcg accctttgta ctatcgcgct c                                    31


<210> 838
<211> 31
<212> DNA
<213> Artificial Sequence

<220>
<223> Seq. ID NO_838

<400> 838
ggctggcgcg accctttgta ctatcccagc c                                    31


<210> 839
<211> 31
<212> DNA
<213> Artificial Sequence


<220>
<223> Seq. ID NO_839

<400> 839
gctgcacgcg accctttgta ctatctgcag c                                    31


<210> 840
<211> 31
<212> DNA
<213> Artificial Sequence


<220>
<223> Seq. ID NO_840

<400> 840
gggtggcgcg accctttgta ctatcccacc c                                    31


<210> 841
<211> 31
<212> DNA
<213> Artificial Sequence


<220>
<223> Seq. ID NO_841

<400> 841
gcggctcgcg accctttgta ctatcagccg c                                    31


<210> 842
<211> 31
<212> DNA
<213> Artificial Sequence


<220>
<223> Seq. ID NO_842

<400> 842
gaccgccgcg accctttgta ctatcgcggt c                                    31


<210> 843
<211> 31
<212> DNA

<213> Artificial Sequence

<220>
<223> Seq. ID NO_843

<400> 843
gacggccgcg accctttgta ctatcgccgt c                                    31

<210> 844
<211> 31
<212> DNA
<213> Artificial Sequence

<220>
<223> Seq. ID NO_844

<400> 844
ggcccacgcg accctttgta ctatctgggc c                                    31

<210> 845
<211> 25
<212> DNA
<213> Artificial Sequence

<220>
<223> Seq. ID NO_845

<400> 845
aatagtttta tgggattagc tccac                                           25

<210> 846
<211> 37
<212> DNA
<213> Artificial Sequence

<220>
<223> Seq. ID NO_846

<400> 846
cgcggaaata gttttatggg attagctcca ctccgcg                              37

<210> 847
<211> 37
<212> DNA
<213> Artificial Sequence

<220>
<223> Seq. ID NO_847

<400> 847
ctcgcgaata gttttatggg attagctcca ccgcgag                              37

<210> 848

<211> 37
<212> DNA
<213> Artificial Sequence

<220>
<223> Seq. ID NO_848

<400> 848
ccgaccaata gttttatggg attagctcca cggtcgg                                37

<210> 849
<211> 37
<212> DNA
<213> Artificial Sequence

<220>
<223> Seq. ID NO_849

<400> 849
cgacgtaata gttttatggg attagctcca cacgtcg                                37

<210> 850
<211> 37
<212> DNA
<213> Artificial Sequence

<220>
<223> Seq. ID NO_850

<400> 850
cccaccaata gttttatggg attagctcca cggtggg                                37

<210> 851
<211> 37
<212> DNA
<213> Artificial Sequence

<220>
<223> Seq. ID NO_851

<400> 851
cgccgaaata gttttatggg attagctcca ctcggcg                                37

<210> 852
<211> 37
<212> DNA
<213> Artificial Sequence

<220>
<223> Seq. ID NO_852

<400> 852
ctggcgaata gttttatggg attagctcca ccgccag                                37

<210> 853
<211> 37
<212> DNA
<213> Artificial Sequence

<220>
<223> Seq. ID NO_853

<400> 853
ctgccgaata gttttatggg attagctcca ccggcag                                37

<210> 854
<211> 37
<212> DNA
<213> Artificial Sequence

<220>
<223> Seq. ID NO_854

<400> 854
ccgggtaata gttttatggg attagctcca cacccgg                                37

<210> 855
<211> 37
<212> DNA
<213> Artificial Sequence

<220>
<223> Seq. ID NO_855

<400> 855
ccccccaata gttttatggg attagctcca cgggggg                                37

<210> 856
<211> 33
<212> DNA
<213> Artificial Sequence

<220>
<223> Seq. ID NO_856

<400> 856
ccgtggaata gttttatggg attagctcca cgg                                    33

<210> 857
<211> 37
<212> DNA
<213> Artificial Sequence

<220>
<223> Seq. ID NO_857

<400> 857

gcgcctaata gttttatggg attagctcca caggcgc 37

<210> 858
<211> 37
<212> DNA
<213> Artificial Sequence

<220>
<223> Seq. ID NO_858

<400> 858
gagcgcaata gttttatggg attagctcca cgcgctc 37

<210> 859
<211> 37
<212> DNA
<213> Artificial Sequence

<220>
<223> Seq. ID NO_859

<400> 859
ggctggaata gttttatggg attagctcca cccagcc 37

<210> 860
<211> 37
<212> DNA
<213> Artificial Sequence

<220>
<223> Seq. ID NO_860

<400> 860
gctgcaaata gttttatggg attagctcca ctgcagc 37

<210> 861
<211> 37
<212> DNA
<213> Artificial Sequence

<220>
<223> Seq. ID NO_861

<400> 861
gggtggaata gttttatggg attagctcca cccaccc 37

<210> 862
<211> 37
<212> DNA
<213> Artificial Sequence

<220>
<223> Seq. ID NO_862

<400> 862
gcggctaata gttttatggg attagctcca cagccgc                                37

<210> 863
<211> 37
<212> DNA
<213> Artificial Sequence

<220>
<223> Seq. ID NO_863

<400> 863
gaccgcaata gttttatggg attagctcca cgcggtc                                37

<210> 864
<211> 37
<212> DNA
<213> Artificial Sequence

<220>
<223> Seq. ID NO_864

<400> 864
gacggcaata gttttatggg attagctcca cgccgtc                                37

<210> 865
<211> 37
<212> DNA
<213> Artificial Sequence

<220>
<223> Seq. ID NO_865

<400> 865
ggcccaaata gttttatggg attagctcca ctgggcc                                37

<210> 866
<211> 21
<212> DNA
<213> Artificial Sequence

<220>
<223> Seq. ID NO_866

<400> 866
gaaaccatct ttcaaaagcg t                                                 21

<210> 867
<211> 33
<212> DNA
<213> Artificial Sequence

<220>
<223> Seq. ID NO_867

<400> 867
cgcggagaaa ccatctttca aaagcgttcc gcg                                    33


<210> 868
<211> 33
<212> DNA
<213> Artificial Sequence


<220>
<223> Seq. ID NO_868

<400> 868
ctcgcggaaa ccatctttca aaagcgtcgc gag                                    33


<210> 869
<211> 30
<212> DNA
<213> Artificial Sequence


<220>
<223> Seq. ID NO_869

<400> 869
ccccgaaacc atctttcaaa agcgtcgggg                                        30


<210> 870
<211> 33
<212> DNA
<213> Artificial Sequence


<220>
<223> Seq. ID NO_870

<400> 870
ccgaccgaaa ccatctttca aaagcgtggt cgg                                    33


<210> 871
<211> 33
<212> DNA
<213> Artificial Sequence


<220>
<223> Seq. ID NO_871

<400> 871
cgacgtgaaa ccatctttca aaagcgtacg tcg                                    33


<210> 872
<211> 33
<212> DNA
<213> Artificial Sequence

<220>
<223> Seq. ID NO_872

<400> 872
cccaccgaaa ccatctttca aaagcgtggt ggg                                    33


<210> 873
<211> 33
<212> DNA
<213> Artificial Sequence


<220>
<223> Seq. ID NO_873

<400> 873
cgccgagaaa ccatctttca aaagcgttcg gcg                                    33


<210> 874
<211> 33
<212> DNA
<213> Artificial Sequence


<220>
<223> Seq. ID NO_874

<400> 874
ctggcggaaa ccatctttca aaagcgtcgc cag                                    33


<210> 875
<211> 33
<212> DNA
<213> Artificial Sequence


<220>
<223> Seq. ID NO_875

<400> 875
ctgccggaaa ccatctttca aaagcgtcgg cag                                    33


<210> 876
<211> 33
<212> DNA
<213> Artificial Sequence


<220>
<223> Seq. ID NO_876

<400> 876
ccgggtgaaa ccatctttca aaagcgtacc cgg                                    33


<210> 877
<211> 33

<212> DNA
<213> Artificial Sequence

<220>
<223> Seq. ID NO_877

<400> 877
ccccccgaaa ccatctttca aaagcgtggg ggg                                    33

<210> 878
<211> 33
<212> DNA
<213> Artificial Sequence

<220>
<223> Seq. ID NO_878

<400> 878
gcgcctgaaa ccatctttca aaagcgtagg cgc                                    33

<210> 879
<211> 33
<212> DNA
<213> Artificial Sequence

<220>
<223> Seq. ID NO_879

<400> 879
gagcgcgaaa ccatctttca aaagcgtgcg ctc                                    33

<210> 880
<211> 33
<212> DNA
<213> Artificial Sequence

<220>
<223> Seq. ID NO_880

<400> 880
ggctgggaaa ccatctttca aaagcgtcca gcc                                    33

<210> 881
<211> 33
<212> DNA
<213> Artificial Sequence

<220>
<223> Seq. ID NO_881

<400> 881
gctgcagaaa ccatctttca aaagcgttgc agc                                    33

<210> 882
<211> 33
<212> DNA
<213> Artificial Sequence

<220>
<223> Seq. ID NO_882

<400> 882
gggtgggaaa ccatctttca aaagcgtcca ccc                                    33

<210> 883
<211> 33
<212> DNA
<213> Artificial Sequence

<220>
<223> Seq. ID NO_883

<400> 883
gcggctgaaa ccatctttca aaagcgtagc cgc                                    33

<210> 884
<211> 33
<212> DNA
<213> Artificial Sequence

<220>
<223> Seq. ID NO_884

<400> 884
gaccgcgaaa ccatctttca aaagcgtgcg gtc                                    33

<210> 885
<211> 33
<212> DNA
<213> Artificial Sequence

<220>
<223> Seq. ID NO_885

<400> 885
gacggcgaaa ccatctttca aaagcgtgcc gtc                                    33

<210> 886
<211> 33
<212> DNA
<213> Artificial Sequence

<220>
<223> Seq. ID NO_886

<400> 886
ggcccagaaa ccatctttca aaagcgttgg gcc                                    33

<210> 887
<211> 14
<212> DNA
<213> Artificial Sequence

<220>
<223> Seq. ID NO_887

<400> 887
tggcatgcgc caca                                                          14

<210> 888
<211> 26
<212> DNA
<213> Artificial Sequence

<220>
<223> Seq. ID NO_888

<400> 888
cgcggatggc atgcgccaca tccgcg                                             26

<210> 889
<211> 26
<212> DNA
<213> Artificial Sequence

<220>
<223> Seq. ID NO_889

<400> 889
ctcgcgtggc atgcgccaca cgcgag                                             26

<210> 890
<211> 26
<212> DNA
<213> Artificial Sequence

<220>
<223> Seq. ID NO_890

<400> 890
ccgacctggc atgcgccaca ggtcgg                                             26

<210> 891
<211> 26
<212> DNA
<213> Artificial Sequence

<220>
<223> Seq. ID NO_891

<400> 891
cgacgttggc atgcgccaca acgtcg                                          26

<210> 892
<211> 26
<212> DNA
<213> Artificial Sequence

<220>
<223> Seq. ID NO_892

<400> 892
cccacctggc atgcgccaca ggtggg                                          26

<210> 893
<211> 26
<212> DNA
<213> Artificial Sequence

<220>
<223> Seq. ID NO_893

<400> 893
cgccgatggc atgcgccaca tcggcg                                          26

<210> 894
<211> 26
<212> DNA
<213> Artificial Sequence

<220>
<223> Seq. ID NO_894

<400> 894
ctggcgtggc atgcgccaca cgccag                                          26

<210> 895
<211> 26
<212> DNA
<213> Artificial Sequence

<220>
<223> Seq. ID NO_895

<400> 895
ctgccgtggc atgcgccaca cggcag                                          26

<210> 896
<211> 26
<212> DNA
<213> Artificial Sequence

<220>

<223> Seq. ID NO_896

<400> 896
ccgggttggc atgcgccaca acccgg                                          26


<210> 897
<211> 26
<212> DNA
<213> Artificial Sequence


<220>
<223> Seq. ID NO_897

<400> 897
   cccccctggc atgcgccaca gggggg                                          26


<210> 898
<211> 26
<212> DNA
<213> Artificial Sequence


<220>
<223> Seq. ID NO_898

<400> 898
gcgccttggc atgcgccaca aggcgc                                          26


<210> 899
<211> 26
<212> DNA
<213> Artificial Sequence


<220>
<223> Seq. ID NO_899

<400> 899
gagcgctggc atgcgccaca gcgctc                                          26


<210> 900
<211> 26
<212> DNA
<213> Artificial Sequence


<220>
<223> Seq. ID NO_900

<400> 900
ggctggtggc atgcgccaca ccagcc                                          26


<210> 901
<211> 26
<212> DNA
<213> Artificial Sequence

<220>
<223> Seq. ID NO_901

<400> 901
gctgcatggc atgcgccaca tgcagc                                            26


<210> 902
<211> 26
<212> DNA
<213> Artificial Sequence


<220>
<223> Seq. ID NO_902

<400> 902
gggtggtggc atgcgccaca ccaccc                                            26


<210> 903
<211> 26
<212> DNA
<213> Artificial Sequence


<220>
<223> Seq. ID NO_903

<400> 903
gcggcttggc atgcgccaca agccgc                                            26


<210> 904
<211> 26
<212> DNA
<213> Artificial Sequence


<220>
<223> Seq. ID NO_904

<400> 904
gaccgctggc atgcgccaca gcggtc                                            26


<210> 905
<211> 26
<212> DNA
<213> Artificial Sequence


<220>
<223> Seq. ID NO_905

<400> 905
gacggctggc atgcgccaca gccgtc                                            26


<210> 906
<211> 26
<212> DNA

<213> Artificial Sequence

<220>
<223> Seq. ID NO_906

<400> 906
ggcccatggc atgcgccaca tgggcc 26

<210> 907
<211> 22
<212> DNA
<213> Artificial Sequence

<220>
<223> Seq. ID NO_907

<400> 907
cctggcatgc gccacagcca gg 22

<210> 908
<211> 20
<212> DNA
<213> Artificial Sequence

<220>
<223> Seq. ID NO_908

<400> 908
catgcgccac actttatcat 20

<210> 909
<211> 32
<212> DNA
<213> Artificial Sequence

<220>
<223> Seq. ID NO_909

<400> 909
cgcggacatg cgccacactt tatcattccg cg 32

<210> 910
<211> 32
<212> DNA
<213> Artificial Sequence

<220>
<223> Seq. ID NO_910

<400> 910
ctcgcgcatg cgccacactt tatcatcgcg ag 32

<210> 911

<211> 32
<212> DNA
<213> Artificial Sequence

<220>
<223> Seq. ID NO_911

<400> 911
ccgacccatg cgccacactt tatcatggtc gg                                    32

<210> 912
<211> 32
<212> DNA
<213> Artificial Sequence

<220>
<223> Seq. ID NO_912

<400> 912
cgacgtcatg cgccacactt tatcatacgt cg                                    32

<210> 913
<211> 32
<212> DNA
<213> Artificial Sequence

<220>
<223> Seq. ID NO_913

<400> 913
cccacccatg cgccacactt tatcatggtg gg                                    32

<210> 914
<211> 32
<212> DNA
<213> Artificial Sequence

<220>
<223> Seq. ID NO_914

<400> 914
cgccgacatg cgccacactt tatcattcgg cg                                    32

<210> 915
<211> 32
<212> DNA
<213> Artificial Sequence

<220>
<223> Seq. ID NO_915

<400> 915
ctggcgcatg cgccacactt tatcatcgcc ag                                    32

<210> 916
<211> 32
<212> DNA
<213> Artificial Sequence

<220>
<223> Seq. ID NO_916

<400> 916
ctgccgcatg cgccacactt tatcatcggc ag                                    32

<210> 917
<211> 32
<212> DNA
<213> Artificial Sequence

<220>
<223> Seq. ID NO_917

<400> 917
ccgggtcatg cgccacactt tatcataccc gg                                    32

<210> 918
<211> 32
<212> DNA
<213> Artificial Sequence

<220>
<223> Seq. ID NO_918

<400> 918
cccccccatg cgccacactt tatcatgggg gg                                    32

<210> 919
<211> 32
<212> DNA
<213> Artificial Sequence

<220>
<223> Seq. ID NO_919

<400> 919
gcgcctcatg cgccacactt tatcataggc gc                                    32

<210> 920
<211> 32
<212> DNA
<213> Artificial Sequence

<220>
<223> Seq. ID NO_920

<400> 920

gagcgccatg cgccacactt tatcatgcgc tc                                    32

<210> 921
<211> 32
<212> DNA
<213> Artificial Sequence

<220>
<223> Seq. ID NO_921

<400> 921
ggctggcatg cgccacactt tatcatccag cc                                    32

<210> 922
<211> 32
<212> DNA
<213> Artificial Sequence

<220>
<223> Seq. ID NO_922

<400> 922
gctgcacatg cgccacactt tatcattgca gc                                    32

<210> 923
<211> 32
<212> DNA
<213> Artificial Sequence

<220>
<223> Seq. ID NO_923

<400> 923
gggtggcatg cgccacactt tatcatccac cc                                    32

<210> 924
<211> 32
<212> DNA
<213> Artificial Sequence

<220>
<223> Seq. ID NO_924

<400> 924
gcggctcatg cgccacactt tatcatagcc gc                                    32

<210> 925
<211> 32
<212> DNA
<213> Artificial Sequence

<220>
<223> Seq. ID NO_925

<400> 925
gaccgccatg cgccacactt tatcatgcgg tc                                    32

<210> 926
<211> 32
<212> DNA
<213> Artificial Sequence

<220>
<223> Seq. ID NO_926

<400> 926
gacggccatg cgccacactt tatcatgccg tc                                    32

<210> 927
<211> 32
<212> DNA
<213> Artificial Sequence

<220>
<223> Seq. ID NO_927

<400> 927
ggcccacatg cgccacactt tatcattggg cc                                    32

<210> 928
<211> 22
<212> DNA
<213> Artificial Sequence

<220>
<223> Seq. ID NO_928

<400> 928
aagcagttac tcttatcctt gt                                               22

<210> 929
<211> 34
<212> DNA
<213> Artificial Sequence

<220>
<223> Seq. ID NO_929

<400> 929
cgcggaaagc agttactctt atccttgttc cgcg                                  34

<210> 930
<211> 34
<212> DNA
<213> Artificial Sequence

<220>
<223> Seq. ID NO_930

<400> 930
ctcgcgaagc agttactctt atccttgtcg cgag                                    34


<210> 931
<211> 34
<212> DNA
<213> Artificial Sequence


<220>
<223> Seq. ID NO_931

<400> 931
ccgaccaagc agttactctt atccttgtgg tcgg                                    34


<210> 932
<211> 34
<212> DNA
<213> Artificial Sequence


<220>
<223> Seq. ID NO_932

<400> 932
cgacgtaagc agttactctt atccttgtac gtcg                                    34


<210> 933
<211> 34
<212> DNA
<213> Artificial Sequence


<220>
<223> Seq. ID NO_933

<400> 933
cccaccaagc agttactctt atccttgtgg tggg                                    34


<210> 934
<211> 34
<212> DNA
<213> Artificial Sequence


<220>
<223> Seq. ID NO_934

<400> 934
cgccgaaagc agttactctt atccttgttc ggcg                                    34


<210> 935
<211> 34
<212> DNA
<213> Artificial Sequence

<220>
<223> Seq. ID NO_935

<400> 935
ctggcgaagc agttactctt atccttgtcg ccag                                    34


<210> 936
<211> 34
<212> DNA
<213> Artificial Sequence


<220>
<223> Seq. ID NO_936

<400> 936
ctgccgaagc agttactctt atccttgtcg gcag                                    34


<210> 937
<211> 34
<212> DNA
<213> Artificial Sequence


<220>
<223> Seq. ID NO_937

<400> 937
ccgggtaagc agttactctt atccttgtac ccgg                                    34


<210> 938
<211> 34
<212> DNA
<213> Artificial Sequence


<220>
<223> Seq. ID NO_938

<400> 938
ccccccaagc agttactctt atccttgtgg gggg                                    34


<210> 939
<211> 34
<212> DNA
<213> Artificial Sequence


<220>
<223> Seq. ID NO_939

<400> 939
gcgcctaagc agttactctt atccttgtag gcgc                                    34


<210> 940
<211> 34

<212> DNA
<213> Artificial Sequence

<220>
<223> Seq. ID NO_940

<400> 940
gagcgcaagc agttactctt atccttgtgc gctc                                    34

<210> 941
<211> 34
<212> DNA
<213> Artificial Sequence

<220>
<223> Seq. ID NO_941

<400> 941
ggctggaagc agttactctt atccttgtcc agcc                                    34

<210> 942
<211> 34
<212> DNA
<213> Artificial Sequence

<220>
<223> Seq. ID NO_942

<400> 942
gctgcaaagc agttactctt atccttgttg cagc                                    34

<210> 943
<211> 34
<212> DNA
<213> Artificial Sequence

<220>
<223> Seq. ID NO_943

<400> 943
gggtggaagc agttactctt atccttgtcc accc                                    34

<210> 944
<211> 34
<212> DNA
<213> Artificial Sequence

<220>
<223> Seq. ID NO_944

<400> 944
gcggctaagc agttactctt atccttgtag ccgc                                    34

<210> 945
<211> 34
<212> DNA
<213> Artificial Sequence

<220>
<223> Seq. ID NO_945

<400> 945
gaccgcaagc agttactctt atccttgtgc ggtc                                    34

<210> 946
<211> 34
<212> DNA
<213> Artificial Sequence

<220>
<223> Seq. ID NO_946

<400> 946
gacggcaagc agttactctt atccttgtgc cgtc                                    34

<210> 947
<211> 34
<212> DNA
<213> Artificial Sequence

<220>
<223> Seq. ID NO_947

<400> 947
ggcccaaagc agttactctt atccttgttg ggcc                                    34

<210> 948
<211> 20
<212> DNA
<213> Artificial Sequence

<220>
<223> Seq. ID NO_948

<400> 948
cggagttatc cccaacttac                                                    20

<210> 949
<211> 32
<212> DNA
<213> Artificial Sequence

<220>
<223> Seq. ID NO_949

<400> 949
cgcggacgga gttatcccca acttactccg cg                                      32

<210> 950
<211> 32
<212> DNA
<213> Artificial Sequence

<220>
<223> Seq. ID NO_950

<400> 950
ctcgcgcgga gttatcccca acttaccgcg ag                                    32

<210> 951
<211> 32
<212> DNA
<213> Artificial Sequence

<220>
<223> Seq. ID NO_951

<400> 951
ccgacccgga gttatcccca acttacggtc gg                                    32

<210> 952
<211> 32
<212> DNA
<213> Artificial Sequence

<220>
<223> Seq. ID NO_952

<400> 952
cgacgtcgga gttatcccca acttacacgt cg                                    32

<210> 953
<211> 32
<212> DNA
<213> Artificial Sequence

<220>
<223> Seq. ID NO_953

<400> 953
cccacccgga gttatcccca acttacggtg gg                                    32

<210> 954
<211> 32
<212> DNA
<213> Artificial Sequence

<220>
<223> Seq. ID NO_954

<400> 954
cgccgacgga gttatcccca acttactcgg cg                                    32

<210> 955
<211> 32
<212> DNA
<213> Artificial Sequence

<220>
<223> Seq. ID NO_955

<400> 955
ctggcgcgga gttatcccca acttaccgcc ag                                    32

<210> 956
<211> 32
<212> DNA
<213> Artificial Sequence

<220>
<223> Seq. ID NO_956

<400> 956
ctgccgcgga gttatcccca acttaccggc ag                                    32

<210> 957
<211> 32
<212> DNA
<213> Artificial Sequence

<220>
<223> Seq. ID NO_957

<400> 957
ccgggtcgga gttatcccca acttacaccc gg                                    32

<210> 958
<211> 32
<212> DNA
<213> Artificial Sequence

<220>
<223> Seq. ID NO_958

<400> 958
cccccccgga gttatcccca acttacgggg gg                                    32

<210> 959
<211> 32
<212> DNA
<213> Artificial Sequence

<220>

<223> Seq. ID NO_959

<400> 959
gcgcctcgga gttatcccca acttacaggc gc                                    32


<210> 960
<211> 32
<212> DNA
<213> Artificial Sequence


<220>
<223> Seq. ID NO_960

<400> 960
gagcgccgga gttatcccca acttacgcgc tc                                    32


<210> 961
<211> 32
<212> DNA
<213> Artificial Sequence


<220>
<223> Seq. ID NO_961

<400> 961
ggctggcgga gttatcccca acttacccag cc                                    32


<210> 962
<211> 32
<212> DNA
<213> Artificial Sequence


<220>
<223> Seq. ID NO_962

<400> 962
gctgcacgga gttatcccca acttactgca gc                                    32


<210> 963
<211> 32
<212> DNA
<213> Artificial Sequence


<220>
<223> Seq. ID NO_963

<400> 963
gggtggcgga gttatcccca acttacccac cc                                    32


<210> 964
<211> 32
<212> DNA
<213> Artificial Sequence

<220>
<223> Seq. ID NO_964

<400> 964
gcggctcgga gttatcccca acttacagcc gc                                    32


<210> 965
<211> 32
<212> DNA
<213> Artificial Sequence


<220>
<223> Seq. ID NO_965

<400> 965
gaccgccgga gttatcccca acttacgcgg tc                                    32


<210> 966
<211> 32
<212> DNA
<213> Artificial Sequence


<220>
<223> Seq. ID NO_966

<400> 966
gacggccgga gttatcccca acttacgccg tc                                    32


<210> 967
<211> 32
<212> DNA
<213> Artificial Sequence


<220>
<223> Seq. ID NO_967

<400> 967
ggcccacgga gttatcccca acttactggg cc                                    32


<210> 968
<211> 22
<212> DNA
<213> Artificial Sequence


<220>
<223> Seq. ID NO_968

<400> 968
ctccatgtat cgctacactt cc                                               22


<210> 969
<211> 34
<212> DNA

<213> Artificial Sequence

<220>
<223> Seq. ID NO_969

<400> 969
cgcggactcc atgtatcgct acacttcctc cgcg                                    34

<210> 970
<211> 34
<212> DNA
<213> Artificial Sequence

<220>
<223> Seq. ID NO_970

<400> 970
ctcgcgctcc atgtatcgct acacttcccg cgag                                    34

<210> 971
<211> 34
<212> DNA
<213> Artificial Sequence

<220>
<223> Seq. ID NO_971

<400> 971
ccgaccctcc atgtatcgct acacttccgg tcgg                                    34

<210> 972
<211> 34
<212> DNA
<213> Artificial Sequence

<220>
<223> Seq. ID NO_972

<400> 972
cgacgtctcc atgtatcgct acacttccac gtcg                                    34

<210> 973
<211> 34
<212> DNA
<213> Artificial Sequence

<220>
<223> Seq. ID NO_973

<400> 973
cccaccctcc atgtatcgct acacttccgg tggg                                    34

<210> 974

<211> 34
<212> DNA
<213> Artificial Sequence

<220>
<223> Seq. ID NO_974

<400> 974
cgccgactcc atgtatcgct acacttcctc ggcg                                    34

<210> 975
<211> 34
<212> DNA
<213> Artificial Sequence

<220>
<223> Seq. ID NO_975

<400> 975
ctggcgctcc atgtatcgct acacttcccg ccag                                    34

<210> 976
<211> 34
<212> DNA
<213> Artificial Sequence

<220>
<223> Seq. ID NO_976

<400> 976
ctgccgctcc atgtatcgct acacttcccg gcag                                    34

<210> 977
<211> 34
<212> DNA
<213> Artificial Sequence

<220>
<223> Seq. ID NO_977

<400> 977
ccgggtctcc atgtatcgct acacttccac ccgg                                    34

<210> 978
<211> 34
<212> DNA
<213> Artificial Sequence

<220>
<223> Seq. ID NO_978

<400> 978
ccccccctcc atgtatcgct acacttccgg gggg                                    34

<210> 979
<211> 34
<212> DNA
<213> Artificial Sequence

<220>
<223> Seq. ID NO_979

<400> 979
gcgcctctcc atgtatcgct acacttccag gcgc                                    34

<210> 980
<211> 34
<212> DNA
<213> Artificial Sequence

<220>
<223> Seq. ID NO_980

<400> 980
gagcgcctcc atgtatcgct acacttccgc gctc                                    34

<210> 981
<211> 34
<212> DNA
<213> Artificial Sequence

<220>
<223> Seq. ID NO_981

<400> 981
ctggcgctcc atgtatcgct acacttcccg ccag                                    34

<210> 982
<211> 34
<212> DNA
<213> Artificial Sequence

<220>
<223> Seq. ID NO_982

<400> 982
ggctggctcc atgtatcgct acacttcccc agcc                                    34

<210> 983
<211> 34
<212> DNA
<213> Artificial Sequence

<220>
<223> Seq. ID NO_983

<400> 983

gctgcactcc atgtatcgct acacttcctg cagc                                    34

<210> 984
<211> 34
<212> DNA
<213> Artificial Sequence

<220>
<223> Seq. ID NO_984

<400> 984
gggtggctcc atgtatcgct acacttcccc accc                                    34

<210> 985
<211> 34
<212> DNA
<213> Artificial Sequence

<220>
<223> Seq. ID NO_985

<400> 985
gcggctctcc atgtatcgct acacttccag ccgc                                    34

<210> 986
<211> 34
<212> DNA
<213> Artificial Sequence

<220>
<223> Seq. ID NO_986

<400> 986
gaccgcctcc atgtatcgct acacttccgc ggtc                                    34

<210> 987
<211> 34
<212> DNA
<213> Artificial Sequence

<220>
<223> Seq. ID NO_987

<400> 987
gacggcctcc atgtatcgct acacttccgc cgtc                                    34

<210> 988
<211> 34
<212> DNA
<213> Artificial Sequence

<220>
<223> Seq. ID NO_988

<400> 988
ggcccactcc atgtatcgct acacttcctg ggcc                                    34

<210> 989
<211> 21
<212> DNA
<213> Artificial Sequence

<220>
<223> Seq. ID NO_989

<400> 989
atgctagcgc acacgtctct t                                                  21

<210> 990
<211> 33
<212> DNA
<213> Artificial Sequence

<220>
<223> Seq. ID NO_990

<400> 990
cgcggaatgc tagcgcacac gtctctttcc gcg                                     33

<210> 991
<211> 33
<212> DNA
<213> Artificial Sequence

<220>
<223> Seq. ID NO_991

<400> 991
ctcgcgatgc tagcgcacac gtctcttcgc gag                                     33

<210> 992
<211> 33
<212> DNA
<213> Artificial Sequence

<220>
<223> Seq. ID NO_992

<400> 992
ccgaccatgc tagcgcacac gtctcttggt cgg                                     33

<210> 993
<211> 33
<212> DNA
<213> Artificial Sequence

<220>
<223> Seq. ID NO_993

<400> 993
cgacgtatgc tagcgcacac gtctcttacg tcg                                    33


<210> 994
<211> 33
<212> DNA
<213> Artificial Sequence


<220>
<223> Seq. ID NO_994

<400> 994
cccaccatgc tagcgcacac gtctcttggt ggg                                    33


<210> 995
<211> 33
<212> DNA
<213> Artificial Sequence


<220>
<223> Seq. ID NO_995

<400> 995
cgccgaatgc tagcgcacac gtctctttcg gcg                                    33


<210> 996
<211> 33
<212> DNA
<213> Artificial Sequence


<220>
<223> Seq. ID NO_996

<400> 996
ctggcgatgc tagcgcacac gtctcttcgc cag                                    33


<210> 997
<211> 30
<212> DNA
<213> Artificial Sequence


<220>
<223> Seq. ID NO_997

<400> 997
cccatgctag cgcacacgtc tcttcatggg                                        30


<210> 998
<211> 33
<212> DNA
<213> Artificial Sequence

<220>
<223> Seq. ID NO_998

<400> 998
ctgccgatgc tagcgcacac gtctcttcgg cag                                    33


<210> 999
<211> 33
<212> DNA
<213> Artificial Sequence


<220>
<223> Seq. ID NO_999

<400> 999
ccgggtatgc tagcgcacac gtctcttacc cgg                                    33


<210> 1000
<211> 33
<212> DNA
<213> Artificial Sequence


<220>
<223> Seq. ID NO_1000

<400> 1000
ccccccatgc tagcgcacac gtctcttggg ggg                                    33


<210> 1001
<211> 33
<212> DNA
<213> Artificial Sequence


<220>
<223> Seq. ID NO_1001

<400> 1001
gcgcctatgc tagcgcacac gtctcttagg cgc                                    33


<210> 1002
<211> 33
<212> DNA
<213> Artificial Sequence


<220>
<223> Seq. ID NO_1002

<400> 1002
gagcgcatgc tagcgcacac gtctcttgcg ctc                                    33


<210> 1003
<211> 33

<212> DNA
<213> Artificial Sequence

<220>
<223> Seq. ID NO_1003

<400> 1003
ggctggatgc tagcgcacac gtctcttcca gcc                                    33

<210> 1004
<211> 33
<212> DNA
<213> Artificial Sequence

<220>
<223> Seq. ID NO_1004

<400> 1004
gctgcaatgc tagcgcacac gtctctttgc agc                                    33

<210> 1005
<211> 33
<212> DNA
<213> Artificial Sequence

<220>
<223> Seq. ID NO_1005

<400> 1005
gggtggatgc tagcgcacac gtctcttcca ccc                                    33

<210> 1006
<211> 33
<212> DNA
<213> Artificial Sequence

<220>
<223> Seq. ID NO_1006

<400> 1006
gcggctatgc tagcgcacac gtctcttagc cgc                                    33

<210> 1007
<211> 33
<212> DNA
<213> Artificial Sequence

<220>
<223> Seq. ID NO_1007

<400> 1007
gaccgcatgc tagcgcacac gtctcttgcg gtc                                    33

<210> 1008
<211> 33
<212> DNA
<213> Artificial Sequence

<220>
<223> Seq. ID NO_1008

<400> 1008
gacggcatgc tagcgcacac gtctcttgcc gtc                                    33

<210> 1009
<211> 33
<212> DNA
<213> Artificial Sequence

<220>
<223> Seq. ID NO_1009

<400> 1009
ggcccaatgc tagcgcacac gtctctttgg gcc                                    33

<210> 1010
<211> 23
<212> DNA
<213> Artificial Sequence

<220>
<223> Seq. ID NO_1010

<400> 1010
gtaccttcgc cgaagctaag cac                                               23

<210> 1011
<211> 35
<212> DNA
<213> Artificial Sequence

<220>
<223> Seq. ID NO_1011

<400> 1011
cgcggagtac cttcgccgaa gctaagcact ccgcg                                  35

<210> 1012
<211> 35
<212> DNA
<213> Artificial Sequence

<220>
<223> Seq. ID NO_1012

<400> 1012
ctcgcggtac cttcgccgaa gctaagcacc gcgag                                  35

<210> 1013
<211> 35
<212> DNA
<213> Artificial Sequence

<220>
<223> Seq. ID NO_1013

<400> 1013
ccgaccgtac cttcgccgaa gctaagcacg gtcgg 35

<210> 1014
<211> 35
<212> DNA
<213> Artificial Sequence

<220>
<223> Seq. ID NO_1014

<400> 1014
cgacgtgtac cttcgccgaa gctaagcaca cgtcg 35

<210> 1015
<211> 35
<212> DNA
<213> Artificial Sequence

<220>
<223> Seq. ID NO_1015

<400> 1015
cccaccgtac cttcgccgaa gctaagcacg gtggg 35

<210> 1016
<211> 35
<212> DNA
<213> Artificial Sequence

<220>
<223> Seq. ID NO_1016

<400> 1016
cgccgagtac cttcgccgaa gctaagcact cggcg 35

<210> 1017
<211> 35
<212> DNA
<213> Artificial Sequence

<220>
<223> Seq. ID NO_1017

<400> 1017
ctggcggtac cttcgccgaa gctaagcacc gccag                                35

<210> 1018
<211> 35
<212> DNA
<213> Artificial Sequence

<220>
<223> Seq. ID NO_1018

<400> 1018
ctgccggtac cttcgccgaa gctaagcacc ggcag                                35

<210> 1019
<211> 35
<212> DNA
<213> Artificial Sequence

<220>
<223> Seq. ID NO_1019

<400> 1019
ccgggtgtac cttcgccgaa gctaagcaca cccgg                                35

<210> 1020
<211> 35
<212> DNA
<213> Artificial Sequence

<220>
<223> Seq. ID NO_1020

<400> 1020
ccccccgtac cttcgccgaa gctaagcacg ggggg                                35

<210> 1021
<211> 35
<212> DNA
<213> Artificial Sequence

<220>
<223> Seq. ID NO_1021

<400> 1021
gcgcctgtac cttcgccgaa gctaagcaca ggcgc                                35

<210> 1022
<211> 35
<212> DNA
<213> Artificial Sequence

<220>

<223> Seq. ID NO_1022

<400> 1022
gagcgcgtac cttcgccgaa gctaagcacg cgctc                                35


<210> 1023
<211> 35
<212> DNA
<213> Artificial Sequence


<220>
<223> Seq. ID NO_1023

<400> 1023
ggctgggtac cttcgccgaa gctaagcacc cagcc                                35


<210> 1024
<211> 35
<212> DNA
<213> Artificial Sequence


<220>
<223> Seq. ID NO_1024

<400> 1024
gctgcagtac cttcgccgaa gctaagcact gcagc                                35


<210> 1025
<211> 35
<212> DNA
<213> Artificial Sequence


<220>
<223> Seq. ID NO_1025

<400> 1025
gggtgggtac cttcgccgaa gctaagcacc caccc                                35


<210> 1026
<211> 35
<212> DNA
<213> Artificial Sequence


<220>
<223> Seq. ID NO_1026

<400> 1026
gcggctgtac cttcgccgaa gctaagcaca gccgc                                35


<210> 1027
<211> 35
<212> DNA
<213> Artificial Sequence

<220>
<223> Seq. ID NO_1027

<400> 1027
gaccgcgtac cttcgccgaa gctaagcacg cggtc                                35


<210> 1028
<211> 35
<212> DNA
<213> Artificial Sequence


<220>
<223> Seq. ID NO_1028

<400> 1028
gacggcgtac cttcgccgaa gctaagcacg ccgtc                                35


<210> 1029
<211> 35
<212> DNA
<213> Artificial Sequence


<220>
<223> Seq. ID NO_1029

<400> 1029
ggcccagtac cttcgccgaa gctaagcact gggcc                                35


<210> 1030
<211> 18
<212> DNA
<213> Artificial Sequence


<220>
<223> Seq. ID NO_1030

<400> 1030
tcgccgaagc taagcact                                                   18


<210> 1031
<211> 30
<212> DNA
<213> Artificial Sequence


<220>
<223> Seq. ID NO_1031

<400> 1031
cgcggatcgc cgaagctaag cacttccgcg                                      30


<210> 1032
<211> 30
<212> DNA

<213> Artificial Sequence

<220>
<223> Seq. ID NO_1032

<400> 1032
ctcgcgtcgc cgaagctaag cactcgcgag 30

<210> 1033
<211> 30
<212> DNA
<213> Artificial Sequence

<220>
<223> Seq. ID NO_1033

<400> 1033
ccgacctcgc cgaagctaag cactggtcgg 30

<210> 1034
<211> 30
<212> DNA
<213> Artificial Sequence

<220>
<223> Seq. ID NO_1034

<400> 1034
cgacgttcgc cgaagctaag cactacgtcg 30

<210> 1035
<211> 30
<212> DNA
<213> Artificial Sequence

<220>
<223> Seq. ID NO_1035

<400> 1035
cccacctcgc cgaagctaag cactggtggg 30

<210> 1036
<211> 30
<212> DNA
<213> Artificial Sequence

<220>
<223> Seq. ID NO_1036

<400> 1036
cgccgatcgc cgaagctaag cacttcggcg 30

<210> 1037

<211> 30
<212> DNA
<213> Artificial Sequence

<220>
<223> Seq. ID NO_1037

<400> 1037
ctggcgtcgc cgaagctaag cactcgccag                                        30

<210> 1038
<211> 30
<212> DNA
<213> Artificial Sequence

<220>
<223> Seq. ID NO_1038

<400> 1038
ctgccgtcgc cgaagctaag cactcggcag                                        30

<210> 1039
<211> 30
<212> DNA
<213> Artificial Sequence

<220>
<223> Seq. ID NO_1039

<400> 1039
ccgggttcgc cgaagctaag cactacccgg                                        30

<210> 1040
<211> 30
<212> DNA
<213> Artificial Sequence

<220>
<223> Seq. ID NO_1040

<400> 1040
cccccctcgc cgaagctaag cactgggggg                                        30

<210> 1041
<211> 29
<212> DNA
<213> Artificial Sequence

<220>
<223> Seq. ID NO_1041

<400> 1041
cccccatcgc cgaagctaag cactggggg                                         29

<210> 1042
<211> 30
<212> DNA
<213> Artificial Sequence

<220>
<223> Seq. ID NO_1042

<400> 1042
gcgccttcgc cgaagctaag cactaggcgc                                        30

<210> 1043
<211> 30
<212> DNA
<213> Artificial Sequence

<220>
<223> Seq. ID NO_1043

<400> 1043
gagcgctcgc cgaagctaag cactgcgctc                                        30

<210> 1044
<211> 30
<212> DNA
<213> Artificial Sequence

<220>
<223> Seq. ID NO_1044

<400> 1044
ggctggtcgc cgaagctaag cactccagcc                                        30

<210> 1045
<211> 30
<212> DNA
<213> Artificial Sequence

<220>
<223> Seq. ID NO_1045

<400> 1045
gctgcatcgc cgaagctaag cacttgcagc                                        30

<210> 1046
<211> 30
<212> DNA
<213> Artificial Sequence

<220>
<223> Seq. ID NO_1046

<400> 1046

gggtggtcgc cgaagctaag cactccaccc 30

<210> 1047
<211> 30
<212> DNA
<213> Artificial Sequence

<220>
<223> Seq. ID NO_1047

<400> 1047
gcggcttcgc cgaagctaag cactagccgc 30

<210> 1048
<211> 30
<212> DNA
<213> Artificial Sequence

<220>
<223> Seq. ID NO_1048

<400> 1048
gaccgctcgc cgaagctaag cactgcggtc 30

<210> 1049
<211> 30
<212> DNA
<213> Artificial Sequence

<220>
<223> Seq. ID NO_1049

<400> 1049
gacggctcgc cgaagctaag cactgccgtc 30

<210> 1050
<211> 30
<212> DNA
<213> Artificial Sequence

<220>
<223> Seq. ID NO_1050

<400> 1050
ggcccatcgc cgaagctaag cacttgggcc 30

<210> 1051
<211> 17
<212> DNA
<213> Artificial Sequence

<220>
<223> Seq. ID NO_1051

<400> 1051
cgtaccttcg ccgaagc                                                    17


<210> 1052
<211> 29
<212> DNA
<213> Artificial Sequence


<220>
<223> Seq. ID NO_1052

<400> 1052
cgcggacgta ccttcgccga agctccgcg                                       29


<210> 1053
<211> 29
<212> DNA
<213> Artificial Sequence


<220>
<223> Seq. ID NO_1053

<400> 1053
ctcgcgcgta ccttcgccga agccgcgag                                       29


<210> 1054
<211> 29
<212> DNA
<213> Artificial Sequence


<220>
<223> Seq. ID NO_1054

<400> 1054
ccgacccgta ccttcgccga agcggtcgg                                       29


<210> 1055
<211> 29
<212> DNA
<213> Artificial Sequence


<220>
<223> Seq. ID NO_1055

<400> 1055
cgacgtcgta ccttcgccga agcacgtcg                                       29


<210> 1056
<211> 29
<212> DNA
<213> Artificial Sequence

<220>
<223> Seq. ID NO_1056

<400> 1056
cgcgagcgta ccttcgccga agcctcgcg                                          29


<210> 1057
<211> 29
<212> DNA
<213> Artificial Sequence


<220>
<223> Seq. ID NO_1057

<400> 1057
cccacccgta ccttcgccga agcggtggg                                          29


<210> 1058
<211> 29
<212> DNA
<213> Artificial Sequence


<220>
<223> Seq. ID NO_1058

<400> 1058
cgccgacgta ccttcgccga agctcggcg                                          29


<210> 1059
<211> 29
<212> DNA
<213> Artificial Sequence


<220>
<223> Seq. ID NO_1059

<400> 1059
ctggcgcgta ccttcgccga agccgccag                                          29


<210> 1060
<211> 29
<212> DNA
<213> Artificial Sequence


<220>
<223> Seq. ID NO_1060

<400> 1060
ctgccgcgta ccttcgccga agccggcag                                          29


<210> 1061
<211> 29
<212> DNA
<213> Artificial Sequence

<220>
<223> Seq. ID NO_1061

<400> 1061
ccgggtcgta ccttcgccga agcacccgg                                          29


<210> 1062
<211> 29
<212> DNA
<213> Artificial Sequence


<220>
<223> Seq. ID NO_1062

<400> 1062
   cccccccgta ccttcgccga agcgggggg                                          29


<210> 1063
<211> 29
<212> DNA
<213> Artificial Sequence


<220>
<223> Seq. ID NO_1063

<400> 1063
gcgcctcgta ccttcgccga agcaggcgc                                          29


<210> 1064
<211> 29
<212> DNA
<213> Artificial Sequence


<220>
<223> Seq. ID NO_1064

<400> 1064
gagcgccgta ccttcgccga agcgcgctc                                          29


<210> 1065
<211> 29
<212> DNA
<213> Artificial Sequence


<220>
<223> Seq. ID NO_1065

<400> 1065
ggctggcgta ccttcgccga agcccagcc                                          29


<210> 1066
<211> 29

<212> DNA
<213> Artificial Sequence

<220>
<223> Seq. ID NO_1066

<400> 1066
gctgcacgta ccttcgccga agctgcagc                                          29

<210> 1067
<211> 29
<212> DNA
<213> Artificial Sequence

<220>
<223> Seq. ID NO_1067

<400> 1067
gggtggcgta ccttcgccga agcccaccc                                          29

<210> 1068
<211> 29
<212> DNA
<213> Artificial Sequence

<220>
<223> Seq. ID NO_1068

<400> 1068
gcggctcgta ccttcgccga agcagccgc                                          29

<210> 1069
<211> 29
<212> DNA
<213> Artificial Sequence

<220>
<223> Seq. ID NO_1069

<400> 1069
gaccgccgta ccttcgccga agcgcggtc                                          29

<210> 1070
<211> 29
<212> DNA
<213> Artificial Sequence

<220>
<223> Seq. ID NO_1070

<400> 1070
gacggccgta ccttcgccga agcgccgtc                                          29

<210> 1071
<211> 29
<212> DNA
<213> Artificial Sequence

<220>
<223> Seq. ID NO_1071

<400> 1071
ggcccacgta ccttcgccga agctgggcc                                          29

<210> 1072
<211> 23
<212> DNA
<213> Artificial Sequence

<220>
<223> Seq. ID NO_1072

<400> 1072
cagtactact cttcctagag gct                                                23

<210> 1073
<211> 35
<212> DNA
<213> Artificial Sequence

<220>
<223> Seq. ID NO_1073

<400> 1073
cgcggacagt actactcttc ctagaggctt ccgcg                                   35

<210> 1074
<211> 35
<212> DNA
<213> Artificial Sequence

<220>
<223> Seq. ID NO_1074

<400> 1074
ctcgcgcagt actactcttc ctagaggctc gcgag                                   35

<210> 1075
<211> 35
<212> DNA
<213> Artificial Sequence

<220>
<223> Seq. ID NO_1075

<400> 1075
ccgacccagt actactcttc ctagaggctg gtcgg                                   35

<210> 1076
<211> 35
<212> DNA
<213> Artificial Sequence

<220>
<223> Seq. ID NO_1076

<400> 1076
cgacgtcagt actactcttc ctagaggcta cgtcg 35

<210> 1077
<211> 35
<212> DNA
<213> Artificial Sequence

<220>
<223> Seq. ID NO_1077

<400> 1077
cccacccagt actactcttc ctagaggctg gtggg 35

<210> 1078
<211> 35
<212> DNA
<213> Artificial Sequence

<220>
<223> Seq. ID NO_1078

<400> 1078
cgccgacagt actactcttc ctagaggctt cggcg 35

<210> 1079
<211> 35
<212> DNA
<213> Artificial Sequence

<220>
<223> Seq. ID NO_1079

<400> 1079
ctggcgcagt actactcttc ctagaggctc gccag 35

<210> 1080
<211> 35
<212> DNA
<213> Artificial Sequence

<220>
<223> Seq. ID NO_1080

<400> 1080
ctgccgcagt actactcttc ctagaggctc ggcag                                35

<210> 1081
<211> 35
<212> DNA
<213> Artificial Sequence

<220>
<223> Seq. ID NO_1081

<400> 1081
ccgggtcagt actactcttc ctagaggcta cccgg                                35

<210> 1082
<211> 35
<212> DNA
<213> Artificial Sequence

<220>
<223> Seq. ID NO_1082

<400> 1082
cccccccagt actactcttc ctagaggctg ggggg                                35

<210> 1083
<211> 35
<212> DNA
<213> Artificial Sequence

<220>
<223> Seq. ID NO_1083

<400> 1083
gcgcctcagt actactcttc ctagaggcta ggcgc                                35

<210> 1084
<211> 35
<212> DNA
<213> Artificial Sequence

<220>
<223> Seq. ID NO_1084

<400> 1084
gagcgccagt actactcttc ctagaggctg cgctc                                35

<210> 1085
<211> 35
<212> DNA
<213> Artificial Sequence

<220>

<223> Seq. ID NO_1085

<400> 1085
ggctggcagt actactcttc ctagaggctc cagcc                                35


<210> 1086
<211> 35
<212> DNA
<213> Artificial Sequence


<220>
<223> Seq. ID NO_1086

<400> 1086
gctgcacagt actactcttc ctagaggctt gcagc                                35


<210> 1087
<211> 35
<212> DNA
<213> Artificial Sequence


<220>
<223> Seq. ID NO_1087

<400> 1087
gggtggcagt actactcttc ctagaggctc caccc                                35


<210> 1088
<211> 35
<212> DNA
<213> Artificial Sequence


<220>
<223> Seq. ID NO_1088

<400> 1088
gcggctcagt actactcttc ctagaggcta gccgc                                35


<210> 1089
<211> 35
<212> DNA
<213> Artificial Sequence


<220>
<223> Seq. ID NO_1089

<400> 1089
gaccgccagt actactcttc ctagaggctg cggtc                                35


<210> 1090
<211> 35
<212> DNA
<213> Artificial Sequence

<220>
<223> Seq. ID NO_1090

<400> 1090
gacggccagt actactcttc ctagaggctg ccgtc                                35


<210> 1091
<211> 35
<212> DNA
<213> Artificial Sequence


<220>
<223> Seq. ID NO_1091

<400> 1091
ggcccacagt actactcttc ctagaggctt gggcc                                35


<210> 1092
<211> 21
<212> DNA
<213> Artificial Sequence


<220>
<223> Seq. ID NO_1092

<400> 1092
ggcagtacta ctcttcctag a                                               21


<210> 1093
<211> 31
<212> DNA
<213> Artificial Sequence


<220>
<223> Seq. ID NO_1093

<400> 1093
ctgcggcagt actactcttc ctagaccgca g                                    31


<210> 1094
<211> 33
<212> DNA
<213> Artificial Sequence


<220>
<223> Seq. ID NO_1094

<400> 1094
cgcggaggca gtactactct tcctagatcc gcg                                  33


<210> 1095
<211> 33
<212> DNA

<213> Artificial Sequence

<220>
<223> Seq. ID NO_1095

<400> 1095
ctcgcgggca gtactactct tcctagacgc gag 33

<210> 1096
<211> 33
<212> DNA
<213> Artificial Sequence

<220>
<223> Seq. ID NO_1096

<400> 1096
ccgaccggca gtactactct tcctagaggt cgg 33

<210> 1097
<211> 33
<212> DNA
<213> Artificial Sequence

<220>
<223> Seq. ID NO_1097

<400> 1097
cgacgtggca gtactactct tcctagaacg tcg 33

<210> 1098
<211> 33
<212> DNA
<213> Artificial Sequence

<220>
<223> Seq. ID NO_1098

<400> 1098
cccaccggca gtactactct tcctagaggt ggg 33

<210> 1099
<211> 33
<212> DNA
<213> Artificial Sequence

<220>
<223> Seq. ID NO_1099

<400> 1099
cgccgaggca gtactactct tcctagatcg gcg 33

<210> 1100

<211> 33
<212> DNA
<213> Artificial Sequence

<220>
<223> Seq. ID NO_1100

<400> 1100
ctggcgggca gtactactct tcctagacgc cag                                    33

<210> 1101
<211> 33
<212> DNA
<213> Artificial Sequence

<220>
<223> Seq. ID NO_1101

<400> 1101
ctgccgggca gtactactct tcctagacgg cag                                    33

<210> 1102
<211> 33
<212> DNA
<213> Artificial Sequence

<220>
<223> Seq. ID NO_1102

<400> 1102
ccgggtggca gtactactct tcctagaacc cgg                                    33

<210> 1103
<211> 33
<212> DNA
<213> Artificial Sequence

<220>
<223> Seq. ID NO_1103

<400> 1103
ccccccggca gtactactct tcctagaggg ggg                                    33

<210> 1104
<211> 33
<212> DNA
<213> Artificial Sequence

<220>
<223> Seq. ID NO_1104

<400> 1104
gcgcctggca gtactactct tcctagaagg cgc                                    33

<210> 1105
<211> 33
<212> DNA
<213> Artificial Sequence

<220>
<223> Seq. ID NO_1105

<400> 1105
gagcgcggca gtactactct tcctagagcg ctc                                    33

<210> 1106
<211> 33
<212> DNA
<213> Artificial Sequence

<220>
<223> Seq. ID NO_1106

<400> 1106
ggctggggca gtactactct tcctagacca gcc                                    33

<210> 1107
<211> 33
<212> DNA
<213> Artificial Sequence

<220>
<223> Seq. ID NO_1107

<400> 1107
gctgcaggca gtactactct tcctagatgc agc                                    33

<210> 1108
<211> 33
<212> DNA
<213> Artificial Sequence

<220>
<223> Seq. ID NO_1108

<400> 1108
gggtggggca gtactactct tcctagacca ccc                                    33

<210> 1109
<211> 33
<212> DNA
<213> Artificial Sequence

<220>
<223> Seq. ID NO_1109

<400> 1109

gcggctggca gtactactct tcctagaagc cgc                                    33

<210> 1110
<211> 33
<212> DNA
<213> Artificial Sequence

<220>
<223> Seq. ID NO_1110

<400> 1110
gaccgcggca gtactactct tcctagagcg gtc                                    33

<210> 1111
<211> 33
<212> DNA
<213> Artificial Sequence

<220>
<223> Seq. ID NO_1111

<400> 1111
gacggcggca gtactactct tcctagagcc gtc                                    33

<210> 1112
<211> 33
<212> DNA
<213> Artificial Sequence

<220>
<223> Seq. ID NO_1112

<400> 1112
ggcccaggca gtactactct tcctagatgg gcc                                    33

<210> 1113
<211> 23
<212> DNA
<213> Artificial Sequence

<220>
<223> Seq. ID NO_1113

<400> 1113
gggcagtact actcttccta gag                                               23

<210> 1114
<211> 35
<212> DNA
<213> Artificial Sequence

<220>
<223> Seq. ID NO_1114

<400> 1114
cgcggagggc agtactactc ttcctagagt ccgcg                                35

<210> 1115
<211> 35
<212> DNA
<213> Artificial Sequence

<220>
<223> Seq. ID NO_1115

<400> 1115
ctcgcggggc agtactactc ttcctagagc gcgag                                35

<210> 1116
<211> 35
<212> DNA
<213> Artificial Sequence

<220>
<223> Seq. ID NO_1116

<400> 1116
ccgaccgggc agtactactc ttcctagagg gtcgg                                35

<210> 1117
<211> 35
<212> DNA
<213> Artificial Sequence

<220>
<223> Seq. ID NO_1117

<400> 1117
cgacgtgggc agtactactc ttcctagaga cgtcg                                35

<210> 1118
<211> 35
<212> DNA
<213> Artificial Sequence

<220>
<223> Seq. ID NO_1118

<400> 1118
cccaccgggc agtactactc ttcctagagg gtggg                                35

<210> 1119
<211> 35
<212> DNA
<213> Artificial Sequence

<220>
<223> Seq. ID NO_1119

<400> 1119
cgccgagggc agtactactc ttcctagagt cggcg                                35


<210> 1120
<211> 35
<212> DNA
<213> Artificial Sequence


<220>
<223> Seq. ID NO_1120

<400> 1120
ctggcggggc agtactactc ttcctagagc gccag                                35


<210> 1121
<211> 34
<212> DNA
<213> Artificial Sequence


<220>
<223> Seq. ID NO_1121

<400> 1121
ctcgccgggc agtactactc ttcctagagg cgag                                 34


<210> 1122
<211> 35
<212> DNA
<213> Artificial Sequence


<220>
<223> Seq. ID NO_1122

<400> 1122
ctgccggggc agtactactc ttcctagagc ggcag                                35


<210> 1123
<211> 35
<212> DNA
<213> Artificial Sequence


<220>
<223> Seq. ID NO_1123

<400> 1123
ccgggtgggc agtactactc ttcctagaga cccgg                                35


<210> 1124
<211> 35
<212> DNA
<213> Artificial Sequence

<220>
<223> Seq. ID NO_1124

<400> 1124
ccccccgggc agtactactc ttcctagagg ggggg                                35


<210> 1125
<211> 35
<212> DNA
<213> Artificial Sequence


<220>
<223> Seq. ID NO_1125

<400> 1125
gcgcctgggc agtactactc ttcctagaga ggcgc                                35


<210> 1126
<211> 35
<212> DNA
<213> Artificial Sequence


<220>
<223> Seq. ID NO_1126

<400> 1126
gagcgcgggc agtactactc ttcctagagg cgctc                                35


<210> 1127
<211> 35
<212> DNA
<213> Artificial Sequence


<220>
<223> Seq. ID NO_1127

<400> 1127
ggctgggggc agtactactc ttcctagagc cagcc                                35


<210> 1128
<211> 35
<212> DNA
<213> Artificial Sequence


<220>
<223> Seq. ID NO_1128

<400> 1128
gctgcagggc agtactactc ttcctagagt gcagc                                35


<210> 1129
<211> 35

<212> DNA
<213> Artificial Sequence

<220>
<223> Seq. ID NO_1129

<400> 1129
gggtgggggc agtactactc ttcctagagc caccc 35

<210> 1130
<211> 35
<212> DNA
<213> Artificial Sequence

<220>
<223> Seq. ID NO_1130

<400> 1130
gcggctgggc agtactactc ttcctagaga gccgc 35

<210> 1131
<211> 35
<212> DNA
<213> Artificial Sequence

<220>
<223> Seq. ID NO_1131

<400> 1131
gaccgcgggc agtactactc ttcctagagg cggtc 35

<210> 1132
<211> 35
<212> DNA
<213> Artificial Sequence

<220>
<223> Seq. ID NO_1132

<400> 1132
gacggcgggc agtactactc ttcctagagg ccgtc 35

<210> 1133
<211> 35
<212> DNA
<213> Artificial Sequence

<220>
<223> Seq. ID NO_1133

<400> 1133
ggcccagggc agtactactc ttcctagagt gggcc 35

<210> 1134
<211> 23
<212> DNA
<213> Artificial Sequence

<220>
<223> Seq. ID NO_1134

<400> 1134
catcggttaa acaattagca ctg                                              23

<210> 1135
<211> 35
<212> DNA
<213> Artificial Sequence

<220>
<223> Seq. ID NO_1135

<400> 1135
cgcggacatc ggttaaacaa ttagcactgt ccgcg                                 35

<210> 1136
<211> 35
<212> DNA
<213> Artificial Sequence

<220>
<223> Seq. ID NO_1136

<400> 1136
ctcgcgcatc ggttaaacaa ttagcactgc gcgag                                 35

<210> 1137
<211> 35
<212> DNA
<213> Artificial Sequence

<220>
<223> Seq. ID NO_1137

<400> 1137
ccgacccatc ggttaaacaa ttagcactgg gtcgg                                 35

<210> 1138
<211> 35
<212> DNA
<213> Artificial Sequence

<220>
<223> Seq. ID NO_1138

<400> 1138
cgacgtcatc ggttaaacaa ttagcactga cgtcg                                 35

<210> 1139
<211> 35
<212> DNA
<213> Artificial Sequence

<220>
<223> Seq. ID NO_1139

<400> 1139
cccacccatc ggttaaacaa ttagcactgg gtggg 35

<210> 1140
<211> 29
<212> DNA
<213> Artificial Sequence

<220>
<223> Seq. ID NO_1140

<400> 1140
cagtgccatc ggttaaacaa ttagcactg 29

<210> 1141
<211> 35
<212> DNA
<213> Artificial Sequence

<220>
<223> Seq. ID NO_1141

<400> 1141
cgccgacatc ggttaaacaa ttagcactgt cggcg 35

<210> 1142
<211> 35
<212> DNA
<213> Artificial Sequence

<220>
<223> Seq. ID NO_1142

<400> 1142
ctggcgcatc ggttaaacaa ttagcactgc gccag 35

<210> 1143
<211> 35
<212> DNA
<213> Artificial Sequence

<220>
<223> Seq. ID NO_1143

<400> 1143
ctgccgcatc ggttaaacaa ttagcactgc ggcag                                35

<210> 1144
<211> 35
<212> DNA
<213> Artificial Sequence

<220>
<223> Seq. ID NO_1144

<400> 1144
ccgggtcatc ggttaaacaa ttagcactga cccgg                                35

<210> 1145
<211> 35
<212> DNA
<213> Artificial Sequence

<220>
<223> Seq. ID NO_1145

<400> 1145
cccccccatc ggttaaacaa ttagcactgg ggggg                                35

<210> 1146
<211> 35
<212> DNA
<213> Artificial Sequence

<220>
<223> Seq. ID NO_1146

<400> 1146
gcgcctcatc ggttaaacaa ttagcactga ggcgc                                35

<210> 1147
<211> 35
<212> DNA
<213> Artificial Sequence

<220>
<223> Seq. ID NO_1147

<400> 1147
gagcgccatc ggttaaacaa ttagcactgg cgctc                                35

<210> 1148
<211> 35
<212> DNA
<213> Artificial Sequence

<220>

<223> Seq. ID NO_1148

<400> 1148
ggctggcatc ggttaaacaa ttagcactgc cagcc                                35


<210> 1149
<211> 35
<212> DNA
<213> Artificial Sequence


<220>
<223> Seq. ID NO_1149

<400> 1149
gctgcacatc ggttaaacaa ttagcactgt gcagc                                35


<210> 1150
<211> 35
<212> DNA
<213> Artificial Sequence


<220>
<223> Seq. ID NO_1150

<400> 1150
gggtggcatc ggttaaacaa ttagcactgc caccc                                35


<210> 1151
<211> 35
<212> DNA
<213> Artificial Sequence


<220>
<223> Seq. ID NO_1151

<400> 1151
gcggctcatc ggttaaacaa ttagcactga gccgc                                35


<210> 1152
<211> 35
<212> DNA
<213> Artificial Sequence


<220>
<223> Seq. ID NO_1152

<400> 1152
gaccgccatc ggttaaacaa ttagcactgg cggtc                                35


<210> 1153
<211> 35
<212> DNA
<213> Artificial Sequence

<220>
<223> Seq. ID NO_1153

<400> 1153
gacggccatc ggttaaacaa ttagcactgg ccgtc                                35


<210> 1154
<211> 35
<212> DNA
<213> Artificial Sequence


<220>
<223> Seq. ID NO_1154

<400> 1154
ggcccacatc ggttaaacaa ttagcactgt gggcc                                35


<210> 1155
<211> 23
<212> DNA
<213> Artificial Sequence


<220>
<223> Seq. ID NO_1155

<400> 1155
acgaacctct aaagaggttc atc                                             23


<210> 1156
<211> 35
<212> DNA
<213> Artificial Sequence


<220>
<223> Seq. ID NO_1156

<400> 1156
cgcggaacga acctctaaag aggttcatct ccgcg                                35


<210> 1157
<211> 35
<212> DNA
<213> Artificial Sequence


<220>
<223> Seq. ID NO_1157

<400> 1157
ctcgcgacga acctctaaag aggttcatcc gcgag                                35


<210> 1158
<211> 35
<212> DNA

<213> Artificial Sequence

<220>
<223> Seq. ID NO_1158

<400> 1158
ccgaccacga acctctaaag aggttcatcg gtcgg 35

<210> 1159
<211> 35
<212> DNA
<213> Artificial Sequence

<220>
<223> Seq. ID NO_1159

<400> 1159
ctcgcggatg aacctcttta gaggttcgtc gcgag 35

<210> 1160
<211> 35
<212> DNA
<213> Artificial Sequence

<220>
<223> Seq. ID NO_1160

<400> 1160
cgacgtacga acctctaaag aggttcatca cgtcg 35

<210> 1161
<211> 35
<212> DNA
<213> Artificial Sequence

<220>
<223> Seq. ID NO_1161

<400> 1161
cccaccacga acctctaaag aggttcatcg gtggg 35

<210> 1162
<211> 35
<212> DNA
<213> Artificial Sequence

<220>
<223> Seq. ID NO_1162

<400> 1162
cgccgaacga acctctaaag aggttcatct cggcg 35

<210> 1163

<211> 35
<212> DNA
<213> Artificial Sequence

<220>
<223> Seq. ID NO_1163

<400> 1163
ctggcgacga acctctaaag aggttcatcc gccag                                35

<210> 1164
<211> 35
<212> DNA
<213> Artificial Sequence

<220>
<223> Seq. ID NO_1164

<400> 1164
ctgccgacga acctctaaag aggttcatcc ggcag                                35

<210> 1165
<211> 35
<212> DNA
<213> Artificial Sequence

<220>
<223> Seq. ID NO_1165

<400> 1165
ccgggtacga acctctaaag aggttcatca cccgg                                35

<210> 1166
<211> 35
<212> DNA
<213> Artificial Sequence

<220>
<223> Seq. ID NO_1166

<400> 1166
ccccccacga acctctaaag aggttcatcg ggggg                                35

<210> 1167
<211> 35
<212> DNA
<213> Artificial Sequence

<220>
<223> Seq. ID NO_1167

<400> 1167
gcgcctacga acctctaaag aggttcatca ggcgc                                35

<210> 1168
<211> 35
<212> DNA
<213> Artificial Sequence

<220>
<223> Seq. ID NO_1168

<400> 1168
gagcgcacga acctctaaag aggttcatcg cgctc                                35

<210> 1169
<211> 35
<212> DNA
<213> Artificial Sequence

<220>
<223> Seq. ID NO_1169

<400> 1169
ggctggacga acctctaaag aggttcatcc cagcc                                35

<210> 1170
<211> 35
<212> DNA
<213> Artificial Sequence

<220>
<223> Seq. ID NO_1170

<400> 1170
gctgcaacga acctctaaag aggttcatct gcagc                                35

<210> 1171
<211> 35
<212> DNA
<213> Artificial Sequence

<220>
<223> Seq. ID NO_1171

<400> 1171
gggtggacga acctctaaag aggttcatcc caccc                                35

<210> 1172
<211> 35
<212> DNA
<213> Artificial Sequence

<220>
<223> Seq. ID NO_1172

<400> 1172

gcggctacga acctctaaag aggttcatca gccgc                                35

<210> 1173
<211> 35
<212> DNA
<213> Artificial Sequence

<220>
<223> Seq. ID NO_1173

<400> 1173
gaccgcacga acctctaaag aggttcatcg cggtc                                35

<210> 1174
<211> 35
<212> DNA
<213> Artificial Sequence

<220>
<223> Seq. ID NO_1174

<400> 1174
gacggcacga acctctaaag aggttcatcg ccgtc                                35

<210> 1175
<211> 35
<212> DNA
<213> Artificial Sequence

<220>
<223> Seq. ID NO_1175

<400> 1175
ggcccaacga acctctaaag aggttcatct gggcc                                35

<210> 1176
<211> 24
<212> DNA
<213> Artificial Sequence

<220>
<223> Seq. ID NO_1176

<400> 1176
acctctaaag aggttcatcc acag                                            24

<210> 1177
<211> 36
<212> DNA
<213> Artificial Sequence

<220>
<223> Seq. ID NO_1177

<400> 1177
cgcggaacct ctaaagaggt tcatccacag tccgcg                                36

<210> 1178
<211> 36
<212> DNA
<213> Artificial Sequence

<220>
<223> Seq. ID NO_1178

<400> 1178
ctcgcgacct ctaaagaggt tcatccacag cgcgag                                36

<210> 1179
<211> 36
<212> DNA
<213> Artificial Sequence

<220>
<223> Seq. ID NO_1179

<400> 1179
ccgaccacct ctaaagaggt tcatccacag ggtcgg                                36

<210> 1180
<211> 36
<212> DNA
<213> Artificial Sequence

<220>
<223> Seq. ID NO_1180

<400> 1180
cgacgtacct ctaaagaggt tcatccacag acgtcg                                36

<210> 1181
<211> 36
<212> DNA
<213> Artificial Sequence

<220>
<223> Seq. ID NO_1181

<400> 1181
cccaccacct ctaaagaggt tcatccacag ggtggg                                36

<210> 1182
<211> 36
<212> DNA
<213> Artificial Sequence

<220>
<223> Seq. ID NO_1182

<400> 1182
cgccgaacct ctaaagaggt tcatccacag tcggcg                                36


<210> 1183
<211> 36
<212> DNA
<213> Artificial Sequence


<220>
<223> Seq. ID NO_1183

<400> 1183
ctggcgacct ctaaagaggt tcatccacag cgccag                                36


<210> 1184
<211> 36
<212> DNA
<213> Artificial Sequence


<220>
<223> Seq. ID NO_1184

<400> 1184
ctgccgacct ctaaagaggt tcatccacag cggcag                                36


<210> 1185
<211> 36
<212> DNA
<213> Artificial Sequence


<220>
<223> Seq. ID NO_1185

<400> 1185
ccgggtacct ctaaagaggt tcatccacag acccgg                                36


<210> 1186
<211> 36
<212> DNA
<213> Artificial Sequence


<220>
<223> Seq. ID NO_1186

<400> 1186
ccccccacct ctaaagaggt tcatccacag gggggg                                36


<210> 1187
<211> 36
<212> DNA
<213> Artificial Sequence

<220>
<223> Seq. ID NO_1187

<400> 1187
gcgcctacct ctaaagaggt tcatccacag aggcgc                                36


<210> 1188
<211> 35
<212> DNA
<213> Artificial Sequence


<220>
<223> Seq. ID NO_1188

<400> 1188
cctacctcta aagaggttca tccacaggag gtagg                                 35


<210> 1189
<211> 36
<212> DNA
<213> Artificial Sequence


<220>
<223> Seq. ID NO_1189

<400> 1189
gagcgcacct ctaaagaggt tcatccacag gcgctc                                36


<210> 1190
<211> 36
<212> DNA
<213> Artificial Sequence


<220>
<223> Seq. ID NO_1190

<400> 1190
ggctggacct ctaaagaggt tcatccacag ccagcc                                36


<210> 1191
<211> 36
<212> DNA
<213> Artificial Sequence


<220>
<223> Seq. ID NO_1191

<400> 1191
gctgcaacct ctaaagaggt tcatccacag tgcagc                                36


<210> 1192
<211> 36

<212> DNA
<213> Artificial Sequence

<220>
<223> Seq. ID NO_1192

<400> 1192
gggtggacct ctaaagaggt tcatccacag ccaccc                                36

<210> 1193
<211> 36
<212> DNA
<213> Artificial Sequence

<220>
<223> Seq. ID NO_1193

<400> 1193
gcggctacct ctaaagaggt tcatccacag agccgc                                36

<210> 1194
<211> 36
<212> DNA
<213> Artificial Sequence

<220>
<223> Seq. ID NO_1194

<400> 1194
gaccgcacct ctaaagaggt tcatccacag gcggtc                                36

<210> 1195
<211> 36
<212> DNA
<213> A

<220>
<223> Seq. ID NO_1195

<400> 1195
gacggcacct ctaaagaggt tcatccacag gccgtc                                36

<210> 1196
<211> 36
<212> DNA
<213> Artificial Sequence

<220>
<223> Seq. ID NO_1196

<400> 1196
ggcccaacct ctaaagaggt tcatccacag tgggcc                                36

<210> 1197
<211> 22
<212> DNA
<213> Artificial Sequence

<220>
<223> Seq. ID NO_1197

<400> 1197
ctgttactta tgcgccctat tc                                                22

<210> 1198
<211> 34
<212> DNA
<213> Artificial Sequence

<220>
<223> Seq. ID NO_1198

<400> 1198
cgcggactgt tacttatgcg ccctattctc cgcg                                   34

<210> 1199
<211> 34
<212> DNA
<213> Artificial Sequence

<220>
<223> Seq. ID NO_1199

<400> 1199
ctcgcgctgt tacttatgcg ccctattccg cgag                                   34

<210> 1200
<211> 34
<212> DNA
<213> Artificial Sequence

<220>
<223> Seq. ID NO_1200

<400> 1200
ccgaccctgt tacttatgcg ccctattcgg tcgg                                   34

<210> 1201
<211> 34
<212> DNA
<213> Artificial Sequence

<220>
<223> Seq. ID NO_1201

<400> 1201
cgacgtctgt tacttatgcg ccctattcac gtcg                                   34

<210> 1202
<211> 34
<212> DNA
<213> Artificial Sequence

<220>
<223> Seq. ID NO_1202

<400> 1202
cccaccctgt tacttatgcg ccctattcgg tggg 34

<210> 1203
<211> 34
<212> DNA
<213> Artificial Sequence

<220>
<223> Seq. ID NO_1203

<400> 1203
cgccgactgt tacttatgcg ccctattctc ggcg 34

<210> 1204
<211> 34
<212> DNA
<213> Artificial Sequence

<220>
<223> Seq. ID NO_1204

<400> 1204
ctggcgctgt tacttatgcg ccctattccg ccag 34

<210> 1205
<211> 34
<212> DNA
<213> Artificial Sequence

<220>
<223> Seq. ID NO_1205

<400> 1205
ctgccgctgt tacttatgcg ccctattccg gcag 34

<210> 1206
<211> 34
<212> DNA
<213> Artificial Sequence

<220>
<223> Seq. ID NO_1206

<400> 1206
ccgggtctgt tacttatgcg ccctattcac ccgg                                    34

<210> 1207
<211> 34
<212> DNA
<213> Artificial Sequence

<220>
<223> Seq. ID NO_1207

<400> 1207
ccccccctgt tacttatgcg ccctattcgg gggg                                    34

<210> 1208
<211> 34
<212> DNA
<213> Artificial Sequence

<220>
<223> Seq. ID NO_1208

<400> 1208
gcgcctctgt tacttatgcg ccctattcag gcgc                                    34

<210> 1209
<211> 34
<212> DNA
<213> Artificial Sequence

<220>
<223> Seq. ID NO_1209

<400> 1209
gagcgcctgt tacttatgcg ccctattcgc gctc                                    34

<210> 1210
<211> 34
<212> DNA
<213> Artificial Sequence

<220>
<223> Seq. ID NO_1210

<400> 1210
ggctggctgt tacttatgcg ccctattccc agcc                                    34

<210> 1211
<211> 34
<212> DNA
<213> Artificial Sequence

<220>

<223> Seq. ID NO_1211

<400> 1211
gctgcactgt tacttatgcg ccctattctg cagc                                    34


<210> 1212
<211> 34
<212> DNA
<213> Artificial Sequence


<220>
<223> Seq. ID NO_1212

<400> 1212
gggtggctgt tacttatgcg ccctattccc accc                                    34


<210> 1213
<211> 34
<212> DNA
<213> Artificial Sequence


<220>
<223> Seq. ID NO_1213

<400> 1213
gcggctctgt tacttatgcg ccctattcag ccgc                                    34


<210> 1214
<211> 34
<212> DNA
<213> Artificial Sequence


<220>
<223> Seq. ID NO_1214

<400> 1214
gaccgcctgt tacttatgcg ccctattcgc ggtc                                    34


<210> 1215
<211> 34
<212> DNA
<213> Artificial Sequence


<220>
<223> Seq. ID NO_1215

<400> 1215
gacggcctgt tacttatgcg ccctattcgc cgtc                                    34


<210> 1216
<211> 34
<212> DNA
<213> Artificial Sequence

<220>
<223> Seq. ID NO_1216

<400> 1216
ggcccactgt tacttatgcg ccctattctg ggcc 34

## Beschreibung

Die Erfindung betrifft ein Verfahren zum Nachweis von Mikroorganismen, z.B. der Bakterienfamilie der Enterobacteriaceaen, in einer Probe mittels Einschleusen identitätsbestimmender Nukleinsäuresonden in die einzelnen Zellkörper, wobei die Nukleinsäuresonden mit den Nukleinsäuren der Mikroorganismen eine Hybridisierung eingehen, und anschließendem optischen Detektieren der in den einzelnen Zellkörpern erzeugten Hybridisierungen, wobei eine Mischung aus wenigstens einer ersten Nukleinsäuresonde und einer zweiten Nukleinsäuresonde verwendet wird, und dass die erste Nukleinsäuresonde und die zweite Nukleinsäuresonde jeweils in einem von zwei miteinander nicht überlappenden Bereichen der Zielnukleinsäure anbinden.

Enterobacteriaceae sind eine Gruppe der gramnegativen, fakultativ anaeroben Stäbchen-Bakterien, die in der Regel auf einfachen Nährböden wachsen können. Derzeit umfasst diese sehr heterogene, phylogenetische Familie etwa 53 Gattungen und über 170 Arten. Viele Enterobacteriaceae sind humanpathogen und sind daher Indikatorkeime in der Hygienekontrolle.

Traditionell werden Enterobacteriaceae über spezifische Kultivierung auf Selektionsnährböden sowie biochemische Sekundärnachweise identifiziert. Die Zugehörigkeit zu einer bestimmten Familie kann auch anhand identitätsbestimmender Nukleinsäure-Sequenzen des zu untersuchenden Mikroorganismus nachgewiesen werden. Solche Nachweise können mittels PCR oder Microarray durchgeführt werden. Zum spezifischen Nachweis von Nukleinsäuren, d.h. DNA und/oder RNA Molekülen in einzelnen Zellen sind beispielsweise auch Verfahren wie In-Situ-Hybridisierung (ISH) bekannt. Dabei werden kurze synthetische Nukleinsäuresonden eingesetzt, welche über Basenpaarungen an die nachzuweisende Zielsequenz binden. Eine Variante der ISH-Technologie, bei welcher die Nukleinsäuresonden fluoreszent markiert sind, ist die Fluoreszenz-In-Situ-Hybridisierung (FISH).

Die bekannten Nukleinsäuresonden, die bei dem FISH-Verfahren zum Nachweis der Bakterienfamilie der Enterobacteriaceaen verwendet werden, weisen allerdings oft ungenügende Spezifität zwischen Enterobacteriaceaen und anderen Bakterien-Familien auf. Häufig treten deshalb Falsch-Positive-Ergebnisse auf, da die Nukleinsäuresonden nicht nur mit den Nukleinsäuren der Enterobacteriaceaen hybridisieren.

Innerhalb der Bakterienfamilie der Enterobacteriaceaen sowie deren Gattungen, Arten und Serotypen wie insbesondere Escherichia coli und insbesondere EHEC und STEC und VTEC und insbesondere E. coli 0157:H7, Salmonella und insbesondere Salmonella enterica, Yersinia und insbesondere Yersinia enterocolitica und Yersinia pestis, Enterobacter, antibiotikaresistente Enterobakterien und weitere Bakterienfamilien sowie deren Gattungen, Arten und Serotypen wie insbesondere Listeria und insbesondere Listeria monocytogenes, Bacillus und insbesondere Bacillus cereus und Bacillus anthracis und Bacillus subtilis, Pseudomonas und insbesondere Pseudomonas aeroginosa, Staphylococcus und insbesondere Staphylococcus aureus und MRSBakterienfamilie der Enterobacteriaceaen sowie deren Gattungen, Arten und Serotypen wie insbesondere Escherichia coli und insbesondere EHEC und STEC und VTEC und insbesondere E. coli 0157:H7, Salmonella und insbesondere Salmonella enterica, Yersinia und insbesondere Yersinia enterocolitica und Yersinia pestis, Enterobacter, antibiotikaresistente Enterobakterien und weitere Bakterienfamilien sowie deren Gattungen, Arten und Serotypen wie insbesondere Listeria und insbesondere Listeria monocytogenes, Bacillus und insbesondere Bacillus cereus und Bacillus anthracis und Bacillus subtilis, Pseudomonas und insbesondere Pseudomonas aeroginosa, Staphylococcus und insbesondere Staphylococcus aureus und MRSA, Campylobacteriaceae und insbesondere Campylobacter, Acinetobacter und insbesondere Acinetobacter baumannii und Acinetobacter johnsonii und insbesondere antibiotikaresistente Acinetobacter, grampositive und gramnegative antibiotikaresistente BakterienA, Campylobacteriaceae und insbesondere Campylobacter, Acinetobacter und insbesondere Acinetobacter baumannii und Acinetobacter johnsonii und insbesondere antibiotikaresistente Acinetobacter, grampositive und gramnegative antibiotikaresistente Bakterienweisen die herkömmlichen Nukleinsäuresonden außerdem eine ungenügende Sensitivität auf, so dass auch Falsch-Negative-Ergebnisse auftreten können. Mit den einzelnen herkömmlichen Nukleinsäuresonden kann oft eine Fluoreszenzintensität erzielt werden, die für eine Detektion der nachzuweisenden Bakterienfamilie unzureichend ist. Die Verwendung mehrerer Nukleinsäuresonden gleichzeitig wird in der Regel dadurch erschwert, dass die herkömmlichen Nukleinsäuresonden für Enterobacteriaceaen nicht miteinander kombinierbar sind, weil sie überlappende Zielsequenzen aufweisen.

Vor diesem Hintergrund ist es Aufgabe der vorliegenden Erfindung, ein verbessertes Verfahren bereitzustellen, das einen spezifischen Nachweis von Mikroorganismen, z.B. der Bakterienfamilie der Enterobacteriaceaen, in einer Probe anhand identitätsspezifischer Nukleinsäuresonden ermöglicht.

Erfindungsgemäß wird diese Aufgabe durch die Merkmale von Anspruch 1 gelöst. Insbesondere wird somit erfindungsgemäß zur Lösung der genannten Aufgabe bei einem Verfahren der eingangs beschriebenen Art vorgeschlagen, dass eine Mischung aus wenigstens einer ersten Nukleinsäuresonde und einer zweiten Nukleinsäuresonde verwendet wird, und dass die erste Nukleinsäuresonde und die zweite Nukleinsäuresonde jeweils in einem von zwei miteinander nicht überlappenden Bereichen der Zielnukleinsäure anbinden. Somit ist einfach erreichbar, dass die erste Nukleinsäuresonde und die zweite Nukleinsäuresonde (und gegebenenfalls weitere Nukleinsäuresonden) gleichzeitig an derselben Zielnukleinsäure anbinden können. Dadurch kann sich die Detektierbarkeit von Enterobacteriaceaen erheblich verbessern, da mit der Kombination mehrerer Nukleinsäuresonden eine höhere Fluoreszenzintensität pro Bakterium erreichbar ist, indem mehrere Zielsequenzen gleichzeitig nachgewiesen werden können.

Eine vorteilhafte Ausführungsform gemäß der Erfindung sieht vor, dass die Mischung so gebildet ist, dass aus der vorliegenden Kombinatorik der Gruppen 1 bis 12 jeweils aus jeder Gruppe ein Vertreter oder aus wenigstens zwei Gruppen ein Vertreter ausgewählt werden, insbesondere wobei die Gruppe 1 einzelne oder alle der SEQ ID NO: 1 bis SEQ ID NO: 19 umfasst, die Gruppe 2 einzelne oder alle der SEQ ID NO: 20 bis SEQ ID NO: 40 umfasst, die Gruppe 3 einzelne oder alle der SEQ ID NO: 41 bis SEQ ID NO: 59 umfasst, die Gruppe 4 einzelne oder alle der SEQ ID NO: 60 bis SEQ ID NO: 78 umfasst, die Gruppe 5 einzelne oder alle der SEQ ID NO: 79 bis SEQ ID NO: 99 umfasst, die Gruppe 6 einzelne oder alle der SEQ ID NO: 100 bis SEQ ID NO: 118 umfasst, die Gruppe 7 einzelne oder alle der SEQ ID NO: 119 bis SEQ ID NO: 137 umfasst, die Gruppe 8 einzelne oder alle der SEQ ID NO: 138 bis SEQ ID NO: 156 umfasst, die Gruppe 9 einzelne oder alle der SEQ ID NO: 157 bis SEQ ID NO: 175 umfasst, die Gruppe 10 einzelne oder alle der SEQ ID NO: 176 bis SEQ ID NO: 196 umfasst, die Gruppe 11 einzelne oder alle der SEQ ID NO: 197 bis SEQ ID NO: 217 umfasst und die Gruppe 12 einzelne oder alle der SEQ ID NO: 218 bis SEQ ID NO: 236 umfasst.

Eine vorteilhafte Ausführungsform gemäß der Erfindung sieht vor, dass die Mischung so gebildet ist, dass aus der vorliegenden Kombinatorik der Gruppen 13 bis 33 jeweils aus jeder Gruppe ein Vertreter oder aus wenigstens zwei Gruppen ein Vertreter ausgewählt werden, insbesondere wobei die Gruppe 13 einzelne oder alle der SEQ ID NO: 237 bis SEQ ID NO: 294 umfasst, die Gruppe 14 einzelne oder alle der SEQ ID NO: 295 bis SEQ ID NO: 314 umfasst, die Gruppe 15 einzelne oder alle der SEQ ID NO: 315 bis SEQ ID NO: 372 umfasst, die Gruppe 16 einzelne oder alle der SEQ ID NO: 373 bis SEQ ID NO: 411 umfasst, die Gruppe 17 einzelne oder alle der SEQ ID NO: 412 bis SEQ ID NO: 450 umfasst, die Gruppe 18 einzelne oder alle der SEQ ID NO: 451 bis SEQ ID NO: 508 umfasst, die Gruppe 19 einzelne oder alle der SEQ ID NO: 509 bis SEQ ID NO: 528 umfasst, die Gruppe 20 einzelne oder alle der SEQ ID NO: 529 bis SEQ ID NO: 547 umfasst, die Gruppe 21 einzelne oder alle der SEQ ID NO: 548 bis SEQ ID NO: 567 umfasst, die Gruppe 22 einzelne oder alle der SEQ ID NO: 568 bis SEQ ID NO: 587 umfasst, die Gruppe 23 einzelne oder alle der SEQ ID NO: 588 bis SEQ ID NO: 607 umfasst, die Gruppe 24 einzelne oder alle der SEQ ID NO: 608 bis SEQ ID NO: 626 umfasst, die Gruppe 25 einzelne oder alle der SEQ ID NO: 627 bis SEQ ID NO: 645 umfasst, die Gruppe 26 einzelne oder alle der SEQ ID NO: 646 bis SEQ ID NO: 664 umfasst, die Gruppe 27 einzelne oder alle der SEQ ID NO: 665 bis SEQ ID NO: 684 umfasst, die Gruppe 28 einzelne oder alle der SEQ ID NO: 685 bis SEQ ID NO: 704 umfasst, die Gruppe 29 einzelne oder alle der SEQ ID NO: 705 bis SEQ ID NO: 724 umfasst, die Gruppe 30 einzelne oder alle der SEQ ID NO: 725 bis SEQ ID NO: 744 umfasst, die Gruppe 31 einzelne oder alle der SEQ ID NO: 745 bis SEQ ID NO: 764 umfasst, die Gruppe 32 einzelne oder alle der SEQ ID NO: 765 bis SEQ ID NO: 804 umfasst und die Gruppe 33 einzelne oder alle der SEQ ID NO: 805 bis SEQ ID NO: 824 umfasst.

Eine vorteilhafte Ausführungsform gemäß der Erfindung sieht vor, dass die Mischung so gebildet ist, dass aus der vorliegenden Kombinatorik der Gruppen 34 bis 46 jeweils aus jeder Gruppe ein Vertreter oder aus wenigstens zwei Gruppen ein Vertreter ausgewählt werden, insbesondere wobei die Gruppe 34 einzelne oder alle der SEQ ID NO: 825 bis SEQ ID NO: 844 umfasst, die Gruppe 35 einzelne oder alle der SEQ ID NO: 845 bis SEQ ID NO: 865 umfasst, die Gruppe 36 einzelne oder alle der SEQ ID NO: 866 bis SEQ ID NO: 886 umfasst, die Gruppe 37 einzelne oder alle der SEQ ID NO: 887 bis SEQ ID NO: 927 umfasst, die Gruppe 38 einzelne oder alle der SEQ ID NO: 928 bis SEQ ID NO: 947 umfasst, die Gruppe 39 einzelne oder alle der SEQ ID NO: 948 bis SEQ ID NO: 967 umfasst, die Gruppe 40 einzelne oder alle der SEQ ID NO: 968 bis SEQ ID NO: 988 umfasst, die Gruppe 41 einzelne oder alle der SEQ ID NO: 989 bis SEQ ID NO: 1009 umfasst, die Gruppe 42 einzelne oder alle der SEQ ID NO: 1010 bis SEQ ID NO: 1071 umfasst, die Gruppe 43 einzelne oder alle der SEQ ID NO: 1072 bis SEQ ID NO: 1133 umfasst, die Gruppe 44 einzelne oder alle der SEQ ID NO: 1134 bis SEQ ID NO: 1154 umfasst, die Gruppe 45 einzelne oder alle der SEQ ID NO: 1155 bis SEQ ID NO: 1196 umfasst und die Gruppe 46 einzelne oder alle der SEQ ID NO: 1197 bis SEQ ID NO: 1216 umfasst.

Hierzu macht sich die Erfindung zunutze, dass sich die Sensitivität (Richtig-Positiv-Rate) und Spezifität (Richtig-Negativ-Rate) im Hinblick auf Enterobacteriaceaen erheblich verbessern lassen können, indem die Kombination mehrerer Nukleinsäuresonden aus der erfindungsgemäßen Kombinatorik der Gruppen 1 bis 12 bzw. im Hinblick auf Listeriaceae die Kombination mehrerer Nukleinsäuresonden aus der erfindungsgemäßen Kombinatorik der Gruppen 13 bis 33, bzw. im Hinblick auf Listeria monocytogenes die Kombination mehrerer Nukleinsäuresonden aus der erfindungsgemäßen Kombinatorik der Gruppen 34 bis 46 verwendet wird. Insbesondere kann durch die Verwendung von mehr als nur einer Nukleinsäuresonde verhindert werden, dass es in den hoch variablen Nukleinsäuresonden-Zielregionen zu Fehlpaarungen kommen kann, wodurch Falsch-Negative Ergebnisse entstehen können.

Eine vorteilhafte Ausführungsform gemäß der Erfindung sieht weiterhin vor, dass die Nachweisreaktion unter Verwendung von Nukleinsäuresonden mittels Fluoreszenz-In-Situ-Hybridisierung (FISH), Nukleinsäuren-Amplifikationsreaktion und/oder Mikroarray erfolgt. Eine Amplifikationsreaktion kann beispielsweise Polymerasekettenreaktion ("PCR") sein. Von Vorteil ist dabei, dass ein spezifischer Nachweis von Enterobacteriaceaen mittels unterschiedlicher Nachweistechniken ermöglicht werden kann.

Eine vorteilhafte Ausführungsform gemäß der Erfindung sieht vor, dass die Nukleinsäuresonden jeweils als lineare Sonden ausgebildet sind. Alternativ oder zusätzlich können die Nukleinsäuresonden Sekundärstruktur aufweisen, beispielsweise als Molecular Beacons und/oder als Scorpion Probes ausgebildet sein. Dadurch sind eine höhere Fluoreszenzintensität sowie ein besseres Signal-Rausch-Verhältnis erreichbar, was insbesondere für eine automatisierte Anwendung vorteilhaftsein kann.

Eine vorteilhafte Ausführungsform gemäß der Erfindung sieht weiterhin vor, dass eine optische Sensitivität so eingestellt ist, dass nur solche Mikroorganismen detektiert werden, die wenigstens zwei Anbindungen aufweisen. Somit kann sichergestellt werden, dass in der nachzuweisenden Bakterienfamilie immer wenigstens zwei Nukleinsäuresonden anbinden. Vorteilhaft ist dabei, dass eine höhere diagnostische Sensitivität erreichbar ist.

Eine vorteilhafte Ausführungsform gemäß der Erfindung sieht vor, dass die erste und/oder zweite Nukleinsäuresonde wenigstens einen ersten Farbstoff konjugiert mit dem 5'-Ende und/oder wenigstens einen zweiten Farbstoff konjugiert mit dem 3'-Ende aufweist. Vorteilhaft ist dabei, dass durch den Einsatz verschiedener Farbstoffe den Nachweis einer bestimmten Farbkombination ermöglicht werden kann.

Eine vorteilhafte Ausführungsform gemäß der Erfindung sieht weiterhin vor, dass die Nukleinsäuresonde am 5'-Ende und/oder 3'-Ende weitere Nukleotide als Stammsequenz und/oder wenigstens einen Funktionsteil aufweist. Es kann insbesondere vorgesehen sein, dass die Stammsequenzen und/oder Funktionsteile so zueinander ausgestaltet sind, dass sie nicht gegenseitig miteinander wechselwirken. Die Erfindung macht sich hierzu zunutze, dass die Stamm-bildenden Nukleotide in Abwesenheit von Zielsequenzen eine "Haarnadel"-Struktur bilden können, wodurch die Fluoreszenz des Farbstoffs unterdrückt wird. Nach der Bindung des Funktionsteils an die Zielsequenz löst sich diese "Haarnadel"-Struktur wieder auf, wobei die Fluoreszenz des Farbstoffs, die nicht mehr unterdrückt wird, detektiert werden kann.

Eine vorteilhafte Ausführungsform gemäß der Erfindung sieht vor, dass die Auswahl der nicht überlappenden Bereiche so gewählt ist, dass ein ausreichend großer räumlicher Abstand vorliegt und sich die Nukleinsäuresonden in ihrem Abstrahlverhalten nicht gegenseitig stören. Somit kann verhindert werden, dass bei der Signalerfassung Fehlmessungen auftreten.

Eine vorteilhafte Ausführungsform gemäß der Erfindung sieht weiterhin vor, dass die Nukleinsäuresonden so ausgestaltet sind, dass eine bestimmte Farbkombination detektierbar ist. Beispielsweise kann hierfür die oder eine erste Nukleinsäuresonde einen ersten Farbstoff und die oder eine zweite Nukleinsäuresonde einen zweiten Farbstoff aufweisen. Vorteilhaft ist dabei, dass durch den Nachweis einer bestimmten Farbkombination eine Alternative zur Messempfindlichkeit für eine spezifische Detektierbarkeit von z.B Enterobacteriaceaen ermöglicht werden kann.

Eine vorteilhafte Ausführungsform gemäß der Erfindung sieht vor, dass die Nukleinsäuresonde wenigstens einen optisch detektierbaren Marker aufweist. Der detektierbare Marker kann beispielsweise eine enzymatisch aktive Gruppe, ein Affinitätsmarker oder ein Farbstoff sein. Der Affinitätsmarker kann beispielsweise Affinitätsbindungspaare Biotin-Streptavidin oder Antigen-Antikörper umfassen. Der enzymatisch aktive Marker kann beispielsweise Peroxidase, Luciferase oder Phosphatase sein. Der Farbstoff kann beispielsweise ein Fluoreszenzmarker sein. Somit ist eine optische Detektion erreichbar.

Eine vorteilhafte Ausführungsform gemäß der Erfindung sieht weiterhin vor, dass jede Nukleinsäuresonde an mindestens 80% der z.B. Bakterienfamilie der Enterobacteriaceaen anbindet. Alternativ oder zusätzlich kann vorgesehen sein, dass jede Nukleinsäuresonde nachweisbar nur an die Nukleinsäuren z.B. der Bakterienfamilie der Enterobacteriaceaen und nicht an die Nukleinsäuren eines Organismus, der zu einer anderen Bakterienfamilie gehört, anbindet. Somit ist eine hoch spezifische Nachweisbarkeit für das Beispiel der Bakterienfamilie der Enterobacteriaceaen erreichbar.

Eine bevorzugte Anwendung sieht eine Verwendung wenigstens einer Nukleinsäuresonde oder wenigstens zwei Nukleinsäuresonden vor, ausgewählt aus der Gruppe bestehend aus SEQ ID NO: 1, SEQ ID NO: 20, SEQ ID NO: 41, SEQ ID NO: 60, SEQ ID NO: 79, SEQ ID NO: 100, SEQ ID NO: 119, SEQ ID NO: 138, SEQ ID NO: 157, SEQ ID NO: 176, SEQ ID NO: 197, SEQ ID NO: 218 und/oder weiteren Sequenzen aus der vorliegenden Kombinatorik der Gruppen 1 bis 12 zum Nachweis der Enterobacteriaceaen und/oder zur Immobilisierung auf einem Trägermaterial, insbesondere einem fluidischen Kanalsystem.

Eine bevorzugte Anwendung sieht eine Verwendung wenigstens einer Nukleinsäuresonde oder wenigstens zwei Nukleinsäuresonden vor, ausgewählt aus der Gruppe bestehend aus SEQ ID NO: 237, SEQ ID NO: 295, SEQ ID NO: 315, SEQ ID NO: 373, SEQ ID NO: 412, SEQ ID NO: 451, SEQ ID NO: 509, SEQ ID NO: 529, SEQ ID NO: 548, SEQ ID NO: 568, SEQ ID NO: 588, SEQ ID NO: 608, SEQ ID NO: 627, SEQ ID NO: 646, SEQ ID NO: 665, SEQ ID NO: 685, SEQ ID NO: 705, SEQ ID NO: 725, SEQ ID NO: 745, SEQ ID NO: 765, SEQ ID NO: 805 und/oder weiteren Sequenzen aus der vorliegenden Kombinatorik der Gruppen 13 bis 33 zum Nachweis der Listeriaceae. und/oder zur Immobilisierung auf einem Trägermaterial, insbesondere einem fluidischen Kanalsystem

Eine bevorzugte Anwendung sieht eine Verwendung wenigstens einer Nukleinsäuresonde oder wenigstens zwei Nukleinsäuresonden vor, ausgewählt aus der Gruppe bestehend aus SEQ ID NO: 825, SEQ ID NO: 845, SEQ ID NO: 866, SEQ ID NO: 887, SEQ ID NO: 928, SEQ ID NO: 948, SEQ ID NO: 968, SEQ ID NO: 989, SEQ ID NO: 1010, SEQ ID NO: 1072, SEQ ID NO: 1134, SEQ ID NO: 1155, SEQ ID NO: 1197 und/oder weiteren Sequenzen aus der vorliegenden Kombinatorik der Gruppen 34 bis 46 zum Nachweis der Listeriaceae, insbesondere Listeria monocytogenes. und/oder zur Immobilisierung auf einem Trägermaterial, insbesondere einem fluidischen Kanalsystem.

Eine bevorzugte Anwendung sieht ein fluidisches Kanalsystem vor, mit Mitteln zum Durchführen des Verfahrens, insbesondere wie zuvor beschrieben und/oder nach einem der auf ein Verfahren gerichteten Ansprüche. Beispielsweise können in dem fluidischen Kanalsystem zum Durchführen des erfindungsgemäßen Verfahrens ein Detektionsbereich und ein Vorbereitungsbereich ausgebildet sein. Insbesondere kann vorgesehen sein, dass die Querschnitte der Kanäle des fluidischen Kanalsystems auf Abmessungen der Mikroorganismen angepasst sein können.

Das fluidische Kanalsystem kann beispielsweise in Form eines Probenträgers ausgebildet sein. Der erfindungsgemäße Probenträger umfasst insbesondere mindestens eine Kavität mit wenigstens einer Nukleinsäuresonde oder wenigstens zwei Nukleinsäuresonden ausgewählt aus der Gruppe bestehend aus SEQ ID NO: 1, SEQ ID NO: 20, SEQ ID NO: 41, SEQ ID NO: 60, SEQ ID NO: 79, SEQ ID NO: 100, SEQ ID NO: 119, SEQ ID NO: 138, SEQ ID NO: 157, SEQ ID NO: 176, SEQ ID NO: 197, SEQ ID NO: 218 und/oder weiteren Sequenzen aus der vorliegenden Kombinatorik der Gruppen 1 bis 12. Alternativ oder zusätzlich kann der Probenträger Mittel zum optischen Detektieren von markierten Mikroorganismen umfassen.

Das fluidische Kanalsystem kann beispielsweise in Form eines Probenträgers ausgebildet sein. Der erfindungsgemäße Probenträger umfasst insbesondere mindestens eine Kavität mit wenigstens einer Nukleinsäuresonde oder wenigstens zwei Nukleinsäuresonden ausgewählt aus der Gruppe bestehend aus SEQ ID NO: 237, SEQ ID NO: 295, SEQ ID NO: 315, SEQ ID NO: 373, SEQ ID NO: 412, SEQ ID NO: 451, SEQ ID NO: 509, SEQ ID NO: 529, SEQ ID NO: 548, SEQ ID NO: 568, SEQ ID NO: 588, SEQ ID NO: 608, SEQ ID NO: 627, SEQ ID NO: 646, SEQ ID NO: 665, SEQ ID NO: 685, SEQ ID NO: 705, SEQ ID NO: 725, SEQ ID NO: 745, SEQ ID NO: 765, SEQ ID NO: 805 und/oder weiteren Sequenzen aus der vorliegenden Kombinatorik der Gruppen 13 bis 33. Alternativ oder zusätzlich kann der Probenträger Mittel zum optischen Detektieren von markierten Mikroorganismen umfassen. Das fluidische Kanalsystem kann beispielsweise in Form eines Probenträgers ausgebildet sein. Der erfindungsgemäße Probenträger umfasst insbesondere mindestens eine Kavität mit wenigstens einer Nukleinsäuresonde oder wenigstens zwei Nukleinsäuresonden ausgewählt aus der Gruppe bestehend aus SEQ ID NO: 825, SEQ ID NO: 845, SEQ ID NO: 866, SEQ ID NO: 887, SEQ ID NO: 928, SEQ ID NO: 948, SEQ ID NO: 968, SEQ ID NO: 989, SEQ ID NO: 1010, SEQ ID NO: 1072, SEQ ID NO: 1134, SEQ ID NO: 1155, SEQ ID NO: 1197 und/oder weiteren Sequenzen aus der vorliegenden Kombinatorik der Gruppen 34 bis 46. Alternativ oder zusätzlich kann der Probenträger Mittel zum optischen Detektieren von markierten Mikroorganismen umfassen.

Der erfindungsgemäße Probenträger kann als ein scheibenförmiger Probenträger ausgebildet sein. Beispielsweise kann der Probenträger als ein flacher Probenträger ausgebildet sein. Von Vorteil ist dabei, dass durch die Scheibenform des Probenträgers die Zentrifugalkraft für die Fluidförderung ausgenutzt werden kann. Die Fluidförderung ist auch über Druck oder auf eine andere Weise erreichbar. Der Probenträger kann alternativ eine dreidimensionale Erstreckung, beispielsweise in Form eines Zylinders oder küvettenartig, aufweisen.

Beispielsweise kann die Scheibenförmigkeit rotationssymmetrisch ausgebildet sein. Dies kann für ein Zentrifugieren von Vorteil sein. Es sind auch alternativ rechteckförmige Probenträger wie bei einer Chipkarte oder segmentförmige Probenträger wie bei einem Pizzastück bildbar. Die Erfindung wird nun anhand von Ausführungsbeispielen näher beschrieben, ist aber nicht auf diese Ausführungsbeispiele beschränkt. Weitere Ausführungsbeispiele ergeben sich durch Kombination der Merkmale einzelner oder mehrerer Schutzansprüche untereinander und/oder mit einzelnen oder mehreren Merkmalen der Ausführungsbeispiele.

Es zeigt:
- Fig. 1: Gruppe 1 mit den Sequenzen SEQ ID NO: 1 bis SEQ ID NO: 19 aus einer Kombinatorik der erfindungsgemäßen Nukleinsäuresonden,
- Fig. 2: Gruppe 2 mit den Sequenzen SEQ ID NO: 20 bis SEQ ID NO: 40 aus einer Kombinatorik der erfindungsgemäßen Nukleinsäuresonden,
- Fig. 3: Gruppe 3 mit den Sequenzen SEQ ID NO: 41 bis SEQ ID NO: 59 aus einer Kombinatorik der erfindungsgemäßen Nukleinsäuresonden,
- Fig. 4: Gruppe 4 mit den Sequenzen SEQ ID NO: 60 bis SEQ ID NO: 78 aus einer Kombinatorik der erfindungsgemäßen Nukleinsäuresonden,
- Fig. 5: Gruppe 5 mit den Sequenzen SEQ ID NO: 79 bis SEQ ID NO: 99 aus einer Kombinatorik der erfindungsgemäßen Nukleinsäuresonden,
- Fig. 6: Gruppe 6 mit den Sequenzen SEQ ID NO: 100 bis SEQ ID NO: 118 aus einer Kombinatorik der erfindungsgemäßen Nukleinsäuresonden,
- Fig. 7: Gruppe 7 mit den Sequenzen SEQ ID NO: 119 bis SEQ ID NO: 137 aus einer Kombinatorik der erfindungsgemäßen Nukleinsäuresonden,
- Fig. 8: Gruppe 8 mit den Sequenzen SEQ ID NO: 138 bis SEQ ID NO: 156 aus einer Kombinatorik der erfindungsgemäßen Nukleinsäuresonden,
- Fig. 9: Gruppe 9 mit den Sequenzen SEQ ID NO: 157 bis SEQ ID NO: 175 aus einer Kombinatorik der erfindungsgemäßen Nukleinsäuresonden,
- Fig. 10: Gruppe 10 mit den Sequenzen SEQ ID NO: 176 bis SEQ ID NO: 196 aus einer Kombinatorik der erfindungsgemäßen Nukleinsäuresonden,
- Fig. 11: Gruppe 11 mit den Sequenzen SEQ ID NO: 197 bis SEQ ID NO: 217 aus einer Kombinatorik der erfindungsgemäßen Nukleinsäuresonden,
- Fig. 12: Gruppe 12 mit den Sequenzen SEQ ID NO: 218 bis SEQ ID NO: 236 aus einer Kombinatorik der erfindungsgemäßen Nukleinsäuresonden,
- Fig. 13: ein fluidisches Kanalsystem zum Durchführen des erfindungsgemäßen Verfahrens in einer schematischen Darstellung.
- Fig. 14: Gruppe 13 mit den Sequenzen SEQ ID NO: 237 bis SEQ IDNO: 294 aus einer Kombinatorik der erfindungsgemäßen Nukleinsäuresonden,
- Fig. 15: Gruppe 14 mit den Sequenzen SEQ ID NO: 295 bis SEQ ID NO: 314 aus einer Kombinatorik der erfindungsgemäßen Nukleinsäuresonden,
- Fig. 16: Gruppe 15 mit den Sequenzen SEQ ID NO: 315 bis SEQ ID NO: 372 aus einer Kombinatorik der erfindungsgemäßen Nukleinsäuresonden,
- Fig. 17: Gruppe 16 mit den Sequenzen SEQ ID NO: 373 bis SEQ ID NO: 411 aus einer Kombinatorik der erfindungsgemäßen Nukleinsäuresonden,
- Fig. 18: Gruppe 17 mit den Sequenzen SEQ ID NO: 412 bis SEQ ID NO: 450 aus einer Kombinatorik der erfindungsgemäßen Nukleinsäuresonden,
- Fig. 19: Gruppe 18 mit den Sequenzen SEQ ID NO: 451 bis SEQ ID NO: 508 aus einer Kombinatorik der erfindungsgemäßen Nukleinsäuresonden,
- Fig. 20: Gruppe 19 mit den Sequenzen SEQ ID NO: 509 bis SEQ ID NO: 528 aus einer Kombinatorik der erfindungsgemäßen Nukleinsäuresonden,
- Fig. 21: Gruppe 20 mit den Sequenzen SEQ ID NO: 529 bis SEQ ID NO: 547 aus einer Kombinatorik der erfindungsgemäßen Nukleinsäuresonden,
- Fig. 22: Gruppe 21 mit den Sequenzen SEQ ID NO: 548 bis SEQ ID NO: 567 aus einer Kombinatorik der erfindungsgemäßen Nukleinsäuresonden,
- Fig. 23: Gruppe 22 mit den Sequenzen SEQ ID NO: 568 bis SEQ ID NO: 587 aus einer Kombinatorik der erfindungsgemäßen Nukleinsäuresonden,
- Fig. 24: Gruppe 23 mit den Sequenzen SEQ ID NO: 588 bis SEQ ID NO: 607 aus einer Kombinatorik der erfindungsgemäßen Nukleinsäuresonden,
- Fig. 25: Gruppe 24 mit den Sequenzen SEQ ID NO: 608 bis SEQ ID NO: 626 aus einer Kombinatorik der erfindungsgemäßen Nukleinsäuresonden,
- Fig. 26: Gruppe 25 mit den Sequenzen SEQ ID NO: 627 bis SEQ ID NO: 645 aus einer Kombinatorik der erfindungsgemäßen Nukleinsäuresonden,
- Fig. 27: Gruppe 26 mit den Sequenzen SEQ ID NO: 646 bis SEQ ID NO: 664 aus einer Kombinatorik der erfindungsgemäßen Nukleinsäuresonden,
- Fig. 28: Gruppe 27 mit den Sequenzen SEQ ID NO: 665 bis SEQ ID NO: 684 aus einer Kombinatorik der erfindungsgemäßen Nukleinsäuresonden,
- Fig. 29: Gruppe 28 mit den Sequenzen SEQ ID NO: 685 bis SEQ ID NO: 704 aus einer Kombinatorik der erfindungsgemäßen Nukleinsäuresonden,
- Fig. 30: Gruppe 29 mit den Sequenzen SEQ ID NO: 705 bis SEQ ID NO: 724 aus einer Kombinatorik der erfindungsgemäßen Nukleinsäuresonden,
- Fig. 31: Gruppe 30 mit den Sequenzen SEQ ID NO: 725 bis SEQ ID NO: 744 aus einer Kombinatorik der erfindungsgemäßen Nukleinsäuresonden,
- Fig. 32: Gruppe 31 mit den Sequenzen SEQ ID NO: 745 bis SEQ ID NO: 764 aus einer Kombinatorik der erfindungsgemäßen Nukleinsäuresonden,
- Fig. 33: Gruppe 32 mit den Sequenzen SEQ ID NO: 765 bis SEQ ID NO: 804 aus einer Kombinatorik der erfindungsgemäßen Nukleinsäuresonden,
- Fig. 34: Gruppe 33 mit den Sequenzen SEQ ID NO: 805 bis SEQ ID NO: 824 aus einer Kombinatorik der erfindungsgemäßen Nukleinsäuresonden.
- Fig. 35: Gruppe 34 mit den Sequenzen SEQ ID NO: 825 bis SEQ ID NO: 844 aus einer Kombinatorik der erfindungsgemäßen Nukleinsäuresonden,
- Fig. 36: Gruppe 35 mit den Sequenzen SEQ ID NO: 845 bis SEQ ID NO: 865 aus einer Kombinatorik der erfindungsgemäßen Nukleinsäuresonden,
- Fig. 37: Gruppe 36 mit den Sequenzen SEQ ID NO: 866 bis SEQ ID NO: 886 aus einer Kombinatorik der erfindungsgemäßen Nukleinsäuresonden,
- Fig. 38: Gruppe 37 mit den Sequenzen SEQ ID NO: 887 bis SEQ ID NO: 927 aus einer Kombinatorik der erfindungsgemäßen Nukleinsäuresonden,
- Fig. 39: Gruppe 38 mit den Sequenzen SEQ ID NO: 928 bis SEQ ID NO: 947 aus einer Kombinatorik der erfindungsgemäßen Nukleinsäuresonden,
- Fig. 40: Gruppe 39 mit den Sequenzen SEQ ID NO: 948 bis SEQ ID NO: 967 aus einer Kombinatorik der erfindungsgemäßen Nukleinsäuresonden,
- Fig. 41: Gruppe 40 mit den Sequenzen SEQ ID NO: 968 bis SEQ ID NO: 988 aus einer Kombinatorik der erfindungsgemäßen Nukleinsäuresonden,
- Fig. 42: Gruppe 41 mit den Sequenzen SEQ ID NO: 989 bis SEQ ID NO: 1009 aus einer Kombinatorik der erfindungsgemäßen Nukleinsäuresonden,
- Fig. 43: Gruppe 42 mit den Sequenzen SEQ ID NO: 1010 bis SEQ ID NO: 1071 aus einer Kombinatorik der erfindungsgemäßen Nukleinsäuresonden,
- Fig. 44: Gruppe 43 mit den Sequenzen SEQ ID NO: 1072 bis SEQ ID NO: 1133 aus einer Kombinatorik der erfindungsgemäßen Nukleinsäuresonden,
- Fig. 45: Gruppe 44 mit den Sequenzen SEQ ID NO: 1134 bis SEQ ID NO: 1154 aus einer Kombinatorik der erfindungsgemäßen Nukleinsäuresonden,
- Fig. 46: Gruppe 45 mit den Sequenzen SEQ ID NO: 1155 bis SEQ ID NO: 1196 aus einer Kombinatorik der erfindungsgemäßen Nukleinsäuresonden,
- Fig. 47: Gruppe 46 mit den Sequenzen SEQ ID NO: 1197 bis SEQ ID NO: 1216 aus einer Kombinatorik der erfindungsgemäßen Nukleinsäuresonden.

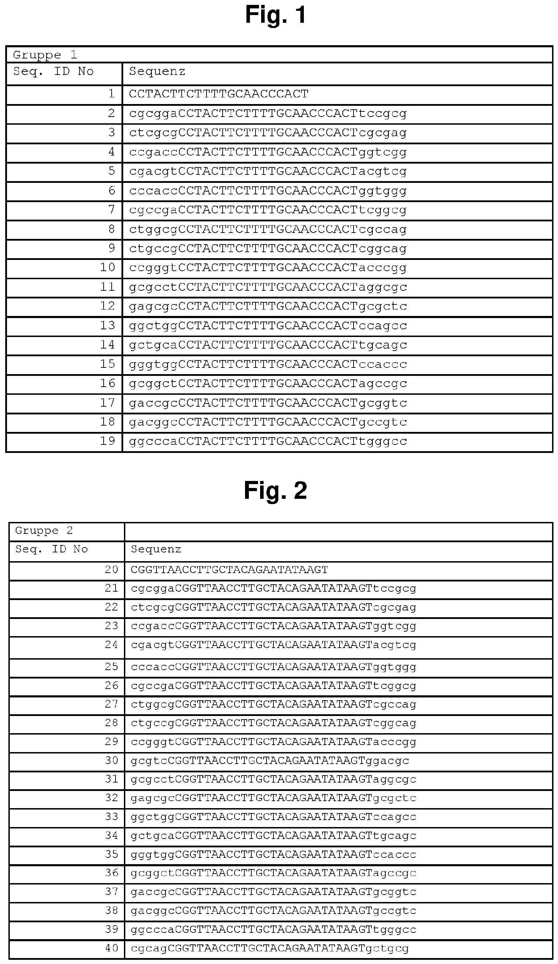

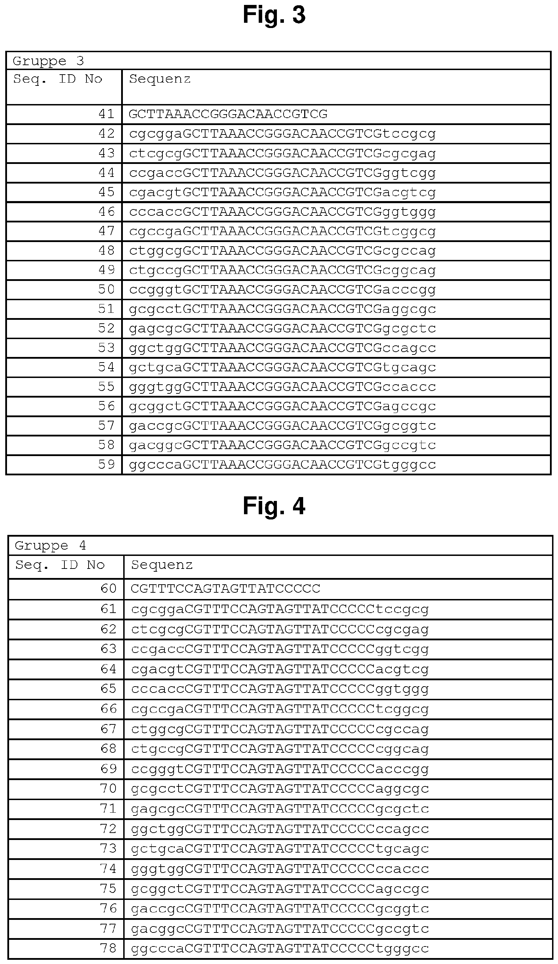

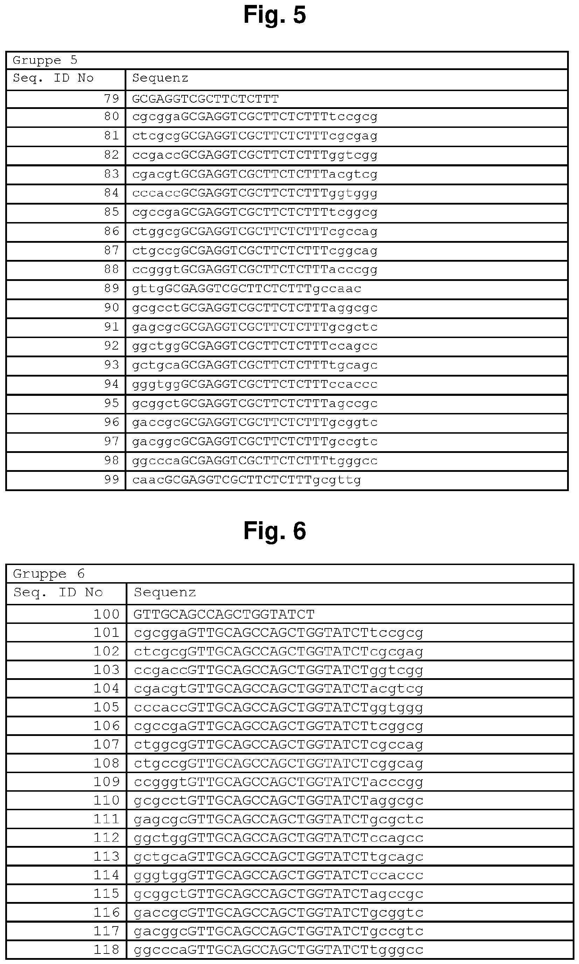

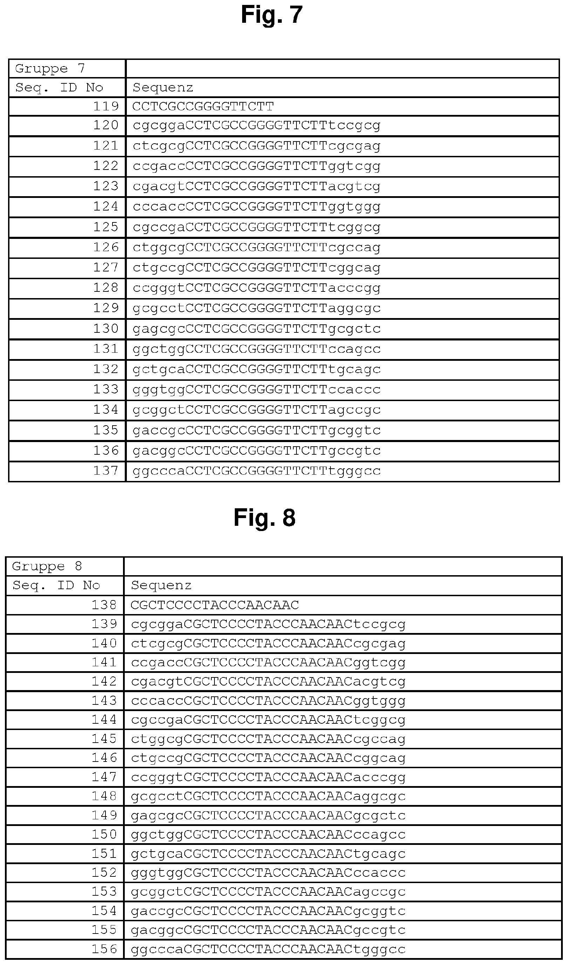

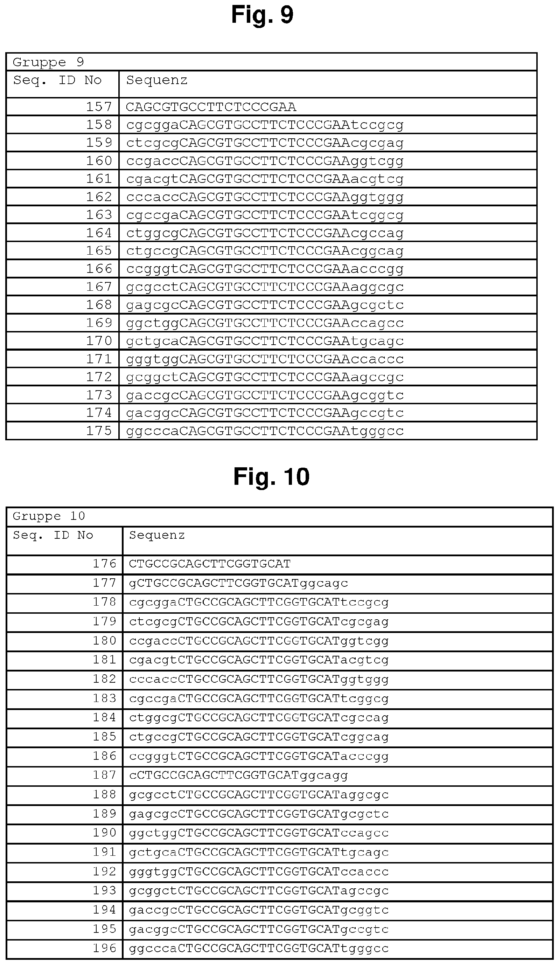

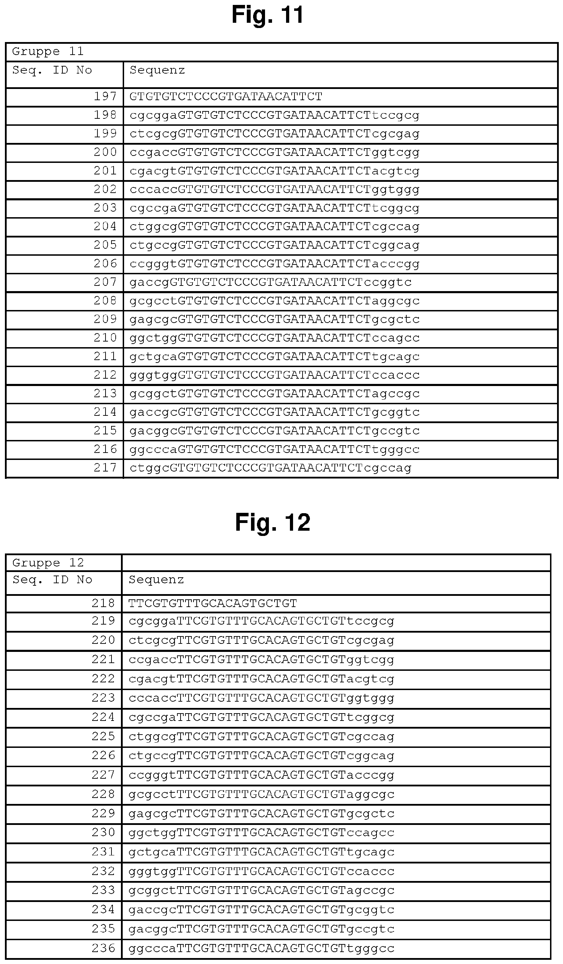

Fig. 1 bis 12 zeigen eine Kombinatorik der erfindungsgemäßen Nukleinsäuresonden, bestehend aus den Gruppen 1 bis 12. Es werden aus jeder Gruppe ein Vertreter oder aus wenigstens zwei Gruppen ein Vertreter für eine Mischung von Nukleinsäuresonden ausgewählt und in einem erfindungsgemäßen Verfahren zum Nachweis von Mikroorganismen, z.B. der Bakterienfamilie der Enterobacteriaceaen, verwendet.

Die Nukleinsäuresonden weisen jeweils am 5'-Ende und/oder am 3'-Ende eine Stammsequenz auf, die in den Beispielen gemäß Fig. 1 bis 12 in Kleinbuchstaben dargestellt ist. Zwischen den Stammsequenzen der Nukleinsäuresonden ist jeweils ein Funktionsteil ausgebildet, der in den Beispielen gemäß Fig. 1 bis 12 in Großbuchstaben dargestellt ist. Der Funktionsteil ist der komplementärer, d.h. bindender Bereich zur Zielsequenz. Die Stammsequenzen innerhalb jeder Nukleinsäuresonde können in Abwesenheit von Zielsequenzen an einander binden und so eine "Haarnadel"-Struktur bilden, wodurch die Fluoreszenz des Farbstoffs unterdrückt wird. Nach der Bindung des Funktionsteils an die Zielsequenz löst sich diese "Haarnadel"-Struktur wieder auf, wobei die Fluoreszenz des Farbstoffs detektiert werden kann. Zur Realisierung des Verfahrens kann ein fluidisches Kanalsystem, beispielsweise ein scheibenförmiger Probenträger, der rotationssymetrisch sein kann aber nicht sein muss, mit Mitteln zum Durchführen dieses Verfahrens versehen sein. Insbesondere kann das Kanalsystem eine Kavität mit wenigstens einer Nukleinsäuresonde oder wenigstens zwei Nukleinsäuresonden ausgewählt aus den erwähnten Gruppen und/oder weiteren Sequenzen aus der vorliegenden Kombinatorik der Gruppen 1 bis 12 umfassen und/oder mit Mitteln zum optischen Detektieren von markierten Mikroorganismen versehen sein.

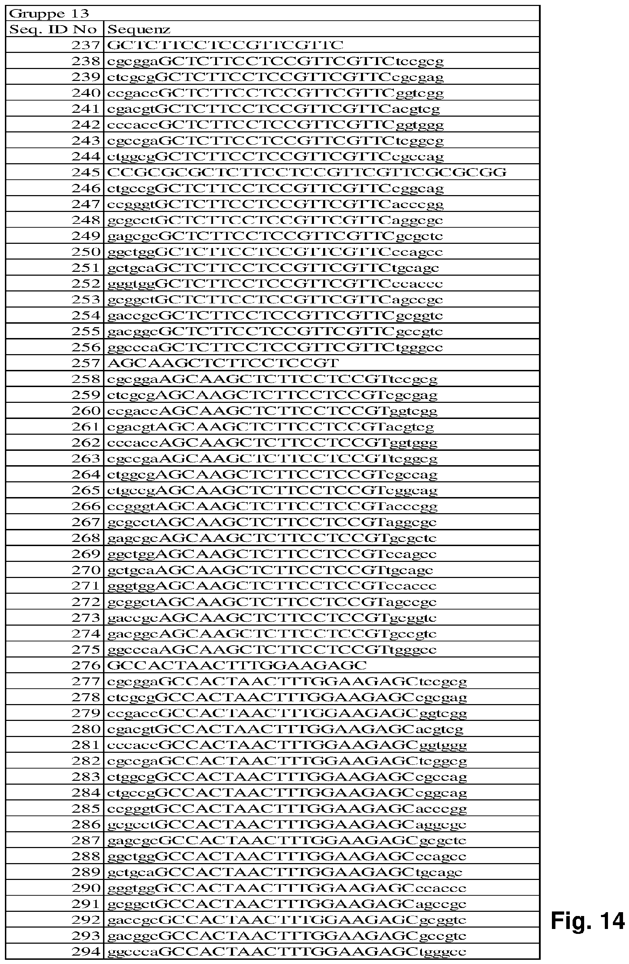

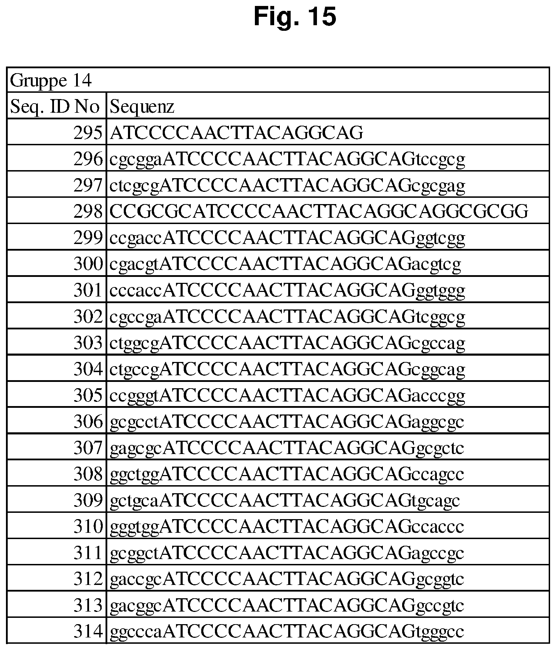

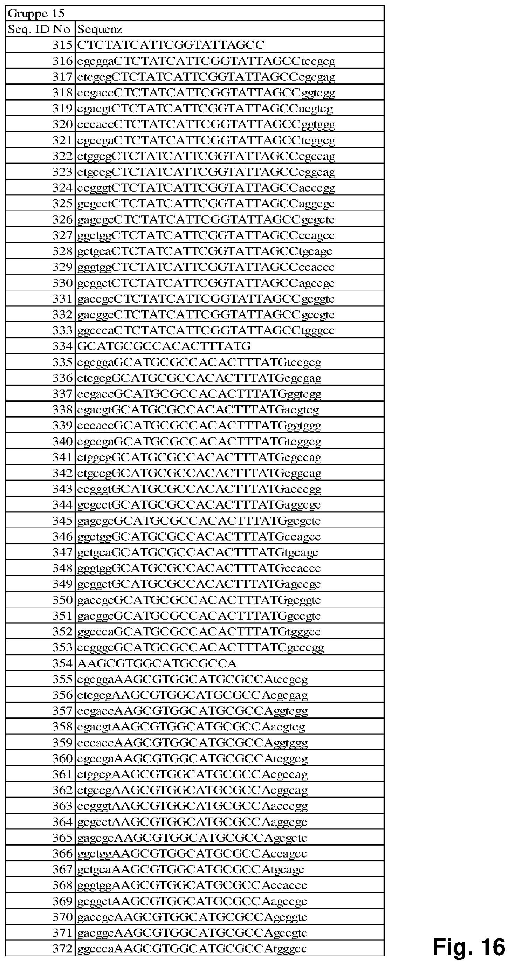

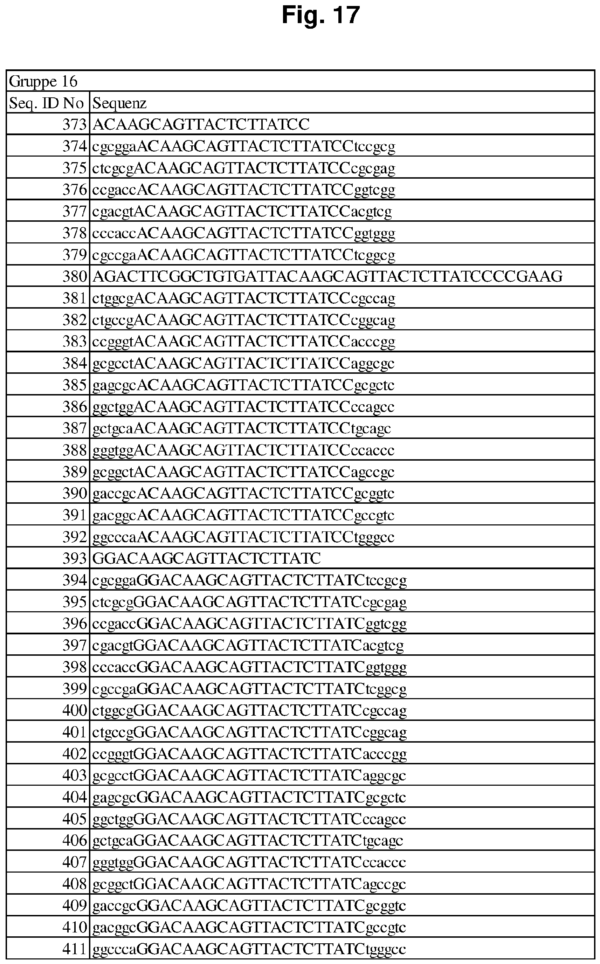

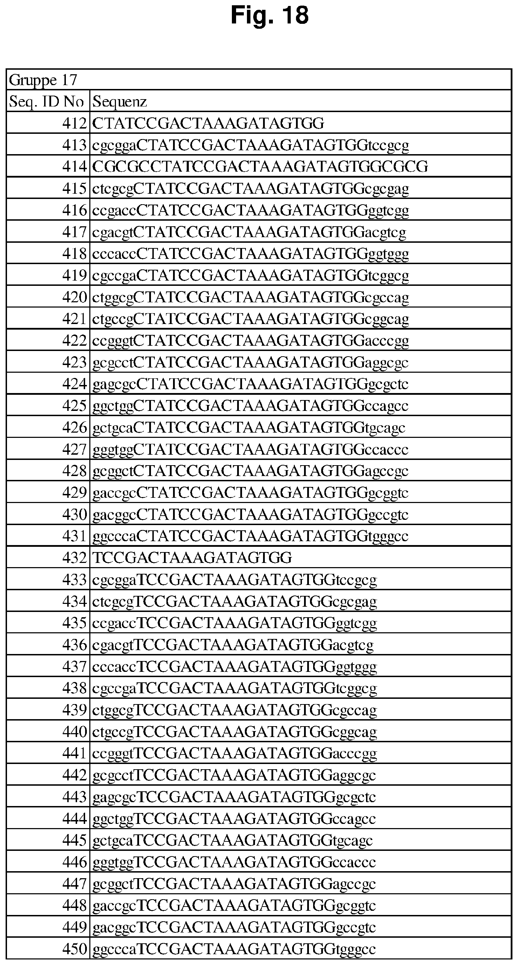

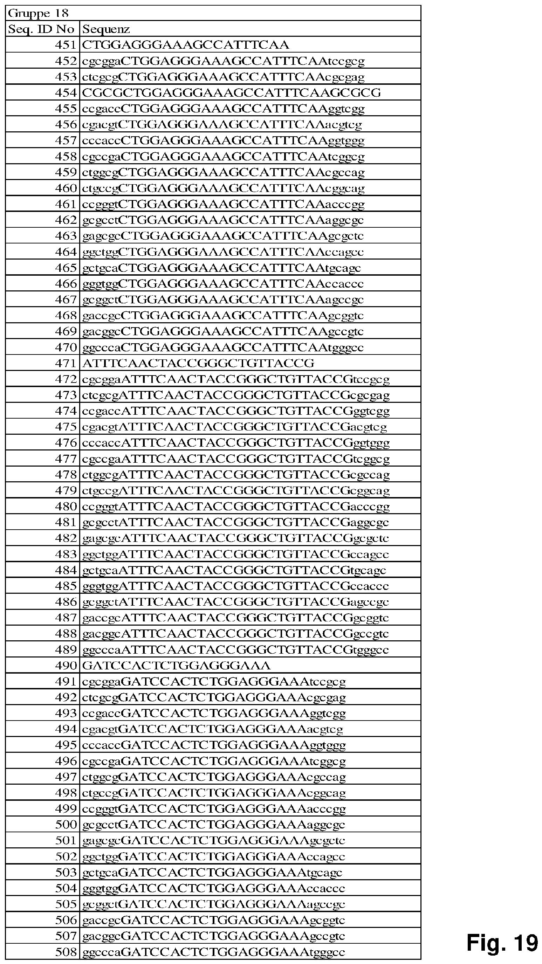

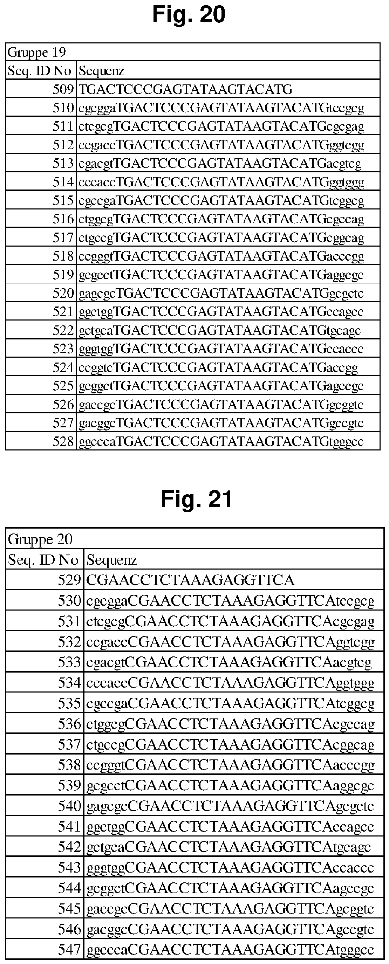

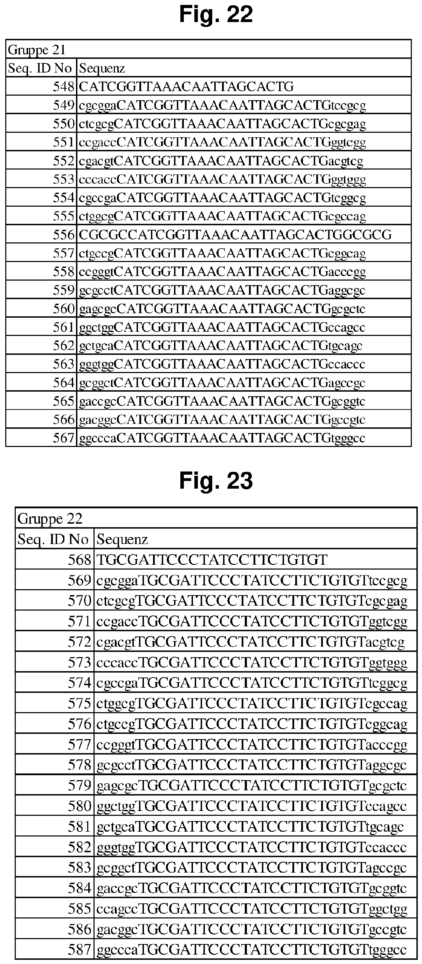

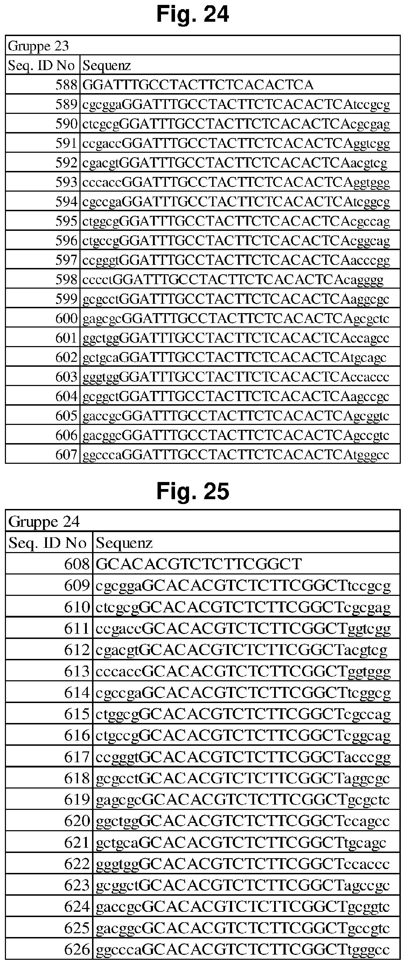

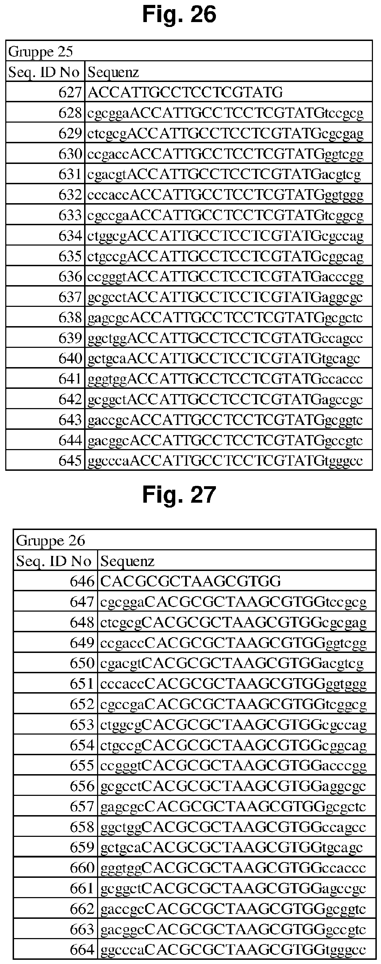

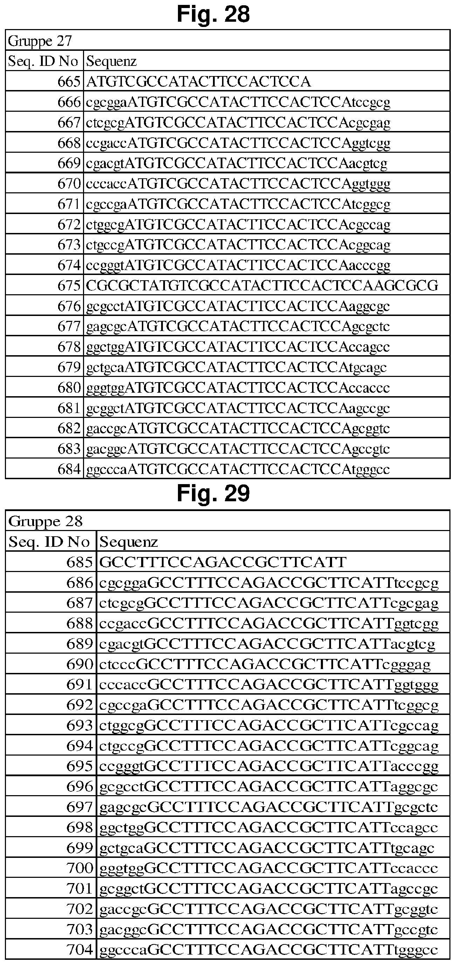

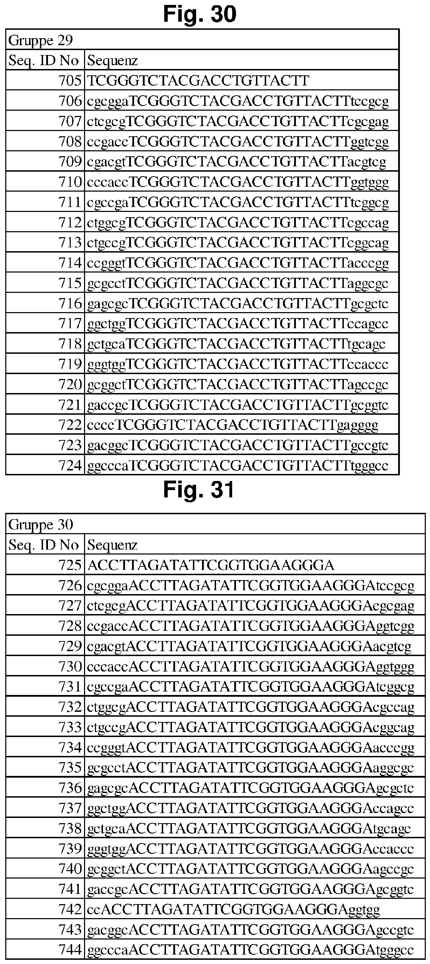

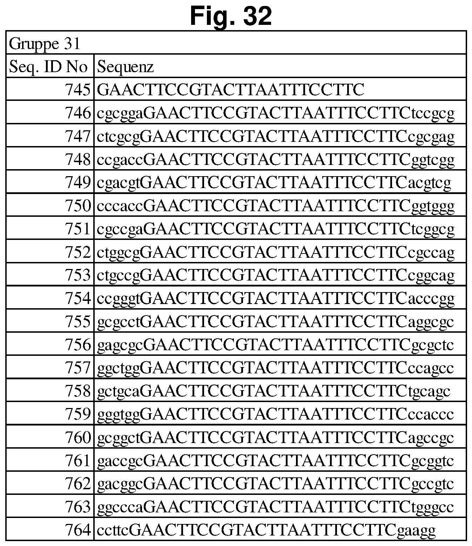

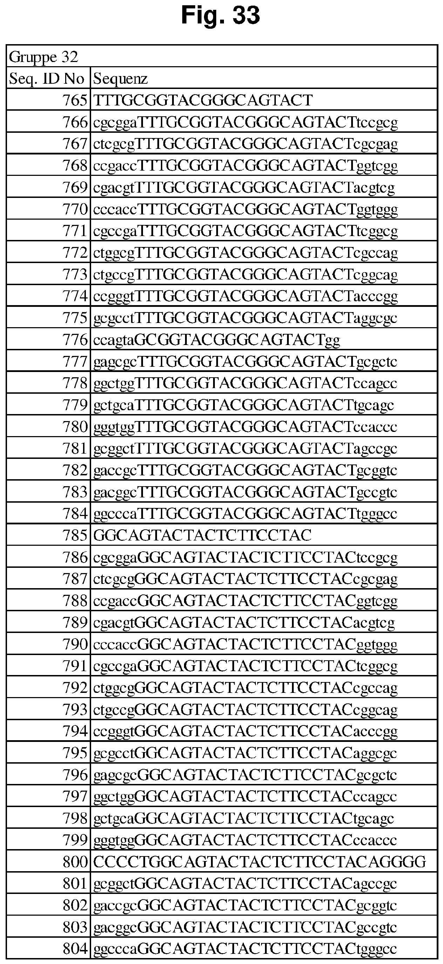

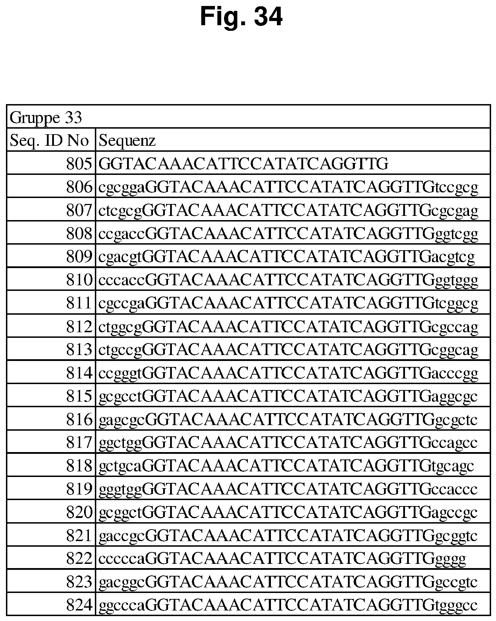

Fig. 14 bis 34 zeigen eine Kombinatorik der erfindungsgemäßen Nukleinsäuresonden, bestehend aus den Gruppen 13 bis 33. Es werden aus jeder Gruppe ein Vertreter oder aus wenigstens zwei Gruppen ein Vertreter für eine Mischung von Nukleinsäuresonden ausgewählt und in einem erfindungsgemäßen Verfahren zum Nachweis von Mikroorganismen, insbesondere der Bakterienfamilie Listeriaceae, verwendet.

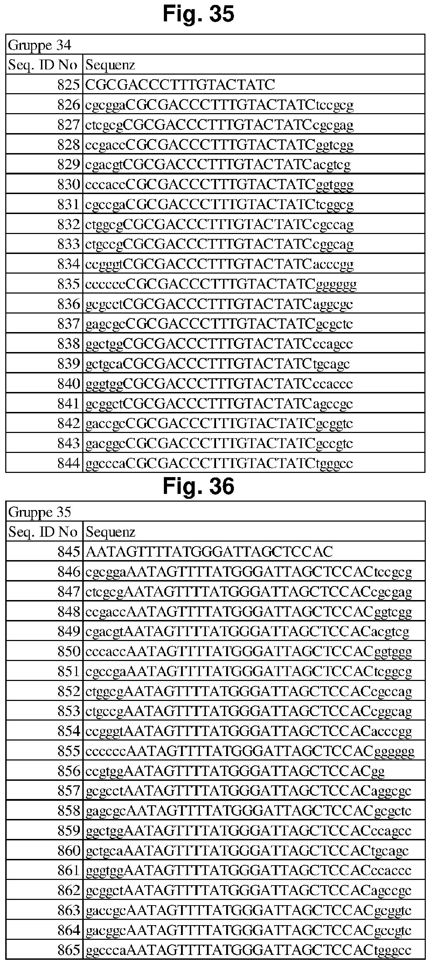

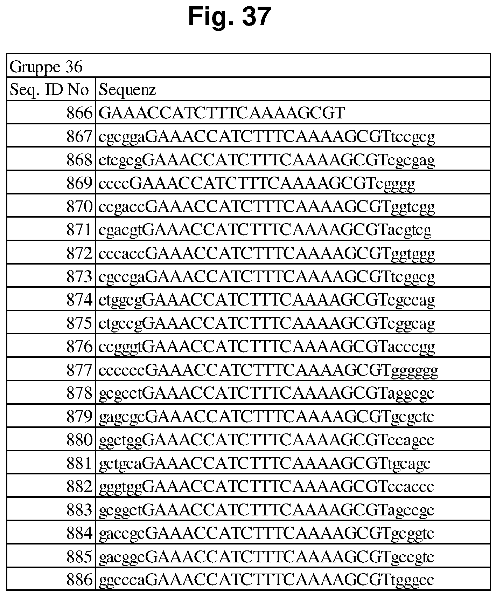

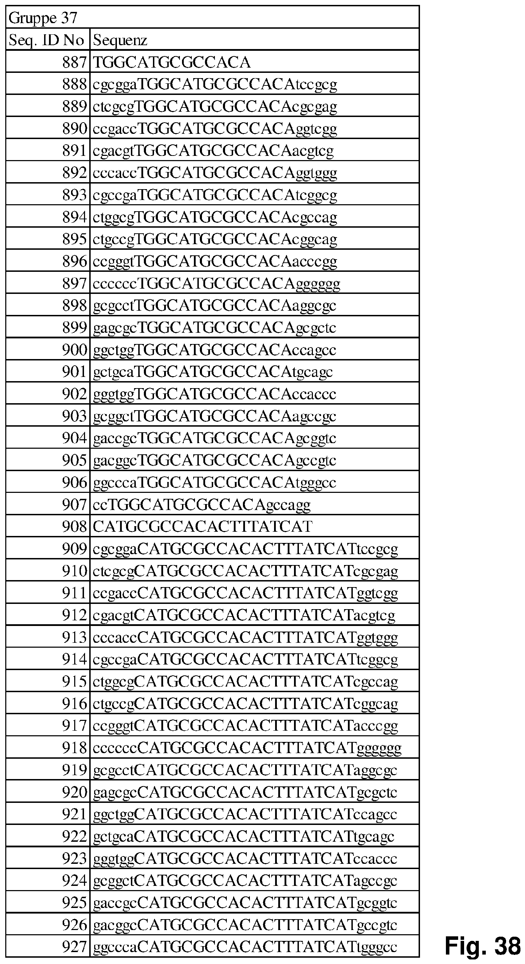

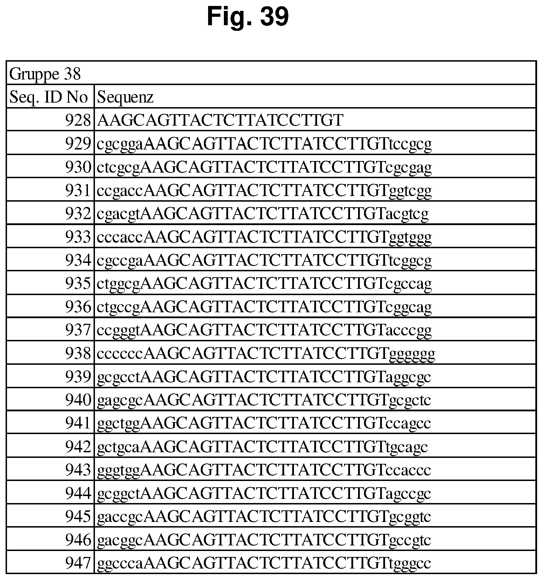

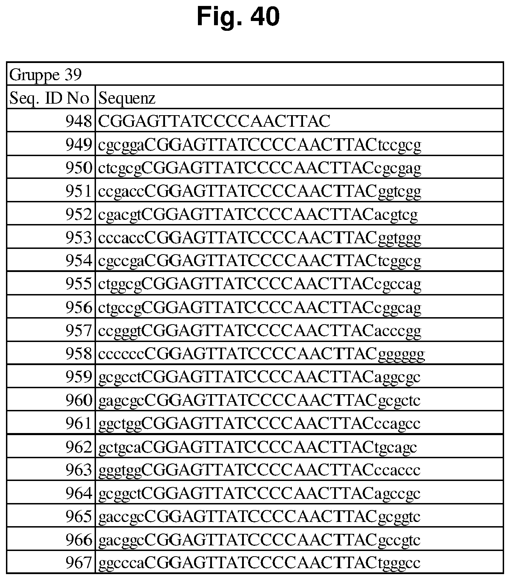

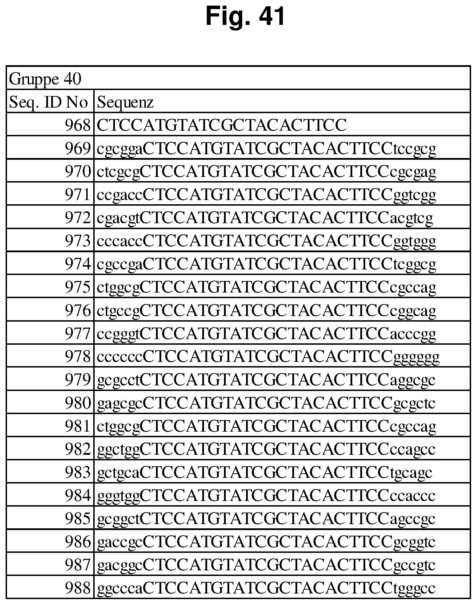

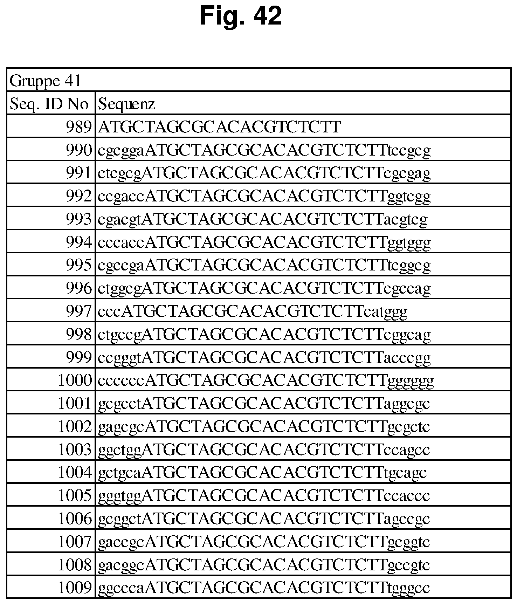

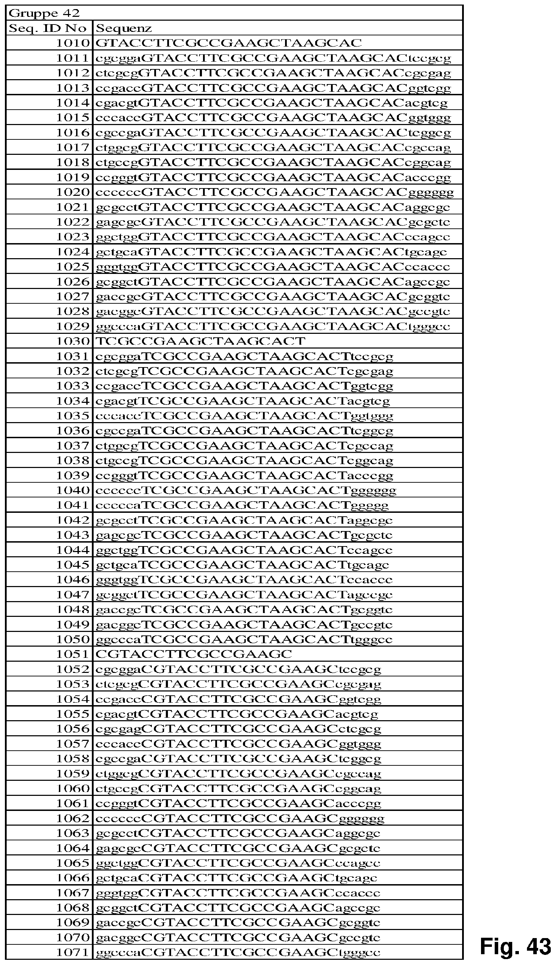

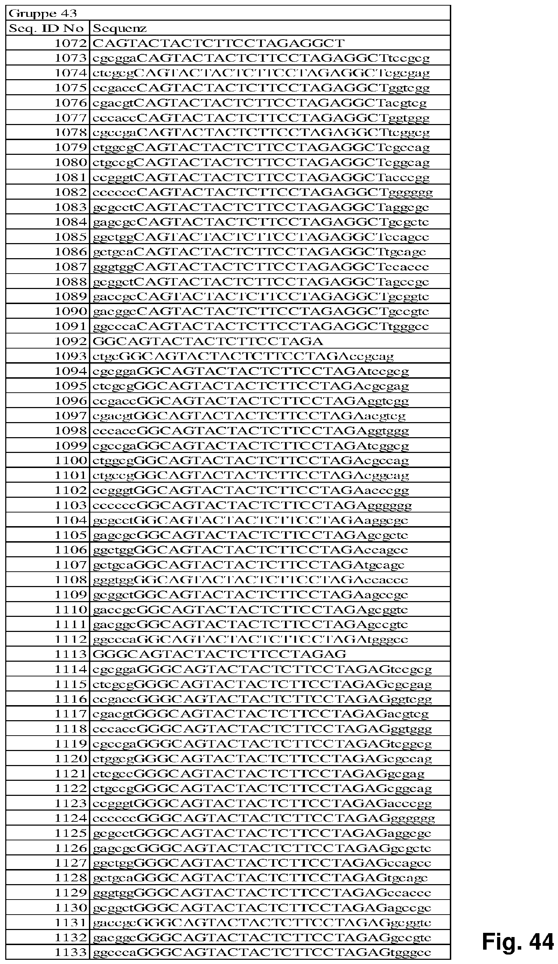

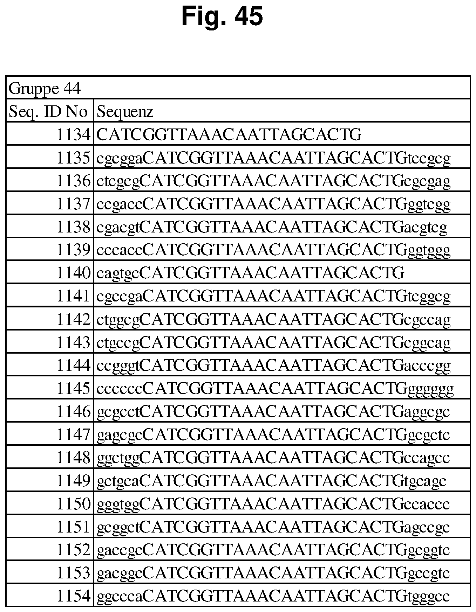

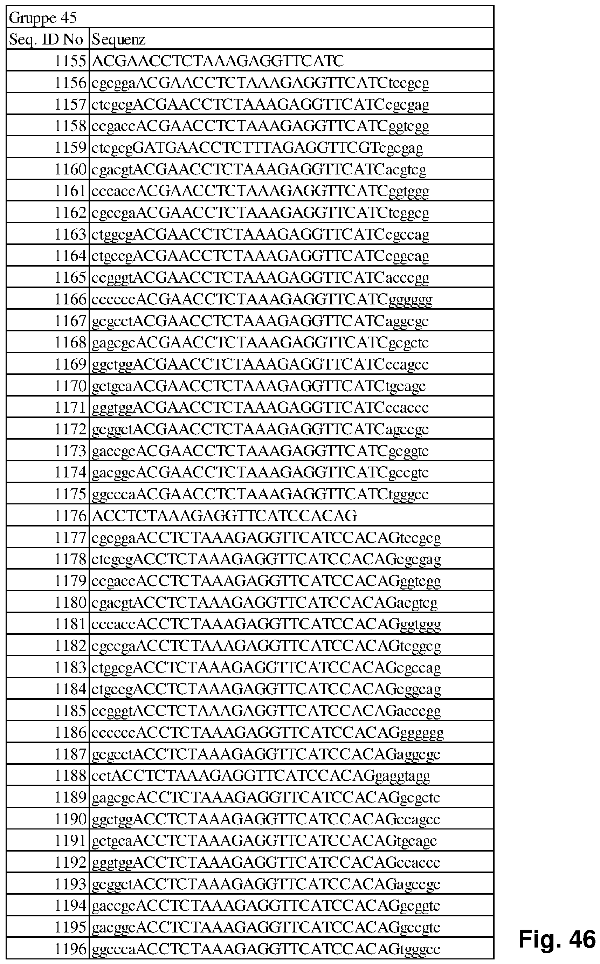

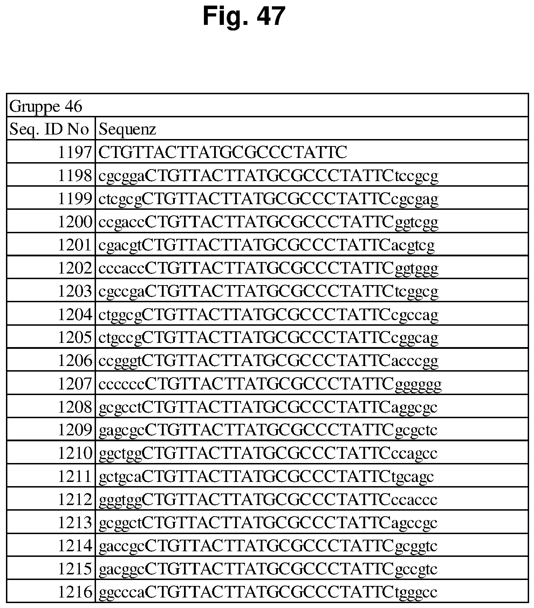

Fig. 35 bis 47 zeigen eine Kombinatorik der erfindungsgemäßen Nukleinsäuresonden, bestehend aus den Gruppen 34 bis 46. Es werden aus jeder Gruppe ein Vertreter oder aus wenigstens zwei Gruppen ein Vertreter für eine Mischung von Nukleinsäuresonden ausgewählt und in einem erfindungsgemäßen Verfahren zum Nachweis von Mikroorganismen, insbesondere der Bakterienspezies Listeria monocytogenes, verwendet.

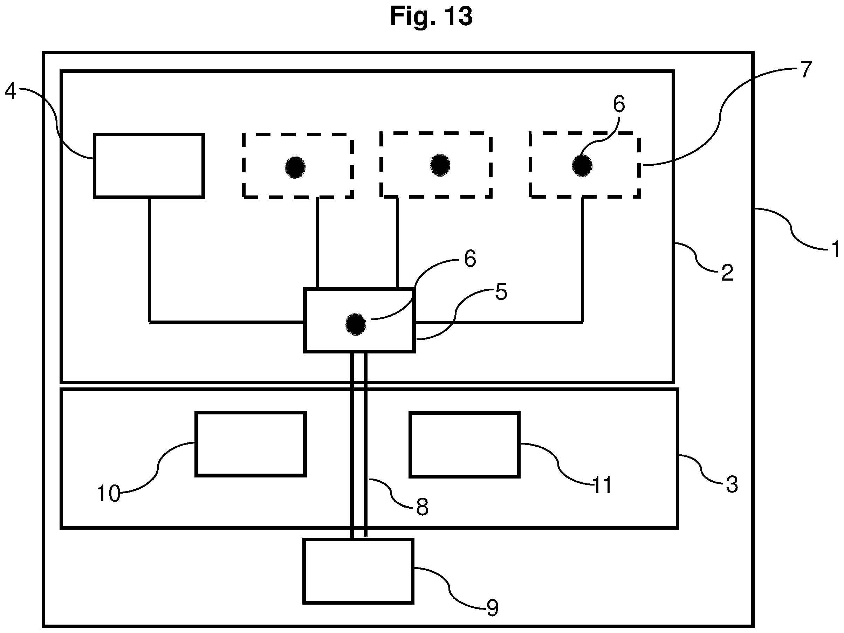

Fig. 13 zeigt ein fluidisches Kanalsystem 1 zum Nachweis von Mikroorganismen, z.B. der Bakterienfamilie der Enterobacteriaceaen, mit einem Vorbereitungsbereich 2 und einem Detektionsbereich 3. Das fluidische Kanalsystem hat eine Probenahme-Kammer 4, in die eine Untersuchungsprobe direkt einführbar ist. Die Probenahme-Kammer 4 ist mit einer Reaktionskammer 5 verbunden, die vorgehaltene Reagenzien 6 enthält.

Aus Fig. 13 ist erkennbar, dass Reaktionskammer 5 mit wenigstens einer Reagenzien-Kammer 7 verbunden ist, die vorgehaltene Reagenzien 6 enthält. In der hier in Fig. 13 dargestellten Kanalsystem-Ausführung ist das Kanalsystem 1 mit drei Reagenzien-Kammern 7 ausgebildet, die parallel zueinander geschaltet sind.

Bei weiteren Ausführungsbeispielen ist eine andere Anzahl von Reagenzien-Kammern 7 vorhanden, beispielsweise mehr als drei oder weniger als drei. Bei einem weiteren Ausführungsbeispiel ist ein Kanalsystem 1 frei von Reagenzien-Kammern 7 ausgebildet.

Die Reaktionskammer 5 ist über ein Detektionskanal 8 mit einer Detektionskammer 9 verbunden. Das Kanalsystem 1 hat eine Lichtquelle 10 und einen Detektor 11, die in dem Detektionsbereich 3 ausgebildet sind.

In einer bevorzugten Anwendung wird zunächst die Untersuchungsprobe in das fluidische Kanalsystem 1 eingeführt. In dem Vorbereitungsbereich 2 wird die Untersuchungsprobe zu der Reaktionskammer 5 geleitet. Gleichzeitig oder anschließend werden die Reagenzien 6 aus den Reagenzien-Kammern 7 zur Reaktionskammer 5 geleitet, wobei dieser Schritt entfallen kann, falls Reagenzien bereits in der Reaktionskammer 5 vorgehalten werden.

Nach der Reaktion mit den vorgehaltenen Reagenzien 6 wird die Untersuchungsprobe über das Detektionskanal 8 in die Detektionskammer 9 geleitet. Anschließend wird die Nachweisreaktion durch die optische Signalerfassung mittels Photometrie durchgeführt. Beispielsweise wird die Nachweisreaktion mittels Turbidometrie, Zytometrie oder Fluoreszenzmessung durchgeführt.

Erfindungsgemäß wird somit vorgeschlagen, ein Verfahren zum Nachweis von Mikroorganismen, z.B. der Bakterienfamilie der Enterobacteriaceaen, in einer Probe bereitzustellen, mittels Einschleusen identitätsbestimmender Nukleinsäuresonden in die einzelnen Zellkörper, wobei die Nukleinsäuresonden mit den Nukleinsäuren der Mikroorganismen eine Hybridisierung eingehen, und anschließendem optischen Detektieren der in den einzelnen Zellkörpern erzeugten Hybridisierungen, wobei eine Mischung aus wenigstens einer ersten Nukleinsäuresonde und einer zweiten Nukleinsäuresonde verwendet wird, und dass die erste Nukleinsäuresonde und die zweite Nukleinsäuresonde jeweils in einem von zwei miteinander nicht überlappenden Bereichen der Zielnukleinsäure anbinden.

### Bezugszeichenliste

- 1: fluidisches Kanalsystem
- 2: Vorbereitungsbereich
- 3: Detektionsbereich
- 4: Probenahme-Kammer
- 5: Reaktionskammer (mit vorgehaltenen Reagenzien)
- 6: vorgehaltene Reagenzien
- 7: optional wenigstens eine Reagenzien-Kammer (mit vorgehaltenen Reagenzien)
- 8: Detektionskanal
- 9: Detektionskammer
- 10: Lichtquelle
- 11: Detektor

## Patentansprüche

1. Verfahren zum Nachweis von Mikroorganismen, z.B. z.B. der Bakterienfamilie der Enterobacteriaceaen, in einer Probe mittels Einschleusen identitätsbestimmender Nukleinsäuresonden in die einzelnen Zellkörper, wobei die Nukleinsäuresonden mit den Nukleinsäuren der Mikroorganismen eine Hybridisierung eingehen, und anschließendem optischen Detektieren der in den einzelnen Zellkörpern erzeugten Hybridisierungen, **dadurch gekennzeichnet, dass** eine Mischung aus wenigstens einer ersten Nukleinsäuresonde und einer zweiten Nukleinsäuresonde verwendet wird, und dass die erste Nukleinsäuresonde und die zweite Nukleinsäuresonde jeweils in einem von zwei miteinander nicht überlappenden Bereichen der Zielnukleinsäure anbinden.

2. Verfahren nach Anspruch 1, **dadurch gekennzeichnet, dass** die Mischung so gebildet ist, dass aus der vorliegenden Kombinatorik der Gruppen 1 bis 12 jeweils aus jeder Gruppe ein Vertreter oder aus wenigstens zwei Gruppen ein Vertreter ausgewählt werden, insbesondere wobei die Gruppe 1 einzelne oder alle der SEQ ID NO: 1 bis SEQ ID NO: 19 umfasst, die Gruppe 2 einzelne oder alle der SEQ ID NO: 20 bis SEQ ID NO: 40 umfasst, die Gruppe 3 einzelne oder alle der SEQ ID NO: 41 bis SEQ ID NO: 59 umfasst, die Gruppe 4 einzelne oder alle der SEQ ID NO: 60 bis SEQ ID NO: 78 umfasst, die Gruppe 5 einzelne oder alle der SEQ ID NO: 79 bis SEQ ID NO: 99 umfasst, die Gruppe 6 einzelne oder alle der SEQ ID NO: 100 bis SEQ ID NO: 118 umfasst, die Gruppe 7 einzelne oder alle der SEQ ID NO: 119 bis SEQ ID NO: 137 umfasst, die Gruppe 8 einzelne oder alle der SEQ ID NO: 138 bis SEQ ID NO: 156 umfasst, die Gruppe 9 einzelne oder alle der SEQ ID NO: 157 bis SEQ ID NO: 175 umfasst, die Gruppe 10 einzelne oder alle der SEQ ID NO: 176 bis SEQ ID NO: 196 umfasst, die Gruppe 11 einzelne oder alle der SEQ ID NO: 197 bis SEQ ID NO: 217 umfasst und die Gruppe 12 einzelne oder alle der SEQ ID NO: 218 bis SEQ ID NO: 236 umfasst.

3. Verfahren nach Anspruch 1, **dadurch gekennzeichnet, dass** die Mischung so gebildet ist, dass aus der vorliegenden Kombinatorik der Gruppen 13 bis 33 jeweils aus jeder Gruppe ein Vertreter oder aus wenigstens zwei Gruppen ein Vertreter ausgewählt werden, insbesondere wobei die Gruppe 13 einzelne oder alle der SEQ ID NO: 237 bis SEQ ID NO: 294 umfasst, die Gruppe 14 einzelne oder alle der SEQ ID NO: 295 bis SEQ ID NO: 314 umfasst, die Gruppe 15 einzelne oder alle der SEQ ID NO: 315 bis SEQ ID NO: 372 umfasst, die Gruppe 16 einzelne oder alle der SEQ ID NO: 373 bis SEQ ID NO: 411 umfasst, die Gruppe 17 einzelne oder alle der SEQ ID NO: 412 bis SEQ ID NO: 450 umfasst, die Gruppe 18 einzelne oder alle der SEQ ID NO: 451 bis SEQ ID NO: 508 umfasst, die Gruppe 19 einzelne oder alle der SEQ ID NO: 509 bis SEQ ID NO: 528 umfasst, die Gruppe 20 einzelne oder alle der SEQ ID NO: 529 bis SEQ ID NO: 547 umfasst, die Gruppe 21 einzelne oder alle der SEQ ID NO: 548 bis SEQ ID NO: 567 umfasst, die Gruppe 22 einzelne oder alle der SEQ ID NO: 568 bis SEQ ID NO: 587 umfasst, die Gruppe 23 einzelne oder alle der SEQ ID NO: 588 bis SEQ ID NO: 607 umfasst, die Gruppe 24 einzelne oder alle der SEQ ID NO: 608 bis SEQ ID NO: 626 umfasst, die Gruppe 25 einzelne oder alle der SEQ ID NO: 627 bis SEQ ID NO: 645 umfasst, die Gruppe 26 einzelne oder alle der SEQ ID NO: 646 bis SEQ ID NO: 664 umfasst, die Gruppe 27 einzelne oder alle der SEQ ID NO: 665 bis SEQ ID NO: 684 umfasst, die Gruppe 28 einzelne oder alle der SEQ ID NO: 685 bis SEQ ID NO: 704 umfasst, die Gruppe 29 einzelne oder alle der SEQ ID NO: 705 bis SEQ ID NO: 724 umfasst, die Gruppe 30 einzelne oder alle der SEQ ID NO: 725 bis SEQ ID NO: 744 umfasst, die Gruppe 31 einzelne oder alle der SEQ ID NO: 745 bis SEQ ID NO: 764 umfasst, die Gruppe 32 einzelne oder alle der SEQ ID NO: 765 bis SEQ ID NO: 804 umfasst und die Gruppe 33 einzelne oder alle der SEQ ID NO: 805 bis SEQ ID NO: 824 umfasst.

4. Verfahren nach Anspruch 1, **dadurch gekennzeichnet, dass** die Mischung so gebildet ist, dass aus der vorliegenden Kombinatorik der Gruppen 34 bis 46 jeweils aus jeder Gruppe ein Vertreter oder aus wenigstens zwei Gruppen ein Vertreter ausgewählt werden, insbesondere wobei die Gruppe 34 einzelne oder alle der SEQ ID NO: 825 bis SEQ ID NO: 844 umfasst, die Gruppe 35 einzelne oder alle der SEQ ID NO: 845 bis SEQ ID NO: 865 umfasst, die Gruppe 36 einzelne oder alle der SEQ ID NO: 866 bis SEQ ID NO: 886 umfasst, die Gruppe 37 einzelne oder alle der SEQ ID NO: 887 bis SEQ ID NO: 927 umfasst, die Gruppe 38 einzelne oder alle der SEQ ID NO: 928 bis SEQ ID NO: 947 umfasst, die Gruppe 39 einzelne oder alle der SEQ ID NO: 948 bis SEQ ID NO: 967 umfasst, die Gruppe 40 einzelne oder alle der SEQ ID NO: 968 bis SEQ ID NO: 988 umfasst, die Gruppe 41 einzelne oder alle der SEQ ID NO: 989 bis SEQ ID NO: 1009 umfasst, die Gruppe 42 einzelne oder alle der SEQ ID NO: 1010 bis SEQ ID NO: 1071 umfasst, die Gruppe 43 einzelne oder alle der SEQ ID NO: 1072 bis SEQ ID NO: 1133 umfasst, die Gruppe 44 einzelne oder alle der SEQ ID NO: 1134 bis SEQ ID NO: 1154 umfasst, die Gruppe 45 einzelne oder alle der SEQ ID NO: 1155 bis SEQ ID NO: 1196 umfasst und die Gruppe 46 einzelne oder alle der SEQ ID NO: 1197 bis SEQ ID NO: 1216 umfasst.

5. Verfahren nach einem der vorangehenden Ansprüche, **dadurch gekennzeichnet, dass** die Nachweisreaktion unter Verwendung von Nukleinsäuresonden mittels In-Situ-Hybridisierung und/oder Fluoreszenz-In-Situ-Hybridisierung (FISH), Nukleinsäuren-Amplifikation und/oder Mikroarray erfolgt.

6. Verfahren nach einem der vorangehenden Ansprüche, **dadurch gekennzeichnet, dass** die Nukleinsäuresonden jeweils als lineare Sonden und/oder Sonden mit Sekundärstruktur, vorzugsweise Molecular Beacons, ausgebildet sind.

7. Verfahren nach einem der vorangehenden Ansprüche, **dadurch gekennzeichnet, dass** eine optische Sensitivität so eingestellt ist, dass nur solche Mikroorganismen detektiert werden, die wenigstens zwei Anbindungen aufweisen.

8. Verfahren nach einem der vorangehenden Ansprüche, **dadurch gekennzeichnet, dass** die erste und/oder zweite Nukleinsäuresonde wenigstens einen ersten Farbstoff konjugiert mit dem 5'-Ende und/oder wenigstens einen zweiten Farbstoff konjugiert mit dem 3'-Ende und/oder intramolekular gebundene Farbstoffe aufweist.

9. Verfahren nach einem der vorangehenden Ansprüche, **dadurch gekennzeichnet, dass** die Nukleinsäuresonde am 5'-Ende und/oder 3'-Ende weitere Nukleotide als Stammsequenz und/oder wenigstens einen Funktionsteil aufweist, insbesondere wobei die Stammsequenzen und/oder Funktionsteile so zueinander ausgestaltet sind, dass sie nicht gegenseitig miteinander wechselwirken.

10. Verfahren nach einem der vorangehenden Ansprüche, **dadurch gekennzeichnet, dass** die Auswahl der nicht überlappenden Bereiche so gewählt ist, dass sich die Nukleinsäuresonden in ihrem Abstrahlverhalten nicht gegenseitig stören.

11. Verfahren nach einem der vorangehenden Ansprüche, **dadurch gekennzeichnet, dass** die Nukleinsäuresonden so ausgestaltet sind, dass eine bestimmte Farbkombination detektierbar ist, insbesondere wobei die oder eine erste Nukleinsäuresonde einen ersten Farbstoff und die oder eine zweite Nukleinsäuresonde einen zweiten Farbstoff aufweist.

12. Verfahren nach einem der vorangehenden Ansprüche, **dadurch gekennzeichnet, dass** die Nukleinsäuresonde wenigstens einen optisch detektierbaren Marker aufweist.

13. Verfahren nach einem der vorangehenden Ansprüche, **dadurch gekennzeichnet, dass** der detektierbare Marker ein Enzymmarker, Affinitätsmarker und/oder ein Farbstoff ist, insbesondere wobei der Farbstoff ein Fluoreszenzfarbstoff ist und/oder der Affinitätsmarker Affinitätsbindungspaare Biotin-Streptavidin oder Antigen-Antikörper umfasst.

14. Verfahren nach einem der vorangehenden Ansprüche, **dadurch gekennzeichnet, dass** jede Nukleinsäuresonde an mindestens 80% der Bakterienfamilie der Enterobacteriaceaen anbindet und/oder jede Nukleinsäuresonde nachweisbar nur an die Nukleinsäuren der Bakterienfamilie der Enterobacteriaceaen und nicht an die Nukleinsäuren eines Organismus, der zu einer anderen Bakterienfamilie gehört, anbindet.

15. Verwendung wenigstens einer Nukleinsäuresonde oder wenigstens zwei Nukleinsäuresonden ausgewählt aus der Gruppe bestehend aus SEQ ID NO: 1, SEQ ID NO: 20, SEQ ID NO: 41, SEQ ID NO: 60, SEQ ID NO: 79, SEQ ID NO: 100, SEQ ID NO: 119, SEQ ID NO: 138, SEQ ID NO: 157, SEQ ID NO: 176, SEQ ID NO: 197, SEQ ID NO: 218 und/oder weiteren Sequenzen aus der vorliegenden Kombinatorik der Gruppen 1 bis 12 zum Nachweis der Enterobacteriaceaen und/oder zur Immobilisierung auf einem Trägermaterial, insbesondere einem fluidischen Kanalsystem.

16. Verwendung wenigstens einer Nukleinsäuresonde oder wenigstens zwei Nukleinsäuresonden vor, ausgewählt aus der Gruppe bestehend aus SEQ ID NO: 237, SEQ ID NO: 295, SEQ ID NO: 315, SEQ ID NO: 373, SEQ ID NO: 412, SEQ ID NO: 451, SEQ ID NO: 509, SEQ ID NO: 529, SEQ ID NO: 548, SEQ ID NO: 568, SEQ ID NO: 588, SEQ ID NO: 608, SEQ ID NO: 627, SEQ ID NO: 646, SEQ ID NO: 665, SEQ ID NO: 685, SEQ ID NO: 705, SEQ ID NO: 725, SEQ ID NO: 745, SEQ ID NO: 765, SEQ ID NO: 805 und/oder weiteren Sequenzen aus der vorliegenden Kombinatorik der Gruppen 13 bis 33 zum Nachweis der Listeriaceae und/oder zur Immobilisierung auf einem Trägermaterial, insbesondere einem fluidischen Kanalsystem.

17. Verwendung wenigstens einer Nukleinsäuresonde oder wenigstens zwei Nukleinsäuresonden vor, ausgewählt aus der Gruppe bestehend aus SEQ ID NO: 825, SEQ ID NO: 845, SEQ ID NO: 866, SEQ ID NO: 887, SEQ ID NO: 928, SEQ ID NO: 948, SEQ ID NO: 968, SEQ ID NO: 989, SEQ ID NO: 1010, SEQ ID NO: 1072, SEQ ID NO: 1134, SEQ ID NO: 1155, SEQ ID NO: 1197 und/oder weiteren Sequenzen aus der vorliegenden Kombinatorik der Gruppen 34 bis 46 zum Nachweis der Listeriaceae, insbesondere Listeria monocytogenes. und/oder zur Immobilisierung auf einem Trägermaterial, insbesondere einem fluidischen Kanalsystem.

18. Fluidisches Kanalsystem, vorzugsweise scheibenförmiger Probenträger, mit Mitteln zum Durchführen des Verfahrens nach einem der Ansprüche 1 bis 12, insbesondere umfassend mindestens eine Kavität mit wenigstens einer Nukleinsäuresonde oder wenigstens zwei Nukleinsäuresonden ausgewählt aus der Gruppe bestehend aus SEQ ID NO: 1, SEQ ID NO: 20, SEQ ID NO: 41, SEQ ID NO: 60, SEQ ID NO: 79, SEQ ID NO: 100, SEQ ID NO: 119, SEQ ID NO: 138, SEQ ID NO: 157, SEQ ID NO: 176, SEQ ID NO: 197, SEQ ID NO: 218 und/oder weiteren Sequenzen aus der vorliegenden Kombinatorik der Gruppen 1 bis 12und/oder einer Nukleinsäuresonde oder wenigstens zwei Nukleinsäuresonden ausgewählt aus der Gruppe bestehend aus SEQ ID NO: 237, SEQ ID NO: 295, SEQ ID NO: 315, SEQ ID NO: 373, SEQ ID NO: 412, SEQ ID NO: 451, SEQ ID NO: 509, SEQ ID NO: 529, SEQ ID NO: 548, SEQ ID NO: 568, SEQ ID NO: 588, SEQ ID NO: 608, SEQ ID NO: 627, SEQ ID NO: 646, SEQ ID NO: 665, SEQ ID NO: 685, SEQ ID NO: 705, SEQ ID NO: 725, SEQ ID NO: 745, SEQ ID NO: 765, SEQ ID NO: 805 und/oder weiteren Sequenzen aus der vorliegenden Kombinatorik der Gruppen 13 bis 33 und/ odereiner Nukleinsäuresonde oder wenigstens zwei Nukleinsäuresonden ausgewählt aus der Gruppe bestehend aus SEQ ID NO: 825, SEQ ID NO: 845, SEQ ID NO: 866, SEQ ID NO: 887, SEQ ID NO: 928, SEQ ID NO: 948, SEQ ID NO: 968, SEQ ID NO: 989, SEQ ID NO: 1010, SEQ ID NO: 1072, SEQ ID NO: 1134, SEQ ID NO: 1155, SEQ ID NO: 1197 und/oder weiteren Sequenzen aus der vorliegenden Kombinatorik der Gruppen 34 bis 46 und/oder mit Mitteln zum optischen Detektieren von markierten Mikroorganismen.
